# FASCICULE DE BREVET EUROPEEN

(11) **EP 2 125 803 B1**
(45) Date de publication et mention de la délivrance du brevet: **10.04.2013**
(21) Numéro de dépôt: 08775557.5
(22) Date de dépôt: 14.02.2008
(51) Int. Cl.: C07D 471/04, A61K 31/437

(54) **COMPOSES PYRROLO[2,3-B]PYRIDINE, COMPOSES AZAINDOLES UTILES DANS LA SYNTHESE DE CES COMPOSES PYRROLO[2,3-B]PYRIDINE, LEURS PROCEDES DE FABRICATION ET LEURS UTILISATIONS**
PYRROLO-[2,3-B]-PYRIDIN-VERBINDUNGEN, AZAINDOL-VERBINDUNGEN ZUR SYNTHESE DIESER PYRROLO-[2,3-B]-PYRIDIN-VERBINDUNGEN, VERFAHREN ZU IHRER HERSTELLUNG UND IHRE ANWENDUNG
PYRROLO[2,3-B]PYRIDINE COMPOUNDS, AZAINDOLE COMPOUNDS USED FOR SYNTHESIZING SAID PYRROLO[2,3-B]PYRIDINE COMPOUNDS, METHODS FOR THE PRODUCTION THEREOF, AND USES THEREOF

(30) Priorité: 16.02.2007 FR 0701138
(43) Date de publication de la demande: 02.12.2009
(73) Titulaire: Centre National de la Recherche Scientifique, 75016 Paris (FR); Université Pierre et Marie Curie (Paris VI), 75252 Paris Cedex 05 (FR); UNIVERSITE CLAUDE BERNARD - LYON 1, 69100 Villeurbanne (FR)
(72) Inventeur: MEIJER, Laurent, F-29680 Roscoff (FR); JOSEPH,Benoît, F-69100 Villeurbanne (FR); LIGER, François, F-69100 Villeurbanne (FR); MARQUET, Bernard, F-69300 Caluire (FR)
(74) Mandataire: Tanty, François
(86) Numéro de dépôt international: PCT/FR2008/000197
(87) Numéro de publication internationale: WO 2008/129152

(56) Documents cités:
- WO-A-2006/050076
- WO-A-2006/124863
- WO-A-2007/062998
- DAVID FERNÁNDEZ ET AL.: "Synthesis of Polyheterocyclic Nitrogen-Containing Marine Natural Products" MONATSHEFTE FÜR CHEMIE, vol. 135, 2004, pages 615-627, XP002452550
- STEWART W. SCHNELLER AND JIANN-KUAN LUO: "Synthesis of 4-Amino-1 H-pyrrolo[2,3-b]pyridine (1,7Dideazaadenine) and 1H-Pyrrolo[2,3-b]pyridin-4-ol (1,7-Dideazahypoxanthine)" JOURNAL OF ORGANIC CHEMISTRY, vol. 45, 1980, pages 4045-4048, XP002452551
- ALEXEI.S. KARPOV ET AL.: "Concise Synthesis of Meridianins by Carbonylative Alkynylation and a Four-Component Pyrimidine Synthesis" ANGEWANDTE CHEMIE, INTERNATIONAL EDITION, vol. 44, 2005, pages 6951-6956, XP002452552
- GOMPEL M ET AL: "Meridianins, a new family of protein kinase inhibitors isolated from the Ascidian Aplidium meridianum" BIOORGANIC & MEDICINAL CHEMISTRY LETTERS, OXFORD, GB, vol. 14, 2004, pages 1703-1707, XP002367721 ISSN: 0960-894X

## Description

L'invention concerne des composés pyrrolo[2,3-*b*]pyridine ainsi que des composés azaindoles utiles dans la synthèse de ces composés pyrrolo[2,3-*b*]pyridine. Elle concerne également un procédé de fabrication de ces composés pyrrolo[2,3-*b*]pyridine ainsi que l'utilisation de ces composés pyrrolo[2,3-*b*]pyridine.

La phosphorylation des protéines est le mécanisme le plus utilisé par la cellule pour moduler l'activité de ses protéines structurales et de ses enzymes. La phosphorylation des résidus sérine, thréonine ou tyrosine est catalysée par une vaste famille d'enzymes, les protéines kinases. Il n'y a pas d'événement physiologique important qui n'implique pas des modifications de la phosphorylation de protéines. De même la très vaste majorité des pathologies humaines implique des anomalies de la phosphorylation souvent associées à des anomalies de la régulation de certaines protéine kinases.

Des efforts croissants ont été portés en recherche cette dernière décade, sur l'optimisation et l'évaluation thérapeutique d'inhibiteurs pharmacologiques, de faibles poids moléculaires, de nombreuses protéines kinases.

Actuellement environ 60 inhibiteurs de kinases sont en évaluation clinique contre les cancers, l'inflammation, les diabètes et les maladies neurodégénératives.

Parmi les 518 kinases humaines, les kinases dépendantes de la cycline (CDKs) attirent un intérêt considérable en raison de leur implication dans de nombreux processus physiologiques essentiels et de multiples maladies humaines, spécialement les cancers, la polykystose rénale et les maladies neurodégénératives telles que la maladie d'Alzheimer, la maladie de Parkinson et les accidents vasculaires cérébraux.

Par conséquent de nombreux inhibiteurs pharmacologiques des CDKs ont été décrits et démontrés comme ayant des activités anti-tumorales, et/ou antiprolifératives, et/ou neuroprotectrices prometteuses.

Certains, comme le flavopiridol, la R-roscovitine, le SNS-032, sont en évaluation clinique en tant que médicaments anti-cancéreux.

Tous les inhibiteurs de CDKs identifiés jusqu'ici agissent par compétition avec l'ATP pour la liaison au site catalytique de leur cible kinase. Beaucoup ont été co-cristallisés avec une cible CDK. La sélectivité de ces inhibiteurs pharmacologiques est l'objet d'une recherche intensive en utilisant une grande variété de méthodes, telles que la recherche de sélectivité sur un vaste échantillon de kinases, la chromatographie d'affinité sur un inhibiteur immobilisé et la technique triple hybride chez la levure.

Alors que quelques inhibiteurs de kinases sont plutôt non sélectifs, comme la staurosporine, beaucoup présentent un profil de spécificité défini. Mais tous inhibent plusieurs kinases.

Certains ciblent également des kinases non protéiques inattendues. Ces inhibiteurs multi-cibles peuvent trouver une utilisation médicale appropriée, car ils sont moins susceptibles d'entraîner un phénomène de résistance.

Parmi les 518 kinases humaines, se trouve la famille des DYRK.

Le gène de la protéine kinase DYRK1A est localisé dans une région bien spécifique du chromosome 21, la « Down syndrome critical région », qui couvre une vingtaine de gènes responsables du phénotype trisomique. De nombreux arguments soutiennent l'hypothèse d'une contribution essentielle de la surexpression, même modeste (X 1.5), de DYRK1A dans le développement anormal du cerveau observé au cours de la trisomie 21. De plus DYRK1A semble également très impliquée dans la maladie d'Alzheimer (qui d'ailleurs apparaît chez les trisomiques 21 de façon systématique et précoce au-delà de la quarantaine). DYRK1A appartient a une petite famille de kinases comprenant 5 membres (DYRK1a, 1B, 2, 3, 4). DYRK1A agit comme "priming kinase" pour GSK-3, elle phosphoryle des protéines de la maladie d'Alzheimer telles que Tau et CRMP. L'inhibition conjointe de CDKs, GSK-3, CK1 et DYRK pourrait constituer un avantage majeur dans le traitement de maladies neurodégénératives.

On a récemment identifié les méridianines, une famille de 3-(2-aminopyrimidine)indoles en tant que structures inhibitrices de kinases prometteuses. Les méridianines sont des produits naturels initialement extraits de *Aplidium meridianum,* une ascidie de l'atlantique sud. Des dérivés de méridianines ont été synthétisés par divers groupes de chercheurs. Bien que certaines méridianines inhibent diverses kinases telles que les CDKs, la synthase kinase-3 (GSK-3), les kinases dépendant de nucléotides cycliques et les caséine kinases 1 (CK1), elles présentent des effets anti-prolifératifs significatifs mais modestes.

Les méridianines partagent une certaine similarité structurelle avec les variolines, une autre famille de composés marins naturels contenant un noyau central pyridopyrrolopyrimidine substitué par un cycle 2-aminopyrimidine. Les variolines ont été initialement extraites de *Kirkpatrickia variolosa,* une éponge de l'antarctique, rare et difficile d'accès. Elles ont été ultérieurement synthétisées. La varioline B et la désoxyvarioline B (PM01218) présentent une cytotoxicité puissante contre plusieurs lignes de cellules cancéreuses humaines. Récemment, la varioline B et la désoxyvarioline B ont été reportées comme inhibant les CDKs.

Les analogues de varioline ont fait l'objet de recherches intensives.

Ces analogues de varioline seront appelés ci-après mériolines. Il s'agit de composés pyrrolo[2,3-b]pyridine.

Ainsi, la demande de brevet WO 2006/050076 divulgue de nombreux composés pyrrolyl fusionnés substitués par un cycle pyrimidinyle utiles dans le traitement des désordres liés aux kinases.

Bien que de nombreux composés mériolines et leurs procédés de synthèse soient décrits dans ce document, il n'y a cependant aucune divulgation de leurs propriétés pharmacologiques, et en particulier d'une quelconque activité antiproliférative associée ou non à une inhibition vis-à-vis des kinases dépendantes des cyclines.

La demande de brevet WO 2006/124863 divulgue également de nombreux composés mériolines présentés comme utilisables pour traiter ou prévenir des maladies ou désordres associés à une activité kinase anormale ou dérégulée, plus particulièrement les maladies ou désordres qui impliquent une activation anormale, en particulier, des CDKs.

Cependant aucune étude de l'activité de ces composés n'est divulguée dans ce document.

La demande de brevet WO 2005/095400 divulgue un très grand nombre de composés azaindoles qui sont présentés comme inhibiteurs des protéines kinases.

Mais une fois encore, outre le fait que cette demande de brevet couvre un très grand nombre de composés, aucun résultat démontrant l'activité effective de ces composés n'est divulgué.

Le document « Synthesis of Polyheterocyclic Nitrogen-Containing Marine Natural Products », Monatsh. Chem. 2004, 135, 615-627, divulgue des mériolines, dont la mérioline appelée mérioline 12 dans ce qui suit, dans lesquelles le substituant sur le cycle pyrimidine est un ²radical -SMe, de formule :

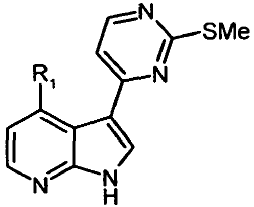

Cependant, ce document enseigne que ces composés n'ont aucune activité antitumorale significative.

Le document « Synthesis of the indole alkaloids meridianins form the tunicate Aplidium meridianum», Tetrahedron 2001, 57, 2355-2363, divulgue une mérioline, appelée dans la suite du texte mérioline 1, comportant un substituant NH₂ sur le cycle pyrimidine, de formule:

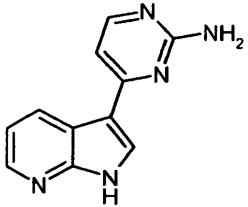

Cependant aucun résultat de tests d'activité biologique de cette molécule n'est décrit.

Le document « Concise Synthesis of Meridianins by Carbonylative Alkynylation and a Four-Component Pyrimidine Synthesis », Angew. Chem. Int. Ed., 2005, 44, 6951-6956, divulgue également la mérioline 1 en indiquant que ce composé inhibe les kinases testées, c'est-à-dire hSGK1, Tie-2, VEGFR2/KDR, PDGF-récepteur β kinase, Meck-EE kinase et IGF1-tyrosine kinase, à des niveaux micromolaires et même nanomolaires.

Plus précisément, ce composé, que l'on appellera ci-après mérioline 1, a une valeur IC₅₀ de 2,4 µM pour la protéine kinase hSGK1.

Ainsi, dans l'art antérieur les composés mériolines sont décrits d'une manière générale, en rapport avec leurs procédés de synthèse, mais aucune activité biologique particulière de ces composés n'est divulguée.

Or, on a maintenant découvert qu'une série particulière de ces composés mériolines présente des activités d'inhibition puissantes envers les kinases CDKs et spécialement CDK9 et d'autres kinases telles que GSK-3 et CK1 et qu'ils sont des agents antiprolifératifs et pro-apoptotiques dans les cultures cellulaires, en raison de leur structure particulière.

En particulier, une relation structure/activité a été mise en évidence pour cette famille d'inhibiteurs agissant dans la poche de liaison d'ATP des kinases. L'efficacité pro-apoptotique de ces mériolines est le mieux mise en évidence par leur activité d'inhibition des CDK9.

Ainsi, l'invention concerne les composés de formule I suivante : 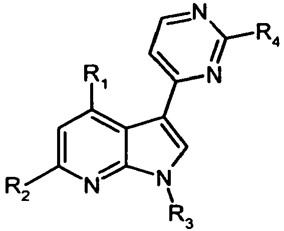 dans laquelle :
- R₁ est choisi parmi un halogène ou un groupe alkyle en C₁-C₁₀ substitué ou non substitué, un groupe (alkyle en C₁-C₁₀)cycloalkyle en C₅-C₈, un groupe (alkyle en C₁-C₁₀)aryle en C₆-C₁₈, un groupe (alkyle en C₁-C₁₀)hétérocyle en C₅-C₁₂ aromatique ou non aromatique comportant un à trois hétéroatomes, un groupe alcoxy en C₁-C₁₀ substitué ou non substitué, un groupe fluoroalcoxy en C₁-C₁₀, un groupe (alcoxy en C₁-C₁₀)alcoxy en C₁-C₁₀, un groupe cycloalcoxy en C₅-C₈, un groupe (alcoxy en C₁-C₁₀)cycloalkyle en C₅-C₈, un groupe (alcoxy en C₁-C₁₀)aryle en C₆-C₁₈, un groupe (alcoxy en C₁-C₁₀)hétérocyle en C₅-C₁₂ aromatique ou non aromatique comportant un à trois hétéroatomes, un groupe alcényle en C₂-C₁₀ substitué ou non substitué, un groupe (alcényle en C₂-C₁₀)cycloakyle en C₅-C₈, un groupe (alcényle en C₂-C₁₀)aryle en C₆-C₁₈, un groupe (alcényle en C₂-C₁₀)hétérocycle en C₅-C₁₂ aromatique ou non aromatique comportant un à trois hétéroatomes, un groupe alcynyle en C₂-C₁₀ substitué ou non substitué, un groupe (alcynyle en C₂-C₁₀)cycloalkyle en C₅-C₈, un groupe (alcynyle en C₂-C₁₀)aryle en C₆-C₁₈, un groupe (alcynyle en C₂-C₁₀)hétérocyle en C₅-C₁₂ aromatique ou non aromatique comportant un à trois hétéroatomes, -OH, -OCORₐ, -CN, -NO₂ -SRₐ, - NRₐR_{b},-NHCORₐ, -NHSO₂Rₐ,-NHSO₂Rₐ,-NHCONRₐR_{b}, NHCO₂Rₐ, un groupe phényle, un groupe aryle en C₆-C₁₈, un groupe hétérocycle en C₅-C₁₂ aromatique ou non aromatique comportant de un à trois hétéroatomes,
- R₂ représente un atome d'hydrogène ou d'halogène, ou indépendamment de R₁, un groupement tel que défini pour R₁,
- R₃ représente H ou un groupe -SO₂Rₐ ou -CORₐ, ou alkyle en C₁-C₁₀,
- R₄ représente un atome d'hydrogène ou un groupe NH₂,
- Rₐ et R_{b} représentent chacun indépendamment l'un de l'autre un atome d'hydrogène, ou un groupe éventuellement substitué choisi parmi un alkyle en C₁-C₁₀, un alcényle en C₂-C₁₀, un alcynyle en C₂-C₁₀, un cycloalkyle en C₅-C₈, un (cycloalkyle en C₅-C₈)alkyle en C₁-C₁₀, un (cycloalkyle en C₅-C₈)alcényle en C₂-C₁₀, un (cycloalkyle en C₅-C₈)alcynyle en C₂-C₁₀, un (hétérocycle en C₅-C₁₂)alkyle en C₁-C₁₀, un (hétérocycloalkyle en C₅-C₁₂)alcényle en C₂-C₁₀, un (hétérocycloalkyle en C₅-C₁₂)alcynyle en C₂-C₁₀, ou bien Rₐ et R_{b} sont liés entre eux pour former, avec l'atome d'azote auquel ils sont liés, un hétérocycle éventuellement substitué choisi parmi un groupement pyrrolidinyle, pipéridinyle, pipérazinyle et morpholinyle,
et les sels pharmaceutiquement acceptables de ces composés de formule I.

De préférence, dans les composés de formule I
- R₁ est choisi parmi un halogène ou un groupe alkyle en C₁-C₁₀, alcoxy en C₁-C₁₀ non substitué ou substitué, fluoroalcoxy en C₁-C₁₀, alcoxy en C₁-C₁₀-alcoxy en C₁-C₁₀, cycloalcoxy en C₅-C₈, -OH, -OCORₐ, -CN, -NO₂, -SRₐ, -NRₐR_{b}, - NHCORₐ, -NHSO₂Rₐ, -NHCONRₐR_{b}, NHCO₂Rₐ, phényle, aryle, hétéroaryle,
- R₂ représente un atome d'hydrogène ou indépendamment de R₁, un halogène ou un groupe tel que défini pour R₁,
- R₃ représente H ou un groupe -SO₂Rₐ ou -CORₐ, ou alkyle,
- R₄ représente un atome d'hydrogène ou un groupe NH₂,
- Rₐ et R_{b} représentent chacun indépendamment l'un de l'autre un atome d'hydrogène, ou un groupe éventuellement substitué choisi parmi les groupes alkyle, alcényle, alcynyle, cycloalkyle, cycloalkylalkyle, cycloalkylalcényle, cycloalkylalcynyle, hétérocycloalkyle, hétérocycloalkylalkyle, hétérocycloalkylalcényle, ou bien Rₐ et R_{b} sont liés entre eux pour former, avec l'atome d'azote auquel ils sont liés, un hétérocycle éventuellement substitué choisi parmi un groupement pyrrolidinyle, pipéridinyle, pipérazinyle ou morpholinyle.

En effet, on a maintenant découvert que pour que les mériolines aient une activité antiproliférative et/ou apoptotique vis-à-vis des cellules, R₃ devait être H ou un groupe -SO₂Rₐ ou -CORₐ, ou alkyle.

De préférence, dans la formule R₃ est H ou un groupe alkyle.

Le plus préférablement, R₃ est H.

On a également découvert que la nature du substituant R₁ sur le cycle pyridine était important pour l'activité inhibitrice des kinases dépendantes des cyclines c'est-à-dire pour l'activité antiproliférative et/ou pour l'activité apoptotique des composés de l'invention et, donc, pour leur activité en particulier antitumorale.

Plus préférablement, R₁ est choisi parmi un groupe OH, Cl, méthoxy, éthoxy, propoxy, butyloxy, isopropoxy, benzyloxy, cyclohéxylméthoxy, cylcohéxyloxy, 2-propyléthynyle, 2-butyléthynyle, 2-cyclohéxyléthynyle, phénéth-1-ynyle, phényle, pentyle ou phényléthyle.

Le plus préférablement, R₁ est choisi dans le groupe formé par un groupe méthoxy, éthoxy, propoxy, isopropoxy, benzyloxy, cyclohexylméthyloxy, cyclohexyloxy, 2-propyléthynyle,pentyle, phényle et phényléthyle.

En effet, les tests effectués avec les mériolines de l'invention sur cinq lignées de cellules de neuroblastomes SH-SY5Y et HEK293 en comparaison à l'effet de la varioline B, de la mérioline 1 et de la mérioline 12, décrites dans l'art antérieur, démontrent un effet particulièrement puissant des mériolines de l'invention, et en particulier des mériolines appelées ci-après mériolines 3, 4, 5, 6, 15, 16, 17, 18, 19, 22 et 23.

L'effet des composés testés sur les protéines kinases CK1 et CDK9 démontre leur exceptionnel effet antiprolifératif entraînant la mort cellulaire, en particulier des cellules tumorales, et l'effet des mériolines testées sur les protéines kinases CDK5, GSK3 et CDK1 démontre leur effet neuroprotecteur, comme cela sera montré dans les exemples qui suivent.

Les composés de l'invention ont été obtenus par quatre procédés qui tous utilisent en tant que composés de départ les composés de formule II suivante :

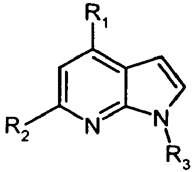

Dans ces composés, les substituants R₁, R₂ et R₃ sont ceux définis pour les composés de formule I, comme étant préférés.

Ces composés permettent d'obtenir en 1 à 5 étapes les mériolines de l'invention.

Ainsi, les mériolines de l'invention peuvent être utilisées à titre de médicament, en particulier pour tous les désordres liés à une activité anormale des kinases dépendantes des cyclines. En particulier, les mériolines de l'invention peuvent être utilisées pour la fabrication d'un médicament pour le traitement des désordres liés à une prolifération anormale des cellules, cancéreuses ou non, ou en tant que neuroprotecteur c'est-à-dire, pour traiter en particulier les tumeurs, les maladies neurodégénératives telles que la maladie d'Alzheimer et la trisomie 21, les leucémies, les maladies rénales (glomérulonéphrites, polykystose rénale), les inflammations et les diabètes de type II. Elles peuvent être également utilisées pour des applications anti-parasitaires.

L'invention sera mieux comprise et d'autres avantages et caractéristiques de celle-ci apparaîtront plus clairement à la lueur de la description explicative qui suit qui est faite en référence à des exemples donnés à titre purement illustratif et non limitatif et aux figures dans lesquelles :
- la Figure 1 représente la diminution de la survie des cellules induite par différentes concentrations de mériolines selon l'invention, en comparaison à celle induite par la varioline B, aux mêmes concentrations.
- la Figure 2 représente le pourcentage de mort cellulaire induite par différentes concentrations de mériolines selon l'invention, en comparaison à celle induite par la varioline B, aux mêmes concentrations, et
- la Figure 3 représente le volume tumoral moyen d'une tumeur exposée à une mérioline selon l'invention, en comparaison à celui d'une tumeur exposée à un composé de contrôle, c'est-à-dire non traitée.

Dans la description qui suit, les abréviations utilisées ont les significations suivantes
CDK : kinase dépendante des cyclines,
CK1 : caséine kinase,
DYRK1A : kinase à double spécificité activée par phosphorylation sur tyrosine (dual-specificity tyrosine phosphorylation activated kinase),
FCS : sérum de veau foetal,
GSK3 : glycogène synthase kinase-3,
LDH : lactate déhydrogénase,
MTS : 3-(4,5-diméthylthiazol-2-yl)-5-(3-carboxyméthoxyphényl)-2-(4-sulfophényl)-2*H*-tétrazolium,
PBS : solution saline tamponnée sulfate,
DMSO : diméthylsulfoxyde.

### I) SYNTHESE DES MERIOLINES DE L'INVENTION.

Les mériolines de l'invention ont été synthétisées par les quatre procédés schématisés au schéma 1 suivant :

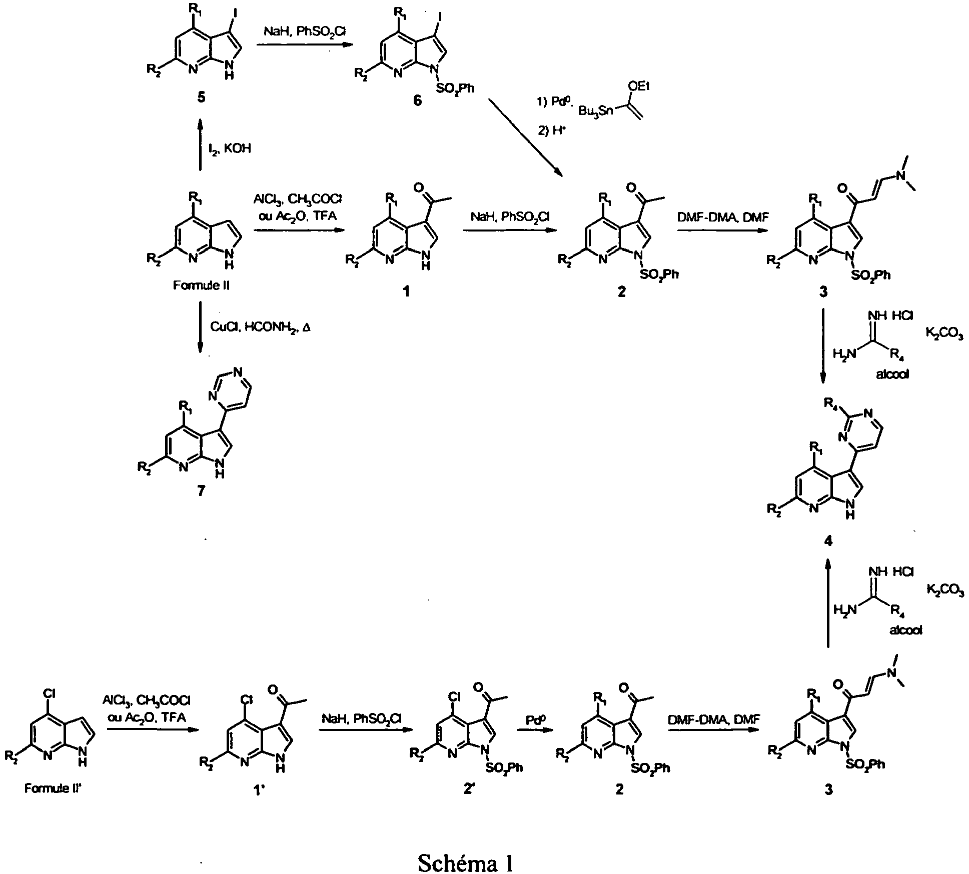

Dans le schéma 1, le composé noté Formule II' est un composé de Formule II dans laquelle R₁ est le chlore

### Exemple 1: Synthèse des composés de formule II

Les composés de départ de formule II ont été synthétisés de la façon suivante.

La réaction de Mitsunobu sur le dérivé 4-hydroxy-7-azaindole (J. Heterocyclic Chem. 1989, 26, 317-325) en présence de divers alcools conduit à l'obtention des 7-azaindoles O-alkylés en position 4.

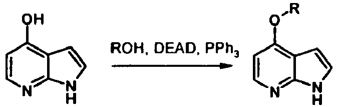

### 4-éthoxy-1H-pyrrolo[2,3-b]pyridine

A température ambiante et sous atmosphère inerte, l'azodicarboxylate de diéthyle (DEAD) (520 µL, 3,3 mmol) est ajouté goutte à goutte à une solution de PPh₃ (1,04 g, 3,96 mmol) dans du THF anhydre (13 mL). Sous atmosphère inerte, cette solution est canulée dans un ballon contenant une solution de 4-hydroxy-7-azaindole (222 mg, 1,65 mmol), d'éthanol (115 µL, 1,98 mmol) dans le tétrahydrofurane (THF) anhydre (41 mL). La solution est agitée à température ambiante pendant 2 h. Le solvant est évaporé. Le résidu obtenu est purifié par colonne de chromatographie (éluant: CH₂Cl₂/MeOH 98:2) pour donner le composé désiré (214 mg, 80%). Solide; PF = 176-178 °C (Et₂O); ¹H RMN (300 MHz, CDCl₃) δ 1,52 (t, 3H, J = 7,1 Hz, CH₃), 4,26 (q, 2H, J = 7,1 Hz, CH₂), 6,53 (d, 1H, J = 5,6 Hz, Hₐᵣₒₘ), 6,59 (s large, 1H, Hₐᵣₒₘ), 7,18 (s large, 1H, Hₐᵣₒₘ), 8,18 (d, 1H, J = 5.6 Hz, Hₐᵣₒₘ), 9,64 (s large, 1H, NH); SM (IS) m/z 163 (M + H⁺).

### 4-propoxy-1H-pyrrolo[2,3-b]pyridine

Suivant la procédure décrite pour la préparation du 4-éthoxy-1*H-*pyrrolo[2,3-*b*]pyridine, le 4-propoxy-1*H*-pyrrolo[2,3-b]pyridine est obtenu avec un rendement de 78% à partir de 4-hydroxy-7-azaindole et de propanol. Solide; PF = 189-191 °C (Et₂O); ¹H RMN (300 MHz, CDCl₃) δ 1,10 (t, 3H, J = 7,4 Hz, CH₃), 1,88-1,99 (m, 2H, CH₂), 4,20 (t, 2H, J = 6,6 Hz, CH₂), 6,59 (d, 1H, J = 5,8 Hz, Hₐᵣₒₘ), 6,63 (d, 1H, J = 3,6 Hz, Hₐᵣₒₘ), 7,21 (d, 1H, J = 3,6 Hz, Hₐᵣₒₘ), 8,15 (d, 1H, J = 5.8 Hz, Hₐᵣₒₘ); SM (IS) m/z 177 (M + H⁺).

### 4-(1-méthyléthoxy)-1H-pyrrolo[2,3-b]pyridine

Suivant la procédure décrite pour la préparation du 4-éthoxy-1*H* pyrrolo[2,3-*b*]pyridine, le 4-(1-méthyléthoxy)-1*H*-pyrrolo[2,3-*b*]pyridine est obtenu avec un rendement de 76% à partir de 4-hydroxy-7-azaindole et de 2-propanol. Solide; PF = 182-184 °C (Et₂O); ¹H RMN (300 MHz, CDCl₃) δ 1,44 (d, 6H, J = 6,0 Hz, 2 CH₃), 4,82 (hept, 1H, J = 6,0 Hz, CH), 6,52 (d, 1H, J = 5,7 Hz, Hₐᵣₒₘ), 6,57 (d, 1H, J = 3,6 Hz, Hₐᵣₒₘ), 7,19 (d, 1H, J = 3,6 Hz, Hₐᵣₒₘ), 8,17 (d, 1H, J = 5.7 Hz, Hₐᵣₒₘ), 10,70 (s large, 1H, NH); SM (IS) m/z 177 (M + H⁺).

### 4-(benzyloxy)-1H-pyrrolo[2,3-b]pyridine

Suivant la procédure décrite pour la préparation du 4-éthoxy-1*H-*pyrrolo[2,3-*b*]pyridine, le 4-(benzyloxy)-1*H*-pyrrolo[2,3-*b*]pyridine est obtenu avec un rendement de 61% à partir de 4-hydroxy-7-azaindole et d'alcool benzylique. Solide; ¹H RMN (300 MHz, CDCl₃) δ 5,46 (s, 2H, CH₂), 6,78 (d, 1H, J = 3,6 Hz, Hₐᵣₒₘ), 6,85 (d, 1H, J = 6,9 Hz, Hₐᵣₒₘ), 7,35 (d, 1H, J = 3,6 Hz, Hₐᵣₒₘ), 7,40-7,50 (m, 5H, Hₐᵣₒₘ), 8,10 (d, 1H, J = 6,9 Hz, Hₐᵣₒₘ), 11,78 (s large, 1H, NH). SM (IS): m/z 225 (M + H⁺).

### 4-(cydohexylméthoxy)-1H-pyrrolo[2,3-b]pyridine

Suivant la procédure décrite pour la préparation du 4-éthoxy-1*H-*pyrrolo[2,3-*b*]pyridine, le 4-(cyclohexylméthoxy)-1*H*-pyrrolo[2,3-*b*]pyridine est obtenu avec un rendement de 52% à partir de 4-hydroxy-7-azaindole et de cyclohexylméthanol. Solide; ¹H RMN (300 MHz, CDCl₃) δ 1,06-1,40 (m, 5H, CH₂), 1,71-1,95 (m, 6H, CH + CH₂), 3,99 (d, 2H, J = 6,0 Hz, CH₂), 6,54 (d, 1H, J = 5,6 Hz, Hₐᵣₒₘ), 6,60 (d, 1H, J = 3,4 Hz, Hₐᵣₒₘ), 7,20 (d, 1H, J = 3,4 Hz, Hₐᵣₒₘ), 8,18 (d, 1H, J = 5,6 Hz, Hₐᵣₒₘ), 10,61 (s large, 1H, NH). SM (IS): m/z 231 (M + H⁺).

### 4-(cydohexyl)-1H-pyrrolo[2,3-b]pyridine

Suivant la procédure décrite pour la préparation du 4-éthoxy-1*H-*pyrrolo[2,3-*b*]pyridine, le 4-(cyclohexyl)-1*H*-pyrrolo[2,3-*b*]pyridine est obtenu avec un rendement de 49% à partir de 4-hydroxy-7-azaindole et de cyclohexanol. Solide; ¹H RMN (300 MHz, CDCl₃): δ 1,30-1,48 (m, 3H, CH₂), 1,60-1,71 (m, 3H, CH₂), 1,85 (s large, 2H, CH₂), 2,04 (s large, 2H, CH₂), 4,54-4,61 (m, 1H, CH), 6,57 (d, 1H, J = 5,8 Hz, Hₐᵣₒₘ), 6,60 (d, 1H, J = 3,5 Hz, Hₐᵣₒₘ), 7,20 (d, 1H, J = 3,5 Hz, Hₐᵣₒₘ), 8,14 (d, 1H, J = 5,8 Hz, Hₐᵣₒₘ), 10,33 (s large, 1H, NH); SM (IS): m/z 217 (M + H⁺).

Les 7-azaindoles de formule II O-alkylés en position 4 (alcoxy en C₁-C₁₀, fluoroalcoxy en C₁-C₁₀, alcoxy substitué en C₁-C₁₀, cycloalcoxy en C₅-C₈, benzyloxy, aryloxy, hétéroaryloxy, hétéroarylalkyloxy) ont été préparés selon cette méthode.

Ainsi, les composés de formule II selon l'invention ont été obtenus.

A partir des composés de formule II, le premier procédé d'obtention des mériolines de l'invention consiste à effectuer une réaction d'acylation en présence de chlorure d'aluminium (AlCl₃), de CH₃COCl comme décrit dans J. Org. Chem. 2002, 67, 6226-6227 ou de Ac₂O et d'acide trifluoroacétique sur les composés de formule II substitués sur la position 4 et/ou la position 6, ce qui conduit à l'obtention du composé noté 1 au schéma 1.

Ensuite une réaction de benzènesulfonylation sur l'azote N-1 indolique des dérivés 1 est réalisée en milieu basique en présence de chlorure de benzènesulfonyle pour donner les composés notés 2 au schéma 1.

Puis les énaninones notées 3 au schéma 1 sont obtenues par réaction des composés 2 avec du DMF-DMA dans du DMF selon la méthode décrite dans Tetrahedron 2001, 57, 2355-2363.

La formation du cycle pyrimidique substitué est réalisée, quant à elle, en traitant le composé **3** en présence de chlrohydrate de guanidinium ou de ses dérivés.

Le composé final noté **4** au schéma 1 (non protégé sur l'azote N-1 indolique) est obtenu avec un bon rendement.

Le second procédé d'obtention des mériolines de l'invention diffère du procédé 1 par l'étape d'obtention des composés 2. Dans ce deuxième procédé, l'iodation sur la position 3 des composés de formule II substitués sur la position 4 et/ou sur la position 6 est conduite en présence de diiode en milieu basique pour donner les dérivés notés 5 au schéma 1. La réaction de benzènesulfonylation sur l'azote N-1 indolique des dérivés 5 donne les composés notés 6 au schéma 1. Ces derniers sont engagés dans une réaction de couplage catalysée par le palladium (réaction de Stille) en présence tri-n-butyl(1-éthoxyvinyl)stannane pour donner les composés 2.

Ensuite à partir de ces composés 2, on obtient les composés 4 par les mêmes étapes que pour le premier procédé décrit.

Le troisième procédé permet d'obtenir les mériolines dans lesquelles le substituant en R₄ sur le cycle pyrimdine est H. Le composé noté 7 au schéma 1 est obtenu directement à partir des composés de formule II par traitement avec CuO et du formamide.

Le quatrième procédé d'obtention des mériolines de l'invention diffère des procédés 1 et 2 par l'étape d'obtention des composés 2. Dans ce quatrième procédé, la fonctionnalisation de la position 4 du 7-azaindole est réalisée à partir de 7-azaindoles de formule II' substitués par un chlore en position 4. Cette étape est réalisée par des réactions catalysées par le palladium (réaction de Sonogashira, réaction de Stille, réaction de Suzuki-Myaura, réaction de Heck,...) pour conduire aux composés 2.

Ensuite à partir de ces composés 2, les composés 4 sont obtenus par les mêmes étapes que pour le premier procédé décrit.

### Exemple 2: Synthèse de la 3-[(2-amino)pyrimidin-4-yl]-4-chloro-1H-pyrrolo[2,3-b]pyridine (4a) : mérioline 10.

### a) 3-acétyl-4-chloro-1H-pyrrolo[2,3-b]pyridine (1a)

*Méthode A:* A température ambiante et sous atmosphère inerte, le chlorure d'aluminium (1,70 g, 12,78 mmol) est ajouté à une solution de 4-chloro-7-azaindole (0,32 g, 2,13 mmol) dans le CH₂Cl₂ anhydre (15 mL). La solution est agitée à température ambiante pendant 90 min. Le chlorure d'acétyle (0,91 mL, 12,78 mmol) est additionné goutte à goutte à température ambiante. La solution finale est agitée pendant 5 jours à température ambiante. Après addition de MeOH, les solvants sont évaporés. Le résidu obtenu est repris dans un mélange NaOH 1 N et AcOEt (200 mL 1: 1) puis les deux phases sont séparées. La phase organique recueillie est séchée sur MgSO₄ puis évaporée. Le solide obtenu est purifié par colonne de chromatographie (éluant: AcOEt) pour donner le composé **1a** (320 mg, 77%).

*Méthode B:* Une solution de 4-chloro-7-azaindole (90 mg, 0,59 mmol), d'anhydride acétique (0,13 mL, 1,8 mmol) et d'acide trifluoroacétique (5 mL) est chauffée à reflux pendant 8 h. Après refroidissement, une solution saturée de Na₂CO₃ est additionnée pour neutraliser le milieu (pH = 7-8). Après addition d'AcOEt, agitation et décantation, les phases sont séparées. La phase organique est séchée sur MgSO₄ puis évaporée. Le résidu est purifié par colonne de chromatographie (éluant: AcOEt) pour donner 1a (98 mg, 85%). FP > 210 °C (MeOH); ¹H RMN (300 MHz, CDCl₃) δ 2,63 (s, 3H, CH₃), 7,41 (d, 1H, J = 5,6 Hz, Hₐᵣₒₘ), 8,12 (s, 1H, Hₐᵣₒₘ), 8,27 (d, 1H, J = 5,6 Hz, Hₐᵣₒₘ); SM (IS) m/z 195 (M + H⁺)

### b) 3-acétyl-1-benzènesulfonyl-4-chloro-1H-pyrrolo[2,3-b]pyridine (2a)

A 0 °C, l'hydrure de sodium (26 mg, 0,64 mmol, 60% dans l'huile) est additionné par petites portions à une solution de composé **1a** (125 mg, 0,64 mmol) dans le THF anhydre (20 mL). La solution est agitée à 0 °C pendant 45 min, puis le chlorure de benzènesulfonyle (0,11 mL, 0,83 mmol) est ajouté au mélange réactionnel. La solution finale est agitée à température ambiante pendant 4 h. L'addition d'H₂O est réalisée à 0 °C, puis les solvants sont évaporés. Le résidu obtenu est repris dans un mélange H₂O et AcOEt (50 mL, 1:1) puis les deux phases sont séparées. La phase organique recueillie est séchée sur MgSO₄ puis évaporée. Le solide obtenu est purifié par colonne de chromatographie (éluant: éther de pétrole/AcOEt 8:2) pour donner le composé **2a** (200 mg, 93%). PF = 160-162 °C (CH₂Cl₂/pentane); ¹H RMN (300 MHz, CDCl₃) δ 2,63 (s, 3H, CH₃), 7,30 (d, 1H, J = 5,2 Hz, Hₐᵣₒₘ), 7,54 (t large, 2H, J = 7,4 Hz, Hₐᵣₒₘ), 7,66 (t large, 1H, J = 7,4 Hz, Hₐᵣₒₘ), 8,25 (d large, 2H, J = 7,4 Hz, Hₐᵣₒₘ), 8,32 (d, 1H, J = 5,2 Hz, Hₐᵣₒₘ), 8,34 (s, 1H, Hₐᵣₒₘ), SM (IS) m/z 335 (M + H⁺).

### c) 1-(1-benzènesulfonyl-4-chloro-1H-pyrrolo[2,3-b] pyridin-3-yl)-3,3-diméthylaminopropénone (3a)

Sous atmosphère inerte, une solution de **2a** (450 mg, 1,34 mmol) et de DMF-DMA (1,07 mL, 8,4 mmol) dans le DMF anhydre (15 mL) est agitée à 90 °C pendant 8 h. Après refroidissement, le solvant est évaporé. Le résidu obtenu est repris dans un mélange H₂O et AcOEt (50 mL 1:1) puis les deux phases sont séparées. La phase organique recueillie est séchée sur MgSO₄ puis évaporée. Le solide obtenu est purifié par colonne de chromatographie (éluant: AcOEt) pour donner le composé **3a** (310 mg, 60%). PF = 139-141 °C (MeOH); ¹H RMN (300 MHz, CDCl₃) δ 2,93 (s large, 3H, CH₃), 3,15 (s large, 3H, CH₃), 5,49 (d, 1H, J = 12,6 Hz, =CH), 7,23 (d, 1H, J = 5,3 Hz, Hₐᵣₒₘ), 7,49 (t, 2H, J = 7,4 Hz, Hₐᵣₒₘ), 7,47-7,65 (m, 2H, Hₐᵣₒₘ + =CH), 8,00 (s, 1H, Hₐᵣₒₘ), 8,19 (d, 2H, J = 7,7 Hz, Hₐᵣₒₘ), 8,29 (d, 1H, J = 5,3 Hz, Hₐᵣₒₘ); SM (IS) m/z 390 (M + H⁺).

### d) 3-[(2-amino)pyrimidin-4-yl]-4-chloro-1H-pyrrolo[2,3-b]pyridine (4a)

Une solution de **3a** (140 mg, 0,36 mmol), de chlorhydrate de guanidinium (52 mg, 0,54 mmol) et de K₂CO₃ (106 mg, 0,76 mmol) dans le 2-méthoxyéthanol (5 mL) est chauffée à 100-110 °C pendant 36 h. Après refroidissement, la solution est jetée dans de l'eau. La solution finale est extraite par de l'AcOEt (2x). La phase organique combinée est séchée sur MgSO₄, puis évaporée. Le résidu est purifié par colonne de chromatographie (CH₂Cl₂/MeOH 95:5) pour donner **4a** (45 mg, 51%). PF > 210 °C (MeOH); ¹H RMN (300 MHz, DMSO-d₆) δ 6,48 (s large, 2H, NH₂), 6,85 (d, 1H, J = 5,3 Hz, Hₐᵣₒₘ), 7,26 (d, 1H, J = 5,3 Hz, Hₐᵣₒₘ), 7,94 (s, 1H, Hₐᵣₒₘ), 8,20 (d, 1H, J = 5,3 Hz, Hₐᵣₒₘ), 8,22 (d, 1H, J = 5,3 Hz, Hₐᵣₒₘ), 12,49 (s large, 1H, NH), SM (IS) m/z 246 (M + H⁺).

### Exemple 3: Synthèse de la 3-[(2-amino)pyrimidin-4-yl]-4-méthoxy-1H-pyrrolo[2,3-b]pyridine (4b) : mérioline 3.

### a) 3-acétyl-4-méthoxy-1H-pyrrolo[2,3-b]pyridine (1b)

Suivant la procédure décrite pour la préparation de **1a**, le composé **1b** est obtenu avec un rendement de 55% à partir du 4-méthoxy-7-azaindole. PF > 210 °C (MeOH); ¹H RMN (300 MHz, DMSO-d₆) δ 2,50 (s, 3H, CH₃), 3,92 (s, 3H, CH₃), 6,79 (d, 1H, J = 5,6 Hz, Hₐᵣₒₘ), 8,11 (s, 1H, Hₐᵣₒₘ), 8,17 (d, 1H, J = 5,6 Hz, Hₐᵣₒₘ), 12,36 (s large, 1H, NH); SM (IS) m/z 191 (M + H⁺).

### b) 3-acétyl-1-benzènesulfonyl-4-méthoxy-1H-pyrrolo[2,3-b]pyridine (2b)

Suivant la procédure décrite pour 1a préparation de **2a**, le composé **2b** est obtenu avec un rendement de 90% à partir de **1b**. PF = 156-158 °C (CH₂Cl₂/pentane); ¹H RMN (300 MHz, CDCl₃) δ 2,63 (s, 3H, CH₃), 3,98 (s, 3H, CH₃), 6,73 (d, 1H, J = 5,7 Hz, Hₐᵣₒₘ), 7,51 (t large, 2H, J = 7,4 Hz, Hₐᵣₒₘ), 7,62 (t large, 1H, J = 7,4 Hz, Hₐᵣₒₘ), 8,21 (s, 1H, Hₐᵣₒₘ), 8,24 (d large, 2H, J = 7,4 Hz, Hₐᵣₒₘ), 8,34 (d, 1H, J = 5,7 Hz, Hₐᵣₒₘ); SM (IS) m/z 331 (M + H⁺).

### c) 1-(1-benzènesulfonyl-4-méthoxy-1H-pyrrolo[2,3-b]pyridin-3-yl)-3,3-diméthylaminopropénone (3b)

Suivant la procédure décrite pour la préparation de **3a**, le composé **3b** est obtenu avec un rendement de 78% à partir de **2b**. PF = 210-212 °C (MeOH); ¹H RMN (300 MHz, CDCl₃) δ 2,91 (s large, 3H, CH₃), 3,12 (s large, 3H, CH₃), 3,94 (s, 3H, CH₃), 5,62 (d, 1H, J = 12,6 Hz, =CH), 6,68 (d, 1H, J = 5,7 Hz, Hₐᵣₒₘ), 7,47 (t, 2H, J = 7,4 Hz, Harom), 7,57 (t, 1H, J = 7,4 Hz, Hₐᵣₒₘ), 7,65 (d large, 1H, J = 12,6 Hz, =CH), 7,96 (s, 1H, Hₐᵣₒₘ), 8,18 (d, 2H, J = 7,4 Hz, Hₐᵣₒₘ), 8,31 (d, 1H, J = 5,7 Hz, Harom); SM (IS) m/z 386 (M + H⁺).

### d) 3-[(2-amino)pyrimidin-4-yl]-4-méthoxy-1H-pyrrolo[2,2-b]pyridine (4b)

Suivant la procédure décrite pour la préparation de **4a**, le composé **4b** est obtenu avec un rendement de 75% à partir de **3b**. PF > 210 °C (MeOH); ¹H RMN (300 MHz, DMSO-d₆) δ 3,97 (s, 3H, CH₃), 6,32 (s large, 2H, NH₂), 6,79 (d, 1H, J = 5,5 Hz, Hₐᵣₒₘ), 7,27 (d, 1H, J = 5,3 Hz, Hₐᵣₒₘ), 7,92 (s, 1H, Hₐᵣₒₘ), 8,16 (d, 1H, J = 5,5 Hz, Harom), 8,17 (d, 1H, J = 5,3 Hz, Hₐᵣₒₘ), 12,13 (s large, 1H, NH); SM (IS) m/z 242 (M + H⁺).

### Exemple 4: Synthèse de la 3-[(2-amino)pyrimidin-4-yl]-4-éthoxy-1H-pyrrolo[2,3-b]pyridine (4c) : mérioline 4.

### a) 3-acétyl-4-éthoxy-1H-pyrrolo[2,3-b]pyridine (1c)

Suivant la procédure décrite pour la préparation de **1a**, le composé **1c** est obtenu avec un rendement de 84% à partir de 4-éthoxy-7-azaindole. PF > 210 °C (MeOH); ¹H RMN (300 MHz, DMSO-d₆) δ 1,43 (t, 3H, J = 7,2 Hz, CH₃), 2,54 (s, 3H, CH₃), 4,22 (q, 2H, J = 7,2 Hz, CH₂), 6,78 (d, 1H, J = S,7 Hz, Hₐᵣₒₘ), 8,04 (s, 1H, Hₐᵣₒₘ), 8,14 (d, 1H, J = 5,7 Hz, Hₐᵣₒₘ), 12,33 (s large, 1H, NH) ; SM (IS) m/z 205 (M + H⁺).

### b) 3-acétyl-1-benzènesulfonyl-4-éthoxy-1H-pyrrolo[2,3-b]pyridine (2c)

Suivant la procédure décrite pour la préparation de 2a, le composé 2c est obtenu avec un rendement de 68% à partir de **1c**. PF = 151-153 °C (CH₂Cl₂/pentane); ¹H RMN (300 MHz, CDCl₃) δ 1,52 (t, 3H, J = 7,2 Hz, CH₃), 2,65 (s, 3H, CH₃), 4,21 (q, 2H, J = 7,2 Hz, CH₂), 6,70 (d, 1H, J = 5,7 Hz, Hₐᵣₒₘ), 7,51 (t large, 2H, J = 7,4 Hz, Hₐᵣₒₘ), 7,61 (t large, 1H, J = 7,4 Hz, Hₐᵣₒₘ), 8,20 (s, 1H, Hₐᵣₒₘ), 8,23 (d large, 2H, J = 7,4 Hz, Hₐᵣₒₘ), 8,31 (d, 1H, J = 5,7 Hz, Hₐᵣₒₘ); SM (IS) m/z 345 (M + H⁺).

### c) 1-(1-benzènesulfonyl-4-éthoxy-1H-pyrrolo[2,3-b]pyridin-3-yl)-3,3-diméthylaminopropénone (3c)

Suivant la procédure décrite pour la préparation de **3a**, le composé **3c** est obtenu avec un rendement de 68% à partir de **2c**. PF = 187-189 °C (MeOH); ¹H RMN (300 MHz, CDCl₃) δ 1,44 (t, 3H, J = 7,2 Hz, CH₃), 2,90 (s large, 3H, CH₃), 3,11 (s large, 3H, CH₃), 4,18 (q, 2H, J = 7,2 Hz, CH₂), 5,60 (d, 1H, J = 12,6 Hz, =CH), 6,65 (d, 1H, J = 5,8 Hz, Hₐᵣₒₘ), 7,46 (t, 2H, J = 7,1 Hz, Harom), 7,55-7,63 (m, 2H, Hₐᵣₒₘ + =CH), 7,94 (s, 1H, Hₐᵣₒₘ), 8,18 (d, 2H, J = 7,7 Hz, Hₐᵣₒₘ), 8,29 (d, 1H, J = 5,6 Hz, Hₐᵣₒₘ); SM (IS) m/z 400 (M + H⁺).

### d) 3-[(2-amino)pyrimidin-4-yl]-4-éthoxy-1H-pyrrolo[2,3-b]pyridine (4c)

Suivant la procédure décrite pour la préparation de **4a**, le composé **4c** est obtenu avec un rendement de 63% à partir de **3c**. PF > 210 °C (MeOH); ¹H RMN (300 MHz, DMSO-d₆) δ 1,45 (t, 3H, J = 7,2 Hz, CH₃), 4,26 (q, 2H, J = 7,2 Hz, CH₂), 6,33 (s large, 2H, NH₂), 6,75 (d, 1H, J = 5,5 Hz, Hₐᵣₒₘ), 7,36 (d, 1H, J = 5,3 Hz, Hₐᵣₒₘ), 7,90 (s, 1H, Hₐᵣₒₘ), 8,13 (d, 1H, J = 5,5 Hz, Hₐᵣₒₘ), 8,17 (d, 1H, J = 5,3 Hz, Hₐᵣₒₘ), 12,09 (s large, 1H, NH); SM (IS) m/z 256 (M + H⁺).

### Exemple 5: Synthèse de la 3-[(2-amino)pyrimidin-4-yl]-4-propoxy-1H-pyrrolo[2,3-b]pyridine (4d) : mérioline 5.

### a) 3-acétyl-4-propoxy-1H-pyrrolo[2,3-b]pyridine (1d)

Suivant la procédure décrite pour la préparation de **1a**, le composé **1d** est obtenu avec un rendement de 80% à partir de 4-propoxy-7-azaindole. PF > 210 °C (MeOH); ¹H RMN (300 MHz, DMSO-d₆) δ 1,07 (t, 3H, J = 7,3 Hz, CH₃), 1,77-1,88 (m, 2H, CH₂), 2,53 (s, 3H, CH₃), 4,11 (t, 2H, J = 6,3 Hz, CH₂), 6,78 (d, 1H, J = 5,6 Hz, Hₐᵣₒₘ), 8,08 (s, 1H, Hₐᵣₒₘ), 8,14 (d, 1H, J = 5,6 Hz, Hₐᵣₒₘ), 12,32 (s large, 1H, NH); SM (IS) m/z 219 (M + H⁺).

### b) 3-acétyl-1-benzènesulfonyl-4-propoxy-1H-pyrrolo[2,3-b]pyridine (2d)

Suivant la procédure décrite pour la préparation de **2a**, le composé **2d** est obtenu avec un rendement de 70% à partir de **1d**. PF = 119-121 °C (CH₂Cl₂/pentane); ¹H RMN (300 MHz, CDCl₃) δ 1,09 (t, 3H, J = 7,3 Hz, CH₃), 1,87-1,96 (m, 2H, CH₂), 2,64 (s, 3H, CH₃), 4,09 (t, 2H, J = 6,6 Hz, CH₂), 6,70 (d, 1H, J = 5,7 Hz, Hₐᵣₒₘ), 7,51 (t large, 2H, J = 7,4 Hz, Hₐᵣₒₘ), 7,61 (t large, 1H, J = 7,4 Hz, Hₐᵣₒᵣₙ), 8,19 (s, 1H, Hₐᵣₒₘ), 8,23 (d large, 2H, J = 7,4 Hz, Hₐᵣₒₘ), 8,31 (d, 1H, J = 5,7 Hz, Hₐᵣₒₘ); SM (IS) m/z 359 (M + H⁺)

### c) 1-(1-benzènesulfonyl-4-propoxy-1H-pyrrolo[2,3-b]pyridin-3-yl)-3,3-diméthylaminopropénone(3d)

Suivant la procédure décrite pour 1a préparation de **3a**, le composé **3d** est obtenu avec un rendement de 69% à partir de **2d**. PF = 102-104 °C (CH₂Cl₂/pentane); ¹H RMN (300 MHz, CDCl₃) δ 1,02 (t, 3H, J = 7,3 Hz, CH₃), 1,77-1,89 (m, 2H, CH₂), 2,90 (s large, 3H, CH₃), 3,10 (s large, 3H, CH₃), 4,05 (q, 2H, J = 6,4 Hz, CH₂), 5,59 (d, 1H, J = 12,6 Hz, =CH), 6,64 (d, 1H, J = 5,6 Hz, Hₐᵣₒₘ), 7,46 (t, 2H, J = 7,4 Hz, Hₐᵣₒₘ), 7,56 (t large, 1H, J = 7,4 Hz, Hₐᵣₒₘ), 7,62 (d large, 1H, J = 12,6 Hz, =CH), 7,91 (s, 1H, Hₐᵣₒₘ), 8,18 (d, 2H, J = 7,4 Hz, Hₐᵣₒₘ), 8,28 (d, 1H, J = 5,6 Hz, Hₐᵣₒₘ); SM (IS) m/z 414 (M + H⁺).

### d) 3-[(2-amino)pyrimidin-4-yl]-4-propoxy-1H-pyrrolo[2,3-b]pyri-dine (4d)

Suivant la procédure décrite pour la préparation de **4a**, le composé **4d** est obtenu avec un rendement de 60% à partir de **3d**. PF > 210 °C (MeOH); ¹H RMN (300 MHz, DMSO-d₆) δ 1,01 (t, 3H, J = 7,3 Hz, CH₃), 1,81-1,90 (m, 2H, CH₂), 4,16 (t, 2H, J = 6,2 Hz, CH₂), 6,32 (s large, 2H, NH₂), 6,76 (d, 1H, J = 5,5 Hz, Hₐᵣₒₘ), 7,34 (d, 1H, J = 5,3 Hz, Hₐᵣₒₘ), 7,90 (s, 1H, Hₐᵣₒₘ), 8,14 (d, 1H, J = 5,5 Hz, Hₐᵣₒₘ), 8,16 (d, 1H, J = 5,3 Hz, Hₐᵣₒₘ), 12,08 (s, 1H, NH); SM (IS) m/z 270 (M + H⁺).

### Exemple 6 : Synthèse de la 3-[(2-amino)pyrimidin-4-yl]-4-(cyclohexylméthoxy)-1H-pyrrolo[2,3-b]pyridine (4e) : mérioline 16

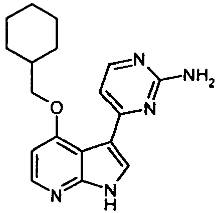

### Mérioline 16

### a) 3-acétyl-4-(cyclohexylméthoxy)-1H-pyrrolo[2,3-b]pyridine (4e)

Suivant la procédure décrite pour la préparation de **1a**, le composé **1e** est obtenu avec un rendement de 99% à partir de 4-cyclohexylméthoxy-7-azaindole. PF > 210 °C (MeOH); ¹H RMN (300 MHz, DMSO-d₆) δ 1,08-1,34 (m, 5H, CH₂), 1,65-1,94 (m, 6H, CH₂), 2,51 (s, 3H, CH₃), 3,95 (d, 2H, J = 6,0 Hz, CH₂), 6,76 (d, 1H, J = 5,6 Hz, Hₐᵣₒₘ), 8,10 (s, 1H, Hₐᵣₒₘ), 8,13 (d, 1H, J = 5,6 Hz, Hₐᵣₒₘ), 12,30 (s large, 1H, NH). SM (IS) m/z 273 (M + H⁺).

### b) 3-acétyl-1-benzènesulfonyl-4-(cyclohexylméthoxy)-1H-pyrrolo(2,3-b]pyridine (2e)

Suivant la procédure décrite pour la préparation de **2a**, le composé **2e** est obtenu avec un rendement de 87% à partir de **1e**. PF = 138-140 °C (MeOH); ¹H RMN (300 MHz, CDCl₃) δ 1,00-1,34 (m, 5H, CH₂), 1,65-1,88 (m, 6H, CH₂), 2,61 (s, 3H, CH₃), 3,89 (d, 2H, J = 6,0 Hz, CH₂), 6,68 (d, 1H, J = 5,8 Hz, Hₐᵣₒₘ), 7,45 (t, 1H, J = 7,9 Hz, Hₐᵣₒₘ), 7,57 (t, 2H, J = 7,4 Hz, Hₐᵣₒₘ), 8,16 (s, 1H, Hₐᵣₒₘ), 8,20 (d, 2H, J = 7,5 Hz, Hₐᵣₒₘ), 8,27 (d, 1H, J = 5,6 Hz, Hₐᵣₒₘ); SM (IS) m/z 413 (M + H⁺).

### c) 1-(1-benzènesulfonyl)-4-(cyclohexylméthoxy)-1H-pyrrolo[2,3-b] pyridin-3-yl)-3,3-diméthylaminopropénone (3e)

Suivant la procédure décrite pour la préparation de **3a**, le composé **3e** est obtenu avec un rendement de 73% à partir de **2e**. PF = 102-104 °C (MeOH); ¹H RMN (300 MHz, CDCl₃) δ 0,97-1,25 (m, 5H, CH₂), 1,65-1,79 (m, 6H, CH + CH₂), 2,83 (s large, 3H, CH₃), 3,04 (s large, 3H, CH₃), 3,82 (d, 2H, J = 5,6 Hz, CH₂), 5,48 (d, 1H, J = 12,6 Hz, =CH), 6,60 (d, 1H, J = 5,6 Hz, Hₐᵣₒₘ), 7,41 (t, 2H, J = 7,5 Hz, Hₐᵣₒₘ), 7,49-7,53 (m, 2H, =CH + Hₐᵣₒₘ), 7,82 (s, 1H, Hₐᵣₒₘ), 8,13 (d large, 2H, J = 8,1 Hz, Hₐᵣₒₘ), 8,22 (d, 1H, J = 5,6 Hz, Hₐᵣₒₘ); SM (IS) m/z 468 (M + H⁺).

### d) 3-[(2-amino)pyrimidin-4-yl]-(4-cyclohexylméthoxy)-1H-pyrrolo[2,3-b] pyridine (4e)

Suivant la procédure décrite pour la préparation de **4a,** le composé **4e** est obtenu avec un rendement de 72% à partir de **3e.** PF > 210 °C (MeOH); ¹H RMN (300 MHz, DMSO-d₆) δ 1,07-1,30 (m, 5H, CH₂), 1,65-1,83 (m, 6H, CH + CH₂), 4,01 (d, 2H, J = 5,7 Hz, CH₂), 6,34 (s large, 2H, NH₂), 6,75 (d, 1H, J = 5,6 Hz, Hₐᵣₒₘ), 7,32 (d, 1H, J = 5,.3 Hz, Harom), 7,87 (s, 1H, Hₐᵣₒₘ), 8,11-8,14 (m, 2H, Hₐᵣₒₘ), 12,08 (s large, 1H, NH); SM (IS) m/z 324 (M + H⁺).

### Exemple 7: Synthèse de la 3-[(2-amino)pyrimidin-4-yl]-4-phényl-1H-pyrrolo[2,3-b]pyridine (4f) : mérioline 23

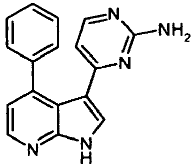

### Mérioline 23

### a) 3-acétyl-4-phényl-1H-pyrrolo[2,3-b]pyridine (1f)

Suivant la procédure décrite pour la préparation de **1a,** le composé **1f** est obtenu avec un rendement de 74% à partir de 4-phényl-7-azaindole *(Synlett* **2001,** 609). PF > 210 °C (MeOH); ¹H RMN (300 MHz, DMSO-d₆) δ 2,28 (s, 3H, CH₃), 7,13 (d, 1H, J = 4,9 Hz, Hₐᵣₒₘ), 7,28-7,41 (m, 5H, Hₐᵣₒₘ), 8,35 (d, 1H, J = 4,9 Hz, Hₐᵣₒₘ), 8,44 (s, 1H, Hₐᵣₒₘ), 12,55 (s large, 1H, NH); SM (IS) m/z 237.

### b) 3-acétyl-1-benzènesulfonyl-4-phényl-1H-pyrrolo[2,3-b]pyridine (2f)

Suivant la procédure décrite pour la préparation de **2a,** le composé **2f** est obtenu avec un rendement de 71% à partir de **1f.** PF = 138-140 °C (CH₂Cl₂/pentane); ¹H RMN (300 MHz, CDCl₃) δ 2,19 (s, 3H, CH₃), 7,24 (d, 1H, J = 4,9 Hz, Hₐᵣₒₘ), 7,26-7,31 (m, 2H, Hₐᵣₒₘ), 7,42-7,44 (m, 3H, Hₐᵣₒₘ), 7,55 (t, 2H, J = 7,5 Hz, Hₐᵣₒₘ), 7,66 (t, 1H, J = 7,5 Hz, Hₐᵣₒₘ), 8,29-8,32 (m, 3H, Hₐᵣₒₘ), 8,49 (d, 1H, J = 4,9 Hz, Hₐᵣₒₘ); SM (IS) m/z 377.

### c) 1-(1-benzènesulfonyl)-4-phényl-1H-pyrrolo[2,3-b]pyridin-3-yl)-3,3-diméthylaminopropénone (3f)

Suivant la procédure décrite pour la préparation de **3a,** le composé **3f** est obtenu avec un rendement de 65% à partir de **2f.** PF = 179-181 °C (MeOH); ¹H RMN (300 MHz, CDCl₃) δ 2,59 (s large, 3H, CH₃), 3,01 (s large, 3H, CH₃), 4,83 (d large, 1H, J = 12,4 Hz, =CH), 7,29 (d, 1H, J = 4,9 Hz, Hₐᵣₒₘ), 7,7,30-7,40 (m, 6H =CH + Hₐᵣₒₘ), 7,59 (t, 2H, J = 7,6 Hz, Hₐᵣₒₘ), 7,69 (t, ] H, J = 7,6 Hz, Hₐᵣₒₘ), 8,12 (s large, 1H, Hₐᵣₒₘ), 8,23 (d, 2H, J = 7,6 Hz, Hₐᵣₒₘ), 8,41 (d, 1H, J = 4,9 Hz, Hₐᵣₒₘ); SM (IS) m/z 432 (M + H⁺).

### d) 3-[(2-amino)pyrimidin-4-yl]-4-phényl-1H-pyrrolo[2,3-b]pyridine (4e) : mérioline 23

Suivant la procédure décrite pour la préparation de **4a,** le composé **4f** est obtenu avec un rendement de 78% à partir de **3f.** PF > 210 °C (MeOH); ¹H RMN (300 MHz, DMSO-d₆) δ 5,76 (d, 1H, J = 5,3 Hz, Hₐᵣₒₘ), 6,03 (s large, 2H, NH₂), 7,12 (d, 1H, J = 4,9 Hz, Harom), 7,25-7,35 (m, 5H, Hₐᵣₒₘ), 7,65 (d, 1H, J = 5,3 Hz, Hₐᵣₒₘ₎, 7,94 (s, 1H, Hₐᵣₒₘ), 8,34 (d, 1H, J = 4,9 Hz, Hₐᵣₒₘ), 12,30 (s large, 1H, NH); SM (IS) m/z 288.

De la même manière, les composés, ci-dessous, ont été préparés à partir de 7-azaindoles substitués en position 4 ou/et 6.
- 3-[(2-amino)pyrimidin-4-yl]-butoxy-1*H*-pyrrolo[2,3-*b*]pyridine
- 3-[(2-amino)pyrimidin-4-yl]-4-pentoxy-1*H*-pyrrolo[2,3-*b*]pyridine
- 3-[(2-amino)pyrimidin-4-yl]-4,3,3,3-trifluoropropoxy)-1*H*-pyrrolo[2,3-*b*]pyridine
- 3-[(2-amino)pyrimidin-4-yl]-4-(3-fluoropropoxy)-1*H*-pyrrolo[2,3-*b*]pyridine
- 3-[(2-amino)pyrimidin-4-yl]-4-(2,2,2-trifluoroéthoxy)-1*H*-pyrolo[2,3-*b*]pyridine
- 3-[(2-amino)pyrimidin-4-yl]-4-(2-fluoroéthoxy)-1*H*-pyrrolo[2,3-*b*]pyridine
- 3-[(2-amino)pyrimidin-4-yl]-4-méthylthio-1*H*-pyrrolo[2,3-*b*]pyridine
- 3-[(2-amino)pyrimidin-4-yl]-4-éthylthio-1*H*-pyrrolo[2,3-*b*]pyridine
- 3-[(2-amino)pyrimidin-4-yl]-4-propylthio-1*H*-pyrrolo[2,3-*b*]pyridine
- 3-[(2-amino)pyrimidin-4-yl]-4-benzylthio-1*H*-pyrrolo[2,3-*b*]pyridine
- 3-[(2-amino)pyrimidin-4-yl]-4-nitro-1*H*-pyrrolo[2,3-*b*]pyridine
- 3-[(2-amino)pyrimidin-4-yl]-4-méthyl-1*H*-pyrrolo[2,3-*b*]pyridine
- 3-[(2-amino)pyrimidin-4-yl]-4-éthyl-1*H*-pyrrolo[2,3-*b*]pyridùie
- 3-[(2-amino)pyrimidin-4-yl]-4-propyl-1*H*-pyrrolo[2,3-*b*]pyridine
- 3-[(2-amino)pyrimidin-4-yl]-4-butyl-6-acétylamino-1*H*-pyrrolo[2,3-*b*]pyridine
- 3-[(2-amino)pyrimidin-4-yl]-4-pentyl-6-phénylamino-1*H*-pyrrolo[2,3-*b*]pyridine
- 3-[(2-amino)pyrimidin-4-yl]-4-pentyl-6-benzylamino-1*H*-pyrrolo[2,3-*b*]pyridine
- 3-[(2-amino)pyrimidin-4-yl]-4-pentyl-6-benzyloxy-1*H*-pyrrolo[2,3-*b*]pyridine
- 3-[(2-amino)pyrimidin-4-yl]-4-cyano-1*H*-pyrrolo[2,3-*b*]pyridine
- 3-[(2-amino)pyrimidin-4-yl]-1*H*-pyrolo[2,3-*b*]pyridine-4-carboxylate de méthyle
- 4-amino-3-[(2-amino)pyrimidin-4-yl]-1*H*-pyrrolo[2,3-*b*]pyridine
- 3-[(2-amino)pyrimidin-4-yl]-4-diméthylamino-1*H*-pyrrolo[2,3-*b*]pyridine
- 3-[(2-amino)pyrimidin-4-yl]-4-propylamino-1*H*-pyrrolo[2,3-*b*]pyridine
- 3-[(2-amino)pyrimidin-4-yl]4-benzylamino-1*H*-pyrrolo[2,3-*b*]pyridine
- 3-[(2-amino)pyrimidin-4-yl]4-trifluorométhyl-1*H*-pyrrolo[2,3-*b*]pyridine
- 3-[(2-amino)pyrimidin-4-yl]-4-méthylsulfonyl-1*H*-pyrrolo[2,3-*b*]pyridine
- 3-[(2-amino)pyrimidin-4-yl]-4-bromo-1*H*-pyrrolo[2,3-*b*]pyridine
- 3-[(2-amino)pyrimidin-4-yl]-4-fluoro-1*H*-pyrrolo[2,3-*b*]pyridine
- 3-[(2-amino)pyrimidin-4-yl]-4-propoxy-6-amino-1*H*-pyrrolo[2,3-*b*]pyridine
- 3-[(2-amino)pyrimidin-4-yl]-4-(4-méthoxyphényl)-1*H*-pyrrolo[2,3-*b*]pyridine
- 3-[(2-amino)pyrimidin-4-yl]-4-(4-chlorophényl)-1*H*-pyrrolo[2,3-*b*]pyridine
- 3-[(2-amino)pyrimidin-4-yl]-4-(4-fluorophényl)-1*H*-pyrrolo[2,3-*b*]pyridine
- 3-[(2-amino)pyrimidin-4-yl]-4-(3-fluorophényl)-1*H*-pyrrolo[2,3-*b*]pyridine
- 3-[(2-amino)pyrimidin-4-yl]-4-(3-chlorophényl)-1*H*-pyrrolo[2,3-*b*]pyridine
- 3-[(2-amino)pyrimidin-4-yl]-4-(3-méthoxyphényl)-1*H*-pyrrolo[2,3-*b*]pyridine
- 3-[(2-amino)pyrimidin-4-yl]-4-méthoxy-6-acétylamino-1*H*-pyrrolo[2,3-*b*]pyridine
- 3-[(2-amino)pyrimidin-4-yl]-4-méthoxy-6-phénylamino-1*H*-pyrolo[2,3-*b*]pyridine
- 3-[(2-amino)pyrimidin-4-yl]-4-éthoxy-6-acétylamino-1*H*-pyrrolo[2,3-*b*]pyridine
- 3-[(2-amino)pyrimidin-4-yl]-4-éthoxy-6-phénylamino-1*H*-pyrrolo[2,3-*b*]pyridine
- 3-[(2-amino)pyrimidin4-yl]-4-propoxy-6-acétylamino-1*H*-pyrrolo[2,3-*b*]pyridine
- 3-[(2-amino)pyrimidin-4-yl]-4-propoxy-6-phénylamino-1*H*pyrrolo[2,3-*b*]pyridine
- 3-[(2-amino)pyrimidin-4-yl]-4-méthoxy-6-benzylamino-1*H*-pyrrolo[2,3-*b*]pyridine
- 3-[(2-amino)pyrimidin-4-yl]-4-éthoxy-6-benzylamino-1*H*-pyrrolo[2,3-*b*]pyridine
- 3-[(2-amino)pyrimidin-4-yl]-4-propoxy-6-benzylamino-1*H*-pyrrolo[2,3-*b*]pyridine
- 3-[(2-amino)pyrimidin-4-yl]-4-méthoxy-6-benzyloxy-1*H*-pyrrolo[2,3-*b*]pyridine
- 3-[(2-amino)pyrimidin-4-yl]-4-éthoxy-6-benzyloxy-1*H*-pyrrolo[2,3-*b*]pyridine
- 3-[(2-amino)pyrimidin-4-yl]-4-propoxy-6-benzyloxy-1*H*-pyrrolo[2,3-*b*]pyridine
- 3-[(2-amino)pyrimidin-4-yl]-4-méthoxy-6-méthylamino-1*H*-pyrrolo[2,3-*b*]pyridine
- 3-[(2-amino)pyrimidin-4-yl]-4-éthoxy-6-méthylamino-1*H*-pyrrolo[2,3-*b*]pyridine
- 3-[(2-amino)pyrimidin-4-yl]-4-propoxy-6-méthylamino-1*H*-pyrrolo[2,3-*b*]pyridine
- 3-[(2-amino)pyrimidin-4-yl]-4-méthoxy-6-cyclohexylamino-1*H*pyrrolo[2,3-*b*]pyridine
- 3-[(2-amino)pyrimidin-4-yl]-4-éthoxy-6-cyclohexylamino-1*H*-pyrrolo[2,3-*b*]pyridine
- 3-[(2-amino)pyrimidin-4-yl]-4-propoxy-6-cyclohexylamino-1*H*-pyrrolo[2,3-*b*]pyridine
- 3-[(2-amino)pyrimidin-4-yl]-4-méthoxy-6-(pyridin-4-ylméthyl)amino-1*H*-pyrrolo[2,3-*b*]pyridine
- 3-[(2-amino)pyrimidin-4-yl]-4-éthoxy-6-(pyridin-4-ylméthyl)amino-1*H*-pyrrolo[2,3-*b*]pyridine
- 3-[(2-amino)pyrimidin-4-yl]-4-propoxy-6-(pyridin-4-ylméthyl)amino-1*H*-pyrrolo[2,3-*b*]pyridine
- 3-[(2-amino)pyrimidin-4-yl]-4-éthoxy-6-[(4-(phényl)benzyloxy]-1*H-*pyrrolo[2,3-*b*]pyridine
- 3-[(2-amino)pyrimidin-4-yl]-4-propoxy-6-[4-(phényl)benzyloxy]-1*H*-pyrrolo[2,3-*b*]pyridine
- 3-[(2-amino)pyrimidin-4-yl]-4-propoxy-6-[4-(2-pyridinyl)benzyloxy]-1*H*-pyrrolo[2,3-*b*]pyridine
- 3-[(2-amino)pyrimidin-4-yl]-4-propoxy-6-[4-(2-thiényl)benzyloxy]-1*H*-pyrrolo[2,3-*b*]pyridine
- 3-[(2-amino)pyrimidin-4-yl]-4-propoxy-6-[4-(2-thiényl)benzylamino]-1*H*-pyrrolo[2,3-*b*]pyridine

### Exemple 8 : Synthèse de la 3-[(2-amino)pyrimidin-4-yl]-4-(1-méthyléthoxy)-1H-pyrrolo[2,3-b]pyridine (4g) : mérioline 6

### a) iodo-4-(1-méthyléthoxy)-1H-pyrrolo[2,3-b]pyridine (5g)

A température ambiante et sous atmosphère inerte, une solution de diiode (74 mg, 10,29 mmol) dans le DMF anhydre (0,5 mL) est ajoutée goutte à goutte à une solution de 4-(1-méthyléthoxy)-1*H*-pyrrolo[2,3-b]pyridine (50 mg, 0.28 mmol) et de KOH (88 mg, 1,56 mmol) dans le DMF (0,5 mL). La solution est agitée à température ambiante pendant 2,5 h. Le solvant est évaporé, puis le résidu est repris dans l'H₂O. La suspension obtenue est filtrée pour donner le composé **5g** (70 mg, 82%). PF = 181-183 °C (MeOH); ¹H RMN (300 MHz, CDCl₃) δ 1,49 (d, 6H, J = 6,0 Hz, CH₃), 4,80 (hept, 1H, J = 6,0 Hz, CH), 6,55 (d, 1H, J = 5,8 Hz, Hₐᵣₒₘ), 7,26 (s, 1H, Hₐᵣₒₘ), 8,14 (d, 1H, J = 5,8 Hz, Hₐᵣₒₘ); SM (IS) m/z 303 (M + H⁺).

### b) 1-benzènesulfonyl-3-iodo-4-(1-méthyléthoxy)-1H-pyrrolo[2,3-b] pyridine (6g)

Suivant la procédure décrite pour la préparation de **2a**, le composé **6g** est obtenu avec un rendement de 68% à partir de **5g**. PF = 151-153 °C (CH₂Cl₂/pentane); ¹H RMN (300 MHz, CDCl₃) δ 1,42 (d, 6H, J = 6,2 Hz, CH₃), 4,71 (hept, 1H, J = 6,2 Hz, CH), 6,59 (d, 1H, J = 5,7 Hz, Hₐᵣₒₘ), 7,48 (t large, 2H, J = 7,9 Hz, Hₐᵣₒₘ), 7,58 (t large, 1H, J = 7,9 Hz, Hₐᵣₒₘ), 7,69 (s, 1H, Hₐᵣₒₘ), 8,17 (d large, 2H, J = 7,9 Hz, Hₐᵣₒₘ), 8,27 (d, 1H, J = 5,7 Hz, Hₐᵣₒₘ); SM (IS) m/z 443 (M + H⁺).

### c) 3-acétyl-1-(1-benzènesulfonyl)-4-(1-méthyléthoxy)-1H-pyrrolo [2,3-b]pyridine (2g)

A une solution de **6g** (67 mg, 0,15 mmol), de Pd(PPh₃)₄ fraîchement préparé (18 mg, 0,015 mmol) et de LiCl (16 mg, 0,38 mmol) dans le DMF anhydre (3 mL) est additionnée le tri-n-butyl(1-éthoxyvinyl)stannane (77 µL, 0,23 mmol). La solution est agitée à 80 °C pendant 18 h. Après refroidissement, une solution de HCl 5% (10 mL) est additionnée et la solution finale est agitée pendant 20 min à température ambiante. Les solvants sont évaporés sous pression réduite. Le résidu obtenu est repris dans un mélange d'une solution de Na₂CO₃ saturé et d'AcOEt (pH = 7-8) puis les deux phases sont séparées. La phase organique recueillie est lavée par une solution de KF, séchée sur MgSO₄ puis évaporée. Le solide obtenu est purifié par colonne de chromatographie (éluant: éther de pétrole/AcOEt 7:3) pour donner le composé **2g** (48 mg, 89%). PF = 118-120 °C (CH₂Cl₂/éther de pétrole); ¹H RMN (300 MHz, CDCl₃) δ 1,43 (d, 6H, J = 6,0 Hz, CH₃), 2,65 (s, 3H, CH₃), 4,78 (hept, 1H, J = 6,0 Hz, CH), 6,69 (d, 1H, J = 6,0 Hz, Hₐᵣₒₘ), 7,51 (t large, 2H, J = 7,9 Hz, Hₐᵣₒₘ), 7,61 (t large, 1H, J = 7,9 Hz, Hₐᵣₒₘ), 8,17 (s, 1H, Hₐᵣₒₘ), 8,23 (d large, 2H, J = 7,9 Hz, Hₐᵣₒₘ), 8,29 (d, 1H, J = 6,0 Hz, Hₐᵣₒₘ); SM (IS) m/z 359 (M + H⁺).

### d) 1-(1-benzènesulfonyl)-4-(1-méthyléthoxy)-1H-pyrrolo[2,3-b] pyridin-3-yl)-3,3-diméthylaminopropénone (3g)

Suivant la procédure décrite pour la préparation de **3a**, le composé **3g** est obtenu avec un rendement de 69% à partir de **2g**. PF = 150-152 °C (MeOH); ¹H RMN (300 MHz, CDCl₃) δ 1,37 (d, 6H, J = 6,0 Hz, CH₃), 2,95 (s large, 3H, CH₃), 3,14 (s large, 3H, CH₃), 4,72 (hept, 1H, J = 6,0 Hz, CH), 5,68 (d large, 1H, J = 12,5 Hz, =CH), 6,65 (d, 1H, J = 5,7 Hz, Hₐᵣₒₘ), 7,47 (t, 2H, J = 7,5 Hz, Hₐᵣₒₘ), 7,57 (t, 1H, J = 7,5 Hz, Hₐᵣₒₘ), 7,73-7,79 (m, 1H, =CH), 7,98 (s, 1H, Hₐᵣₒₘ), 8,20 (d, 2H, J = 7,5 Hz, Hₐᵣₒₘ), 8,28 (d, 1H, J = 5,7 Hz, Hₐᵣₒₘ); SM (IS) m/z 414 (M + H⁺).

### e) 3-[(2-amino)pyrimidin-4-yl]-4-(1-méthyléthoxy)-1H-pyrrolo[2,3-b]pyridine (4g)

Suivant la procédure décrite pour la préparation de **4a**, le composé **4g** est obtenu avec un rendement de 60% à partir de **3g**. PF > 210 °C (MeOH); ¹H RMN (300 MHz, DMSO-d₆) δ 1,39 (d, 6H, J = 6,0 Hz, CH₃), 4,91 (hept, 1H, J= 6,0 Hz, CH), 6,30 (s large, 2H, NH₂), 6,77 (d, 1H, J = 5,6 Hz, Hₐᵣₒₘ), 7,34 (d, 1H, J = 5,3 Hz, Hₐᵣₒₘ), 7,87 (s, 1H, Hₐᵣₒₘ), 8,11 (d, 1H, J = 5,6 Hz, Hₐᵣₒₘ), 8,17 (d, 1H, J = 5,3 Hz, Hₐᵣₒₘ), 12,03 (s large, 1H, NH); SM (IS) m/z 270 (M + H⁺).

### Exemple 9 : 3-[(2-amino)pyrimidin-4-yl]-4-(benzyloxy)-1H-pyrrolo[2,3-b] pyridine (4h) : mérioline 15

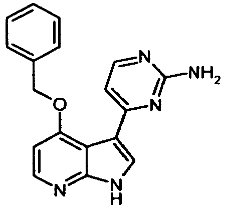

### Mérioline 15

### a) 4-(benzyloxy)-3-iodo-1H-pyrrolo[2,3-b]pyridine (5h)

Suivant la procédure décrite pour la préparation de **5g**, le composé **5h** est obtenu avec un rendement de 88% à partir de 4-(benzyloxy)-1*H*-pyrrolo[2,3-*b*]pyridine. PF = 180-182 °C (CH₂Cl₂); ¹H RMN (300 MHz, DMSO-d₆) δ 5,33 (s, 2H, CH₂), 6,77 (d, 1H, J = 5,3 Hz, Hₐᵣₒₘ), 7,30-7,44 (m, 3H, Hₐᵣₒₘ), 7,48 (s, 1H, Hₐᵣₒₘ), 7,62 (d large, 2H, J = 7,4 Hz, Hₐᵣₒₘ), 8,1 (d, 1H, J = 5,3 Hz, Hₐᵣₒₘ), 11,95 (s large, 1H, NH); SM (IS) m/z 351 (M + H⁺).

### b) 1-benzènesulfonyl-3-iodo-4-(benzyloxy)-1H-pyrrolo[2,3-b] pyridine (6h)

Suivant la procédure décrite pour la préparation de **5g**, le composé **6h** est obtenu avec un rendement de 77% à partir de **5h**. PF = 177-179 °C (CH₂Cl₂/pentane); ¹H RMN (300 MHz, CDCl₃) δ 5,23 (s, 2H, CH₂), 6.69 (d, 1H, J = 5,7 Hz, Hₐᵣₒₘ), 7,31-7,61 (m, 8H, Hₐᵣₒₘ), 7,73 (s, 1H, Hₐᵣₒₘ), 8,18 (d large, 2H, J = 7,9 Hz, Hₐᵣₒₘ), 8,29 (d, 1H, J = 5,7 Hz, Hₐᵣₒₘ); SM (IS) m/z 491 (M + H⁺).

### c) 3-acétyl-1-(1-benzènesulfonyl)-4-(benzyloxy)-1H-pyrrolo[2,3-b] pyridine (2h)

Suivant la procédure décrite pour la préparation de 2g, le composé 2g est obtenu avec un rendement de 86% à partir de **6g**. PF = 164-166 °C (CH₂Cl₂/EP); ¹H RMN (300 MHz, CDCl₃) δ 2,53 (s, 3H, CH₃), 5,23 (s, 2H, CH₂), 6,78 (d, 1H, J = 5,6 Hz, Hₐᵣₒₘ), 7,28-7,61 (m, 8H, Hₐᵣₒₘ), 8,20 (s, 1H, Hₐᵣₒₘ), 8,23 (d large, 2H, J = 7,5 Hz, Hₐᵣₒₘ), 8,29 (d, 1H, J = 5,6 Hz, Hₐᵣₒₘ); SM (IS) m/z 407 (M + H⁺).

### d) 1-(1-benzènesulfonyl-4-(benzyloxy)-1H-pyrrolo[2,3-b]pyridin-3-yl)-3-diméthylaminopropénone (3h).

Suivant la procédure décrite pour la préparation de **3a**, le composé **3h** est obtenu avec un rendement de 66% à partir de **2h**. PF = 190-192 °C (MeOH); ¹H RMN (300 MHz, CD₃OD) δ 2,56 (s large, 3H, CH₃), 3,06 (s large, 3H, CH₃), 5,27 (s, 2H, CH₂), 5,61 (d large, 1H, J = 11,5 Hz, =CH), 7,00 (d, 1H, J = 5,8 Hz, Hₐᵣₒₘ), 7,34-7,69 (m, 9H, =CH + Hₐᵣₒₘ), 7,99 (s, 1H, Hₐᵣₒₘ), 8,17 (d, 2H, J = 7,4 Hz, Hₐᵣₒₘ), 8,24 (d, 1H, J = 5,8 Hz, Hₐᵣₒₘ); SM (IS) m/z 462 (M + H⁺).

### e) 3-[(2-amino)pyrimidin-4-yl]-4-(benzyloxy)-1H-pyrrolo[2,3-b] pyridine (4h): mérioline 15

Suivant la procédure décrite pour la préparation de **4a**, le composé **4h** est obtenu avec un rendement de 90% à partir de **3h**. PF > 210 °C (MeOH); ¹H RMN (300 MHz, DMSO-d₆) δ 5,34 (s, 2H, CH₂), 6,29 (s large, 2H, NH₂), 6,90 (d, 1H, J = 5,5 Hz, Hₐᵣₒₘ), 7,23 (d, 1H, J = 5,3 Hz, Hₐᵣₒₘ), 7,37-7,45 (m, 3H, Hₐᵣₒₘ), 7,52-7,54 (m, 2H, Hₐᵣₒₘ), 7,85 (d, 1H, J = 5,3 Hz, Hₐᵣₒₘ), 7,90 (s, 1H, Hₐᵣₒₘ), 8,16 (d, 1H, J = 5,5 Hz, Hₐᵣₒₘ), 12,12 (s large, 1H, NH); SM (IS) m/z 318 (M + H⁺).

### Exemnle 10 : 3-[(2-amino)pyrimidin-4-yl]-4-(cyclohexyloxy)-1H-pyrrolo[2,3-b]pyridine (4i) : mérioline 17

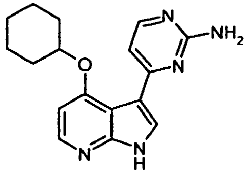

### Mérioline 17

### a) 4-(cyclohexyloxy)-3-iodo-1H-pyrrolo[2,3-b]pyridine (5h)

Suivant la procédure décrite pour la préparation de **5g**, le composé **5i** est obtenu avec un rendement de 82% à partir de 4-(cyclohexyloxy)-1*H*-pyrrolo[2,3-b]pyridine. PF = 199-201 °C (H₂O); ¹H RMN (300 MHz, CDCl₃) δ 1,40-1,60 (m, 5H, CH₂), 1,82-2,00 (m, 5H, CH₂), 4,67 (s large, 1H, CH), 6,62 (d, 1H, J = 6,1 Hz, Hₐᵣₒₘ), 7,30 (s, 1H, Hₐᵣₒₘ), 8,12 (d, 1H, J = 6,1 Hz, Hₐᵣₒₘ); SM (IS) m/z 343 (M + H⁺).

### b) 1-benzènesulfonyl-3-iodo-4-(cyclohexyloxy)-1H-pyrrolo[2,3-b] pyridine (6i)

Suivant la procédure décrite pour la préparation de **5g**, le composé **6i** est obtenu avec un rendement de 85% à partir de 5i. PF = 112-114 °C (CH₂Cl₂/pentane); ¹H RMN (300 MHz, CDCl₃) δ 1,40-1,60 (m, 5H, CH₂), 1,70-2,00 (m, 5H, CH₂), 4,52 (s large, 1H, CH), 6,60 (d, 1H, J = 5,7 Hz, Hₐᵣₒₘ), 7,48 (t, 2H, J = 7,2 Hz, Hₐᵣₒₘ), 7,58 (t, 1H, J = 7,7 Hz, Hₐᵣₒₘ), 7,69 (s, 1H, Hₐᵣₒₘ), 8,18 (d large, 2H, J = 8,0 Hz, Hₐᵣₒₘ), 8,26 (d, 1H, J = 5,7 Hz, Hₐᵣₒₘ); SM (IS) m/z 483 (M + H⁺).

### c) 3-acétyl-1-(1-benzènesulfonyl)-4-(cyclohexyloxy)-1H-pyrrolo[2,3-b]pyridine (2i)

Suivant la procédure décrite pour la préparation de **2g**, le composé **2i** est obtenu avec un rendement de 73% à partir de **6i**. PF = 107-109 °C (CH₂Cl₂/EP); ¹H RMN (300 MHz, CDCl₃) δ 1,29-1,44 (m, 3H, CH₂), 1,50-1,70 (m, 3H, CH₂), 1,80-1,90 (m, 2H, CH₂), 2,00-2,10 (m, 2H, CH₂), 2,64 (s, 3H, CH₃), 4,44-4,52 (m, 1H, CH), 6,70 (d, 1H, J = 5,7 Hz, Hₐᵣₒₘ), 7,49 (t, 2H, J = 7,4 Hz, Hₐᵣₒₘ), 7,59 (t, 1H, J = 7,2 Hz, Hₐᵣₒₘ), 8,15 (s, 1H, Hₐᵣₒₘ), 8,22 (d large, 2H, J = 7,7 Hz, Hₐᵣₒₘ), 8,27 (d, 1H, J = 5,7 Hz, Hₐᵣₒₘ); SM (IS) m/z 399 (M + H⁺).

### d) 1-(1-benzènesulfonyl-4-(cyclohexyloxy)-1H-pyrrolo[2,3-b]pyridin-3-yl)-3-diméthylaminopropénone (3i)

Suivant la procédure décrite pour la préparation de **3a**, le composé **3i** est obtenu avec un rendement de 72% à partir de **2i**. PF = 95-97 °C (MeOH); ¹H RMN (300 MHz, CDCl₃) δ 1,29-1,90 (m, 10H, CH₂), 2,95 (s large, 3H, CH₃), 3,04 (s large, 3H, CH₃), 4,41-4,48 (m, 1H, CH), 5,53 (d, 1H, J = 12,6 Hz, =CH), 6,62 (d, 1H, J = 5,7 Hz, Hₐᵣₒₘ), 7,40-7,55 (m, 4H, =CH + Hₐᵣₒₘ), 7,85 (s, 1H, Hₐᵣₒₘ), 8,16 (d, 2H, J = 7,9 Hz, Hₐᵣₒₘ), 8,22 (d, 1H, J = 5,7 Hz, Hₐᵣₒₘ); SM (IS) m/z 454 (M + H⁺).

### e) 3-[(2-amino)pyrimidin-4-yl]-4-(cyclohexyloxy)-1H-pyrrolo[2,3-b] pyridine (4i): mérioline 17

Suivant la procédure décrite pour la préparation de **4a**, le composé **4i** est obtenu avec un rendement de 65% à partir de **3i**. PF > 210 °C (MeOH); ¹H RMN (300 MHz, DMSO-d₆) δ 1,28-1,63 (m, 6H, CH₂), 1,69 (s large, 2H, CH₂), 2,01 (s large, 2H, CH₂), 4,64-4,70 (m, 1H, CH), 6,31 (s large, 2H, NH₂), 6,79 (d, 1H, J = 5,5 Hz, Hₐᵣₒₘ), 7,31 (d, 1H, J = 5,3 Hz, Hₐᵣₒₘ), 7,86 (d, 1H, J = 2,3 Hz, Hₐᵣₒₘ), 8,10 (d, 1H, J = 5,5 Hz, Hₐᵣₒₘ), 8,16 (d, 1H, J = 5,3 Hz, Hₐᵣₒₘ), 12,04 (s large, 1H, NH); SM (IS) m/z 310 (M + H⁺).

De la même manière, les composés, ci-dessous, ont été préparés à partir de 7-azaindoles substitués en position 4.
- 3-[(2-amino)pyrimidin-4-yl]-4-(1-éthylpropoxy)-1*H*-pyrrolo[2,3-*b*]pyridine
- 3-[(2-amino)pyrimidin-4-yl]-4-(1-méthyléthoxy)-1*H*-pyrrolo[2,3-*b*]pyridine
- 3-[(2-amino)pyrimidin-4-yl]-4-(1,1-diméthyléthoxy)-1*H*-pyrrolo[2,3-*b*]pyridine
- 3-[(2-amino)pyrimidin-4-yl]-4-(1-méthylpropoxy)-1*H*-pyrrolo[2,3-*b*]pyridine
- 3-[(2-amino)pyrimidin-4-yl]-4-(1-cyclopentoxy)-1*H*-pyrrolo[2,3-*b*]pyridine
- 3-[(2-amino)pyrimidin-4-yl]-4-(1-cycloheptyloxy)-1*H*-pyrrolo[2,3-*b*]pyridine
- 3-[(2-amino)pyrimidin-4-yl]-4-(4-méthoxybenzyloxy)-1*H*-pyrrolo[2,3-*b*]pyridine
- 3-[(2-amino)pyrimidin-4-yl]-4-(3-méthoxybenzyloxy)-1*H*-pyrrolo[2,3-*b*]pyridine
- 3-[(2-amino)pyrimidin-4-yl]-4-(2-méthoxybenzyloxy)-1*H*-pyrrolo[2,3-*b*]pyridine
- 3-[(2-amino)pyrimidin-4-yl]-4-(4-chlorobenzyloxy)-1*H*-pyrrolo[2,3-*b*]pyridine
- 3-[(2-amino)pyrimidin-4-yl]-4-(3-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(4-fluoroobenzyloxy)-1*H*-pyrrolo[2,3-*b*]pyridine
- 3-[(2-amino)pyrimidin-4-yl]-4-(3-fluorobenzyloxy)-1*H*-pyrrolo[2,3-*b*]pyridine
- 3-[(2-amino)pyrimidin-4-yl]-4-(4-hydroxybenzyloxy)-1*H*-pyrrolo[2,3-*b*]pyridine
- 3-[(2-amino)pyrimidin-4-yl]-4-(3-hydroxybenzyloxy)-1*H*-pyrrolo[2,3-*b*]pyridine
- 3-[(2-amino)pyrimidin-4-yl]-4-(4-méthanesulfonylbenzyloxy)-1*H*-pyrrolo[2,3-*b*]pyridine
- 3-[(2-amino)pyrimidin-4-yl]-4-(pyridin-4-ylméthoxy)-1*H*-pyrrolo[2,3-*b*]pyridine
- 3-[(2-amino)pyrimidin-4-yl]-4-(pyridin-3-ylméthoxy)-1*H*-pyrrolo[2,3-*b*]pyridine
- 3-[(2-amino)pyrimidin-4-yl]-4-(pyridin-2-ylméthoxy)-1*H*-pyrrolo[2,3-*b*]pyridine
- 3-[(2-amino)pyrimidin-4-yl]-4-(pyrimidin-5-ylméthoxy)-1*H*-pyrrolo[2,3-*b*]pyridine
- 3-[(2-amino)pyrimidin-4-yl]-4-(pyridazin-4-ylméthoxy)-1*H*-pyrrolo[2,3-*b*]pyridine
- 3-[(2-amino)pyrimidin-4-yl]-4-(pipéridin-4-ylméthoxy)-1*H*-pyrrolo[2,3-*b*]pyridine
- 3-[(2-amino)pyrimidin-4-yl]-4-(1-méthanesulfonylpipéridin-4-ylméthoxy)-1*H*-pyrrolo[2,3-*b*]pyridine
- 3-[(2-amino)pyrimidin-4-yl]-4-benzyloxy-6-[4-(2-pyridinyl)benzyloxy]-1*H*-pyrrolo[2,3-*b*]pyridine
- 3-[(2-amino)pyridin-4-yl]-4-benzyloxy-6-[4-(3-pyridinyl)benzyloxy]-1*H*-pyrrolo[2,3-*b*]pyridine
- 3-[(2-amino)pyridin-4-yl]-4-benzyloxy-6-[4-(2-pyridinyl)benzyloxy]-1*H*-pyrrolo[2,3-*b*]pyridine
- 3-[(2-amino)pyridin-4-yl]-4-benzyloxy-6-[4-(3-pyridinyl)benzyloxy]-1*H*-pyrrolo[2,3-*b*]pyridine

### Exempte 11 : Synthèse de la 4-méthoxy-3-(pyrimidin-4-yl)-1H pyrrolo[2,3-b]pyridine (7) : mérioline 14

Une solution de 4-méthoxy-7-azaindole (100 mg, 0,67 mmol), de CuCl (27 mg, 0,27 mmol) et de formamide (300 µL) est chauffée à 170 °C pendant 18 h. Après refroidissement, l'H₂O est ajoutée à la solution (pH = 9), puis cette solution est extraite par de l'AcOEt. La phase organique est séchée sur MgSO₄, puis évaporée. Le résidu est purifié par colonne de chromatographie (éluant: AcOEt/MeOH 9: 1) pour donner 7 (40 mg, 26%). PF > 210 °C (MeOH); ¹H RMN (300 MHz, CD₃OD + D₂O) δ 4,09 (s, 3H, CH₃), 6,88 (d, 1H, J = 5,6 Hz, Hₐᵣₒₘ), 8,12 (s, 1H, Hₐᵣₒₘ), 8,20-8,22 (m, 2H, Hₐᵣₒₘ), 8,65 (d, 1H, J = 5,3 Hz, Hₐᵣₒₘ), 8,99 (s, 1H, Hₐᵣₒₘ); SM (IS) m/z 227 (M + H⁺).

De la même manière, les composés, ci-dessous, ont été préparés à partir de 7-azaindoles substitués en position 4.
- 3-(pyrimidin-4-yl)-4-éthoxy-1*H*-pyrrolo[2,3-*b*]pyridine
- 3-(pyrimidin-4-yl)-4-propoxy-1*H*-pyrrolo[2,3-*b*]pyridine
- 3-(pyrimidin-4-yl)-4-(1-méthyléthoxy)-1*H*-pyrrolo[2,3-*b*]pyridine
- 3-(pyrimidin-4-yl)-4-nitro-1*H*-pyrrolo[2,3-b]pyridine
- 3-(pyrimidin-4-yl)-4-chloro-1*H*-pyrrolo[2,3-*b*]pyridine
- 3-(pyrimidin-4-yl)-4-fluoro-1*H*-pyrrolo[2,3-*b*]pyridine
- 3-(pyrimidin-4-yl)-4-méthyl-1*H*-pyrrolo[2,3-*b*]pyridine
- 3-(pyrimidin-4-yl)-4-éthyl-1*H*-pyrrolo[2,3-*b*]pyridine
- 3-(pyrimidin-4-yl)-4-cyano-1*H*-pyrrolo[2,3-*b*]pyridine
- 3-(pyrimidin-4-yl)-4-phényl-1*H*-pyrrolo[2,3-*b*]pyridine
- 3-(pyrimidin-4-yl)-4-méthylamino-1*H*-pyrrolo[2,3-*b*]pyridine

### Exemple 12 : Synthèse de la 3-[(2-amino)pyrimidin-4-yl]-4-(pent-1-ynyl)-1H-pyrrolo[2,3-b]pyridine (4j) : mérioline 18

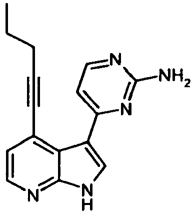

### Mérioline 18

### a) 3-acétyl-1-(1-benzènesulfonyl)-4-(pent-1-ynyl)-1H-pyrrolo[2,3-b] pyridine (2j)

A une solution de **2a** (150 mg, 0,45 mmol), de PdCl₂(PPh₃)₂ (63 mg, 0,09 mmol) et de CuI (34 mg, 0,18 mmol) dans le DMF anhydre (4,5 mL) est additionnée la triéthylamine (250 µL, 1,80 mmol) et la pent-1-yne (225 µL, 2,30 mmol). La solution est agitée à 50 °C pendant 72 h dans un tube scellé. Après refroidissement, la solution est reprise dans un mélange NH₄OH 1 M (50 mL) et de CH₂Cl₂ (50 mL). Les phases sont séparées. La phase aqueuse est extraite une nouvelle fois par du CH₂Cl₂ (25 mL). Les phases organiques combinées sont séchées sur MgSO₄ puis évaporées. Le résidu obtenu est purifié par colonne de chromatographie (éluant: éther de pétrole/AcOEt 85:15) pour donner le composé **2j** (110 mg, 67%). PF = 134-136 °C (CH₂Cl₂/pentane); ¹H RMN (300 MHz, CDCl₃)δ1,01 (t, 3H, J = 7,3 Hz, CH₃), 1,60-1,72 (m, 2H, CH₂), 2,47 (t, 2H, J = 7,3 Hz, CH₂), 2,60 (s, 3H, CH₃), 7,25 (d, 1H, J = 5,0 Hz, Hₐᵣₒₘ), 7,49 (t, 2H, J = 7,4 Hz, Hₐᵣₒₘ), 7,59 (t, 1H, J = 7,4 Hz, Hₐᵣₒₘ), 8,20 (d, 2H, J = 7,5 Hz, Hₐᵣₒₘ), 8,28 (s, 1H, Hₐᵣₒₘ), 8,31 (d, 1H, J = 5,0 Hz, Hₐᵣₒₘ); SM (IS) m/z 367 (M + H⁺).

### b) 1-(1-benzènesulfonyl-4-(pent-1-ynyl)-1H-pyrrolo[2,3-b]pyridin-3-yl)-3-diméthylaminopropénone (3j)

Suivant la procédure décrite pour la préparation de **3a**, le composé **3j** est obtenu avec un rendement de 66% à partir de **2j**. Huile; ¹H RMN (300 MHz, CDCl₃) δ 0,95 (t, 3H, J = 7,3 Hz, CH₃), 1,50-1,62 (m, 2H, CH₂), 2,34 (t, 2H, J = 7,3 Hz, CH₂), 2,84 (s large, 3H, CH₃), 3,04 (s large, 3H, CH₃), 5,45 (d, 1H, J = 12,6 Hz, =CH), 7,15 (d, 1H, J = 5,1 Hz, Hₐᵣₒₘ), 7,40-7,55 (m, 4H, =CH + Hₐᵣₒₘ), 7,94 (s, 1H, Hₐᵣₒₘ), 8,13 (d, 2H, J = 7,5 Hz, Hₐᵣₒₘ), 8,28 (d, 1H, J = 5,1 Hz, Hₐᵣₒₘ); SM (IS) m/z 422 (M + H⁺).

### c) 3-[(2-amino)pyrimidin-4-yl]-4-(pent-1-ynyl)-1H-pyrrolo[2,3-b] pyridine (4j) : mérioline 18

Suivant la procédure décrite pour la préparation de **4a**, le composé **4j** est obtenu avec un rendement de 50% à partir de **3j**. PF > 210 °C (MeOH); ¹H RMN (300 MHz, DMSO-d₆) δ 0,89 (t, 3H, J = 7,3 Hz, CH₃), 1,43-1,55 (m, 2H, CH₂), 2,40 (t, 2H, J = 7,3 Hz, CH₂), 6,43 (s large, 2H, NH₂), 7,01 (d, 1H, J = 5,1 Hz, Hₐᵣₒₘ), 7,14 (d, 1H, J = 4,9 Hz, Hₐᵣₒₘ), 7,92 (s, 1H, Hₐᵣₒₘ), 8,19 (d, 1H, J = 5,5 Hz, Hₐᵣₒₘ), 8,21 (d, 1H, J = 5,1 Hz, Hₐᵣₒₘ), 12,29 (s large, 1H, NH); SM (IS) m/z 278 (M + H⁺).

### Exemple 13 : Synthèse de la 3-[(2-amino)pyrimidin-4-yl]-4-(phényléth-1-ynyl)-1H-pyrrolo[2,3-b]pyridine (4k) : mérioline 21

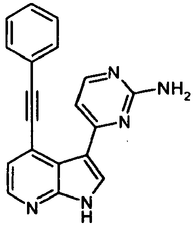

### Mérioline 21

### a) 3-acétyl-1-(1-benzènesulfonyl)-4-(phényléth-1-ynyl)-1H-pyrrolo [2,3-b]pyridine (2k)

Suivant la procédure décrite pour la préparation de **2j**, le composé **2k** est obtenu avec un rendement de 33% à partir de **2a**. ¹H RMN (300 MHz, CDCl₃) δ 2,66 (s, 3H, CH₃), 7,37-7,42 (m, 4H, Hₐᵣₒₘ), 7,54 (t, 2H, J = 7,8 Hz, Hₐᵣₒₘ), 7,62-7,71 (m, 3H, Hₐᵣₒₘ), 8,23-8,27 (m, 2H, Hₐᵣₒₘ), 8,36 (s, 1H, Hₐᵣₒₘ) 8,40 (d, 1H, J = 5,1 Hz, Hₐᵣₒₘ); SM (IS) m/z 401 (M + H⁺).

### b) 1-(1-benzènesulfonyl-4-(phényléth-1-ynyl)-1H-pyrrolo[2,3-b] pyridin-3-yl)-3-diméthylaminopropénone (3k)

Suivant la procédure décrite pour la préparation de **3a**, le composé **3k** est obtenu avec un rendement de 43% à partir de **2k**. PF 94-96 °C (MeOH); ¹H RMN (300 MHz, CD₃OD + D₂O) δ 2,81 (s large, 3H, CH₃), 3,07 (s large, 3H, CH₃), 5,62 (d, 1H, J = 12,4 Hz, =CH), 7,39-7,42 (m, 4H, Hₐᵣₒₘ), 7,54-7,72 (m, 6H, =CH + Hₐᵣₒₘ), 8,15 (s, 1H, Hₐᵣₒₘ), 8,20 (d, 2H, J = 7,5 Hz, Hₐᵣₒₘ), 8,36 (d, 1H, J = 4,8 Hz, Hₐᵣₒₘ); SM (IS) m/z 456 (M + H⁺).

### c) 3-[(2-amino)pyrimidin-4-yl]-4-(phényléth-1-ynyl)-1H-pyrrolo [2,3-b]pyridine (4k): mérioline 21

Suivant la procédure décrite pour la préparation de **4a**, le composé **4k** est obtenu avec un rendement de 73% à partir de **3k**. PF > 210 °C (MeOH); ¹H RMN (300 MHz, CD₃OD + D₂O) δ 7,20 (d, 1H, J = 5,3 Hz, Hₐᵣₒₘ), 7,36 (d, 1H, J = 4,9 Hz, Hₐᵣₒₘ), 7,38-7,42 (m, 5H, Hₐᵣₒₘ), 7,95 (s, 1H, Hₐᵣₒₘ), 8,18 (d, 1H, J = 5,3 Hz, Hₐᵣₒₘ), 8,29 (d, 1H, J = 4,9 Hz, Hₐᵣₒₘ); SM (IS) m/z 312 (M + H⁺).

De la même manière, les composés, ci-dessous, ont été préparés à partir de **2a**.
- 3-[(2-amino)pyrimidin-4-yl]-4-(prop-1-ynyl)-1*H*-pyrrolo[2,3-b]pyridine
- 3-[(2-amino)pyrimidin-4-yl]-4-(but-1-ynyl)-1*H*-pyrrolo[2,3-*b*]pyridine
- 3-[(2-amino)pyrimidin-4-yl]-4-(hex-1-ynyl)-1*H*-pyrrolo[2,3-*b*]pyridine
- 3-[(2-amino)pyrimidin-4-yl]-4-(4-méthylpent-1-ynyl)-1*H* pyrrolo[2,3-*b*]pyridine
- 3-[(2-amino)pyrimidin-4-yl]-4-(4-cyclohexyléth-1-ynyl)-1*H*-pyrrolo[2,3-*b*]pyridine

La synthèse des composés **4l** et **4m** de l'invention est réalisée par réaction d'hydrogénation catalytique respectivement des alcynes 4j et 4k. Ce procédé est représenté au schéma 2 ci-dessous

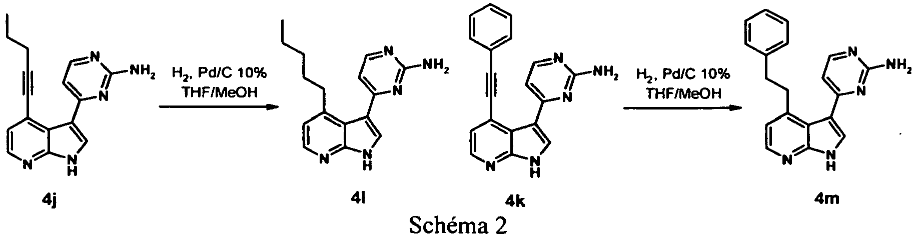

### Exemple 14 : Synthèse de la 3-[(2-amino)pyrimidin-4-yl]-4-pentyl-1H-pyrrolo[2,3-b]pyridine (41) : mérioline 19

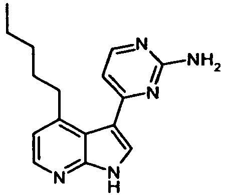

### Mérioline 19

Le composé **4j** (28 mg, 0,1 mmol) et de Pd/C 10% (9 mg) dans un mélange THF/MeOH (2 mL; 1:1) est agité sous une pression de 5 atm de dihydrogène pendant 24 h. Après évaporation des solvants, le résidu est purifié par colonne de chromatographie (éluant: AcOEt/EtOH 9:1) pour donner le composé **4l** (22 mg, 78%). PF > 210 °C (MeOH); ¹H RMN (300 MHz, DMSO-d₆) δ 0,69 (t, 3H, J = 7,0 Hz, CH₃), 1,09-1,23 (m, 6H, CH₂), 3,23 (t, 2H, J = 7,9 Hz, CH₂), 6,48 (s large, 2H, NH₂), 6,82 (d, 1H, J = 5,1 Hz, Hₐᵣₒₘ), 6,94 (d, 1H, J = 4,9 Hz, Hₐᵣₒₘ), 7,84 (s, 1H, Hₐᵣₒₘ), 8,14 (d, 1H, J = 4,9 Hz, Hₐᵣₒₘ), 8,17 (d, 1H, J = 5,1 Hz, Hₐᵣₒₘ), 12,03 (s large, 1H, NH); SM (IS) m/z 282 (M + H⁺).

### Exemple 15: Synthèse de la 3-[(2-amino)pyrimidin-4-yl]-4-phénéthyl-1H-pyrrolo[2,3-b]pyridine (4m) : mérioline 22

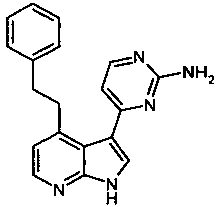

### Mérioline 22

Suivant la procédure décrite pour la préparation de **4l**, le composé **4l** est obtenu avec un rendement de 77% à partir de **4k**. PF = 196-198 °C (MeOH); ¹H RMN (300 MHz, DMSO-d₆) δ 2,56 (t large, 2H, J = 8,1 Hz, CH₂), 3,53 (t large, 2H, J = 8,1 Hz, CH₂), 6,47 (s large, 1H, NH₂), 6,88 (d, 1H, J = 5,1 Hz, Hₐᵣₒₘ), 6,96-6,99 (m, 3H, Hₐᵣₒₘ), 7,11-7,20 (m, 3H, Hₐᵣₒₘ), 7,89 (s, 1H, Hₐᵣₒₘ), 8,15 (d, 1H, J = 4,9 Hz, Hₐᵣₒₘ), 8,20 (d, 1H, J = 5,1 Hz, Hₐᵣₒₘ); SM (IS) m/z 316 (M + H⁺).

De la même manière, les composés, ci-dessous, ont été préparés.
- 3-[(2-amino)pyrimidin-4-yl]-4-propyl-1*H*-pyrrolo[2,3-b]pyridine
- 3-[(2-amino)pyrimidin-4-yl]-4-butyl-1*H*-pyrrolo[2,3-*b*]pyridine
- 3-[(2-amino)pyrimidin-4-yl]-4-hexyl-1*H*-pyrrolo[2,3-*b*]pyridine
- 3-[(2-amino)pyrimidin-4-yl]-4-(4-méthylpentyl)-1*H*-pyrrolo[2,3-*b*]pyridine
- 3-[(2-amino)pyrimidin-4-yl]-4-(4-cyclohexyléthyl)-1*H*-pyrrolo[2,3-*b*]pyridine

Les composés **4** sont préparés selon la même approche synthétique à partir de **1** ou **5** mais en remplaçant le groupement protecteur SO₂Ph par le groupe éthoxyméthyle, comme représenté au schéma 3 suivant :

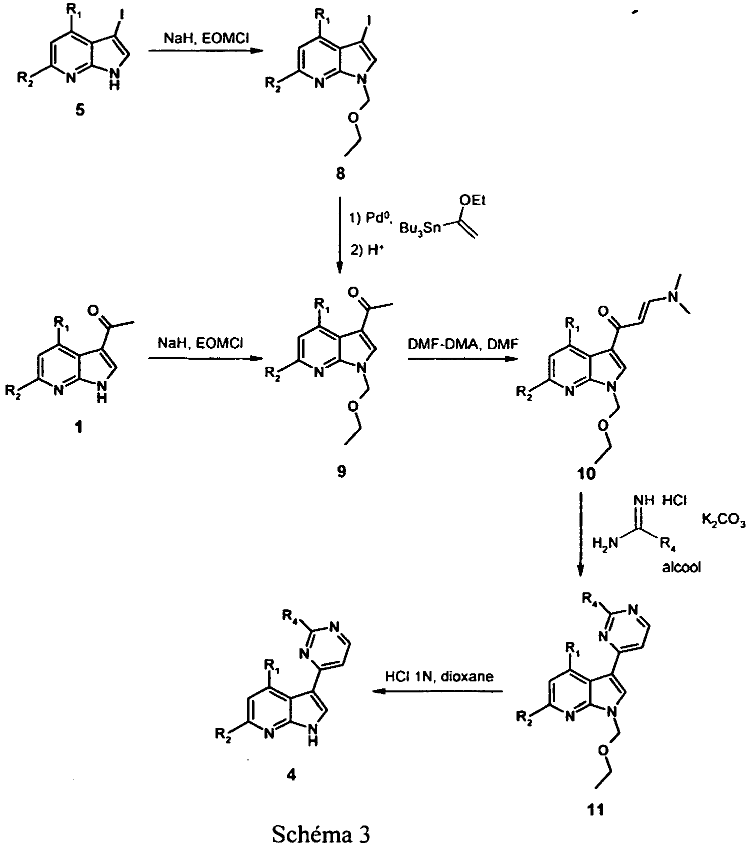

### Exemple 16 : Synthèse de la 3-[(2-amino)pyrimidin-4-yl]-6-bromo-4-méthoxy-1H-pyrrolo[2,3-b]pyridine (4n)

### a) 3-acétyl-6-bromo-4-méthoxy-1H-pyrrolo[2,3-b]pyridine (1n)

Suivant la procédure décrite pour la préparation de 1a, le composé **1n** est obtenu avec un rendement de 91% à partir de 6-bromo-4-methoxy-7-azaindole. PF > 210 °C (MeOH); ¹H RMN (300 MHz, DMSO-d₆) δ 2,50 (s, 3H, CH₃), 3,95 (s, 3H, CH₃), 6,96 (s, 1H, Hₐᵣₒₘ), 8,14 (s, 1H, Hₐᵣₒₘ), 12,55 (s large, 1H, NH); SM (IS) m/z 271 (Br⁸¹, M + H⁺), 269 (Br⁷⁹, M + H⁺).

### b) 3-acétyl-6-bromo-1-(1-éthoxyméthyl)-4-méthoxy-1H-pyrrolo [2,3-b]pyridine (9n)

A 0 °C, l'hydrure de sodium (26 mg, 0,64 mmol, 60% dans l'huile) est additionné par petites portions à une solution de **1n** (80 mg, 0,30 mmol) dans le DMF anhydre (3 mL). La solution est agitée à 0 °C pendant 45 min, puis le chlorure d'éthoxyméthyle (42 µL, 0,50 mmol) est additionné au mélange réactionnel. La solution finale est agitée à température ambiante pendant 4 h. L'addition d'H₂O est réalisée à 0 °C, puis les solvants sont éliminés sous pression réduite. Le résidu obtenu est repris dans un mélange H₂O et AcOEt, puis les deux phases sont séparées. La phase organique recueillie est séchée sur MgSO₄ puis évaporée. Le solide obtenu est purifié par colonne de chromatographie (éluant: éther de pétrole/AcOEt 7:3) pour donner le composé **9n** (57 mg, 58%). PF = 114-116 °C (CH₂Cl₂/pentane); ¹H RMN (300 MHz, CDCl₃) δ 1,18 (t, 3H, J = 7,2 Hz, CH₃), 2,62 (s, 3H, CH₃), 3,53 (q, 2H, J = 7,2 Hz, CH₂), 4,03 (s, 3H, CH₃), 5,64 (s, 2H, CH₂), 6,84 (s, 1H, Hₐᵣₒₘ), 7,86 (s, 1H, Hₐᵣₒₘ); SM (IS) m/z 329 (Br⁸¹, M + H⁺), 327 (Br⁷⁹, M + H⁺).

### c) 6-bromo-1-(1-éthoxyméthyl)-4-méthoxy-1H-pyrroto[2,3-b] pyridin-3-yl)-3,3-diméthylaminopropénone (10n)

Suivant la procédure décrite pour la préparation de **3a**, le composé **10n** est obtenu avec un rendement de 76% à partir de **9n**. PF = 177-179 °C (MeOH); ¹H RMN (300 MHz, CDCl₃) δ 1,11 (t, 3H, J = 7,2 Hz, CH₃), 2,90-3,10 (m, 6H, 2 CH₃), 3,46 (q, 2H, J = 7,2 Hz, CH₂), 3,96 (s, 3H, CH₃), 5,58 (s, 2H, CH₂), 5,83 (d, 1H, J = 12,6 Hz, =CH), 6,74 (s, 1H, Hₐᵣₒₘ), 7,69 (d, 1H, J = 12,4 Hz, =CH), 7,73 (s, 1H, Hₐᵣₒₘ); SM (IS) m/z 384 (Br⁸¹, M + H⁺), 382 (Br⁷⁹, M + H⁺)_{.}

### d) 3-[(2-amino)pyrimidin-4-yl]-6-bromo-1-(1-éthoxyméthyl)-4-méthoxy-1H-pyrrolo[2,3-b]pyridine (11n)

Suivant la procédure décrite pour la préparation de **4a**, le composé **11n** est obtenu avec un rendement de 95% à partir de 10n. PF > 210 °C (MeOH); ¹H RMN (300 MHz, CD₃OD + D₂O) δ 1,15 (t, 3H, J = 7,2 Hz, CH₃), 3,54 (q, 2H, J = 7,2 Hz, CH₂), 4,04 (s, 3H, CH₃), 5,67 (s, 2H, CH₂), 7,00 (s, 1H, Hₐᵣₒₘ), 7,32 (d, 1H, J = 5,5 Hz, Hₐᵣₒₘ), 8,04 (s, 1H, Hₐᵣₒₘ), 8,20 (d, 1H, J = 5,5 Hz, Hₐᵣₒₘ); SM (IS) m/z 380 (Br⁸¹, M + H⁺), 378 (Br⁷⁹, M + H⁺).

### e) 3-[(2-amino)pyrimidin-4-yl]-6-bromo-4-méthoxy-1H-pyrrolo(2,3-b]pyridine (4n)

Une solution de **11n** (50 mg, 0,13 mmol) et de HCl 1 N (1 mL) dans le 1,4-dioxane (2 mL) est agitée à 70 °C pendant 30 min. Après refroidissement, la solution est neutralisée par addition d'une solution saturée de NaHCO₃, puis extraite par le CH₂Cl₂ (2 x 5 mL) La phase organique est séchée sur MgSO₄, puis évaporée. Le solide brut est recristallisé dans le MeOH pour donner **4n** (30 mg, 70%). PF > 210 °C (MeOH); ¹H RMN (300 MHz, CD₃OD + D₂O) δ 4,04 (s, 3H, CH₃), 6,95 (s, 1H, Hₐᵣₒₘ), 7,32 (d, 1H, J = 5,5 Hz, Harom), 7,97 (s, 1H, Hₐᵣₒₘ), 8,20 (d, 1H, J = 5,5 Hz, Hₐᵣₒₘ); SM (IS) m/z 322 (Br⁸¹, M + H⁺), 320 (Br⁷⁹, M + H⁺).

De façon identique, les composés, ci-dessous, ont été préparés à partir du 6-bromo-4-éthyloxy-1*H*-pyrrolo[2,3-*b*]pyridine et du 6-bromo-4-propyloxy-1*H-*pyrrolo[2,3-b]pyridine.
- 3-[(2-amino)pyrimidin-4-yl]-6-bromo-4-éthoxy-1*H*-pyrrolo[2,3-b]pyridine
- 3-[(2-amino)pyrimidin-4-yl]-6-bromo-4-propoxy-1*H*-pyrrolo[2,3-b]pyridine
- 3-[(2-amino)pyrimidin-4-yl]-6-chloro-4-éthoxy-1*H*-pyrrolo[2,3-b]pyridine
- 3-[(2-amino)pyrimidin-4-yl]-6-chloro-4-proppxy-1*H*-pyrrolo[2,3-b]pyridine
- 3-[(2-amino)pyrimidin-4-yl]-6-fluoro-4-éthoxy-1*H*-pyrrolo[2,3-b]pyridine
- 3-[(2-amino)pyrimidin-4-yl]-6-fluoro-4-propoxy-1*H*-pyrrolo[2,3-b]pyridine

Un autre procédé de synthèse des mériolines de l'invention passe par la *N*-alkylation (NaH, EX) ou la *N*-arylation (ArX, Cu₂O, K₂CO₃) des composés **1** conduit à l'obtention des produits **12.** Les énaninones **13** sont préparées par traitement de **12** en présence de DMF-DMA *(Tetrahedron* **2001,** *57*, 2355-2363). Les composés finaux **14** sont obtenus par chauffage de **13** en présence de chlorhydrate de guanidinium ou de dérivés.

Ce procédé est représenté au schéma 4 ci-dessous

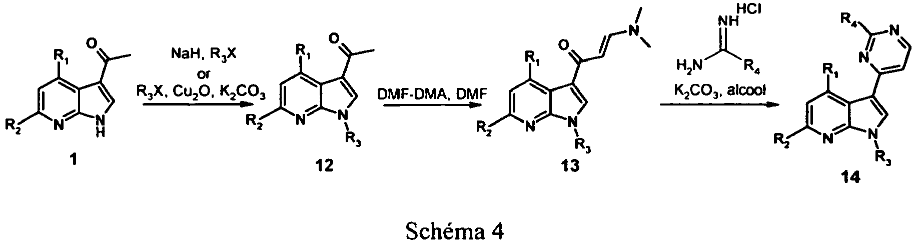

### Exemple 17 : Synthèse de la 3-((2-amino)pyrimidin-4-yl]-4-méthoxy-1-méthyl-1H-pyrrolo[2,3-b]pyridine (14b) : mérioline 9

### a) 3-acétyl-4-méthoxy-1-méthyl-1H-pyrrolo[2,3-b]pyridine (12b)

Sous atmosphère inerte, la solution de **1b** (80 mg, 0,42 mmol), de diméthylsulfate (0,07 mL, 0,74 mmol) et de K₂CO₃ (82 mg, 0,59 mmol) dans l'acétone anhydre (15 mL) est chauffée à reflux pendant 5,5 h. Après refroidissement, le solvant est évaporé. Le résidu obtenu est repris dans un mélange H₂O et AcOEt (30 mL, 1: 1), puis les deux phases sont séparées. La phase organique recueillie est séchée sur MgSO₄ puis évaporée. Le solide obtenu est purifié par colonne de chromatographie (éluant: AcOEt) pour donner le composé **12b** (70 mg, 81%). PF = 79-81 °C (CH₂Cl₂/hexane); ¹H RMN (300 MHz, CDC1₃) δ 2,61 (s, 3H, CH₃), 3,85 (s, 3H, CH₃), 3,99 (s, 3H, CH₃), 6,64 (d, 1H, J = 5,5 Hz, Hₐᵣₒₘ), 7,76 (s, 1H, Hₐᵣₒₘ), 8,23 (d, 1H, J = 5,5 Hz, Hₐᵣₒₘ); SM (IS) m/z 205 (M + H⁺).

### b) 1-(4-méthoxy-1-méthyl-1H-pyrrolo[2,3-b]pyridin-3-yl)-3,3-diméthylaminopropénone (13b)

Suivant la procédure décrite pour la préparation de **3a**, le composé **13b** est obtenu avec un rendement de 82% à partir de **12b**. PF = 138-140 °C (MeOH); ¹H RMN (300 MHz, CDCl₃) δ 3,00 (s large, 6H, CH₃), 3,87 (s, 3H, CH₃), 4,00 (s, 3H, CH₃), 6,08 (d, 1H, J = 12,6 Hz, =CH), 6,63 (d, 1H, J = 5,6 Hz, Hₐᵣₒₘ), 7,74 (d, 1H, J = 12,6 Hz, =CH), 7,76 (s, 1H, Hₐᵣₒₘ), 8,23 (d, 1H, J = 5,6 Hz, Hₐᵣₒₘ); SM (IS) m/z 260 (M + H⁺).

### c) 3-[(2-amino)pyrimidin-4-yl]-4-méthoxy-1-méthyl-1H-pyrrolo[2,3-b]pyridine (14b) : mérioline 9

Suivant la procédure décrite pour la préparation de **4a,** le composé **14b** est obtenu avec un rendement de 92% à partir de **13b.** PF > 210 °C (MeOH); ¹H RMN (300 MHz, CDCl₃) δ 3,91 (s, 3H, CH₃), 4,02 (s, 3H, CH₃), 4,93 (s large, 2H, NH₂), 6,65 (d, 1H, J = 5,5 Hz, Hₐᵣₒₘ), 7,45 (d, 1H, J = 5,3 Hz, Hₐᵣₒₘ), 7,92 (s, 1H, Hₐᵣₒₘ), 8,26 (d, 1H, J = 5,3 Hz, Hₐᵣₒₘ), 8,27 (d, 1H, J = 5,5 Hz, Hₐᵣₒₘ); SM (IS) m/z 256 (M + H⁺).

De façon identique, les composés suivants ont été préparés à partir des 3-acétyl-7-azaindoles substitués en position 4 ou/et 6:
- 3-[(2-amino)pyrimidin-4-yl]-4-éthoxy-1-méthyl-1*H*-pyrrolo[2,3-b]pyridine
- 3-[(2-amino)pyrimidin-4-yl]-4-nitro-1-méthyl-1*H*-pyrrolo[2,3-b]pyridine
- 3-[(2-amino)pyrimidin-4-yl]-4-fluoro-1-méthyl-1*H*-pyrrolo[2,3-b]pyridine
- 3-[(2-amino)pyrimidin-4-yl]-1,4-diméthyl-1*H*-pyrrolo[2,3-b]pyridine
- 3-[(2-amino)pyrimidin-4-yl]-4-cyano-1-méthyl-1*H*-pyrrolo[2,3-b]pyridine
- 3-[(2-amino)pyrimidin-4-yl]-4-phényl-1-méthyl-1*H*-pyrrolo[2,3-*b*]pyridine
- 3-[(2-amino)pyrimidin-4-yl]-1-méthyl-1*H*-pyrrolo[2,3-*b*]pyridine-4-carboxylate de méthyle
- 4-amino-3-[(2-amino)pyrimidin-4-yl]-1-méthyl-1*H*-pyrrolo[2,3-*b*]pyridine
- 3-[(2-amino)pyrimidin-4-yl]-4-propoxy-1-méthyl-1*H*-pyrrolo[2,3-*b*]pyridine
- 3-[(2-amino)pyrimidin-4-yl]-6-chloro-1-méthyl-1*H*-pyrrolo[2,3-*b*]pyridine
- 3-[(2-amino)pyrimidin-4-yl]-6-bromo-1-méthyl-1*H*-pyrrolo[2,3-*b*]pyridine
- 3-[(2-amino)pyrimidin-4-yl]-6-bromo-4-méthoxy-1-méthyl-1*H*-pyrrolo[2,3-*b*]pyridine
- 3-[(2-amino)pyrimidin-4-yl]-6-cyano-1-méthyl-1*H*-pyrrolo[2,3-*b*]pyridine

La réaction de O-déméthylation (HBr/CH₃CO₂H) des composés **4b, 5** et **14b** donnent les dérivés **15-17** avec de bons rendements (schéma 5).

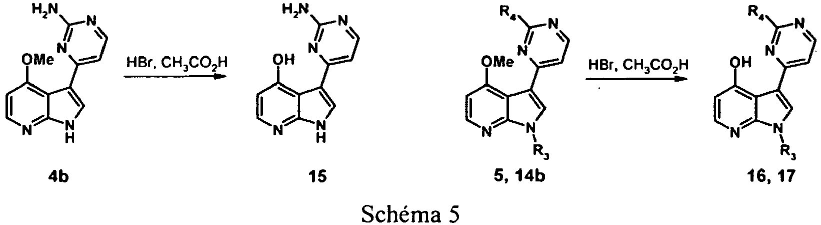

### Exemple 18 : Synthèse de la 3-[(2-amino)pyrimidin-4-yl]-4-hydroxy-1H-pyrrolo[2,3-b]pyridine (15) : mérioline 2

Une solution de **4b** (50 mg, 0,20 mmol) dans 48% HBr/CH₃CO₂H (6 mL) est chauffée à reflux pendant 2 h. Après refroidissement, le solvant est évaporé. Le résidu obtenu est dissout dans de l'AcOEt (10 mL), puis la solution est neutralisée par addition d'une solution saturée de Na₂CO₃ (pH = 7-8). Les deux phases sont séparées et la phase aqueuse recueillie est lavée par de l'AcOEt (2 x). Les phases organiques sont combinées, séchées sur MgSO₄ et évaporées. Le solide obtenu est recristallisé dans le MeOH pour donner le composé **15** (43 mg, 90%). PF > 210°C (MeOH); ¹H RMN (300 MHz, DMSO-d₆ + D₂O) δ 6,51 (d, 1H, J = 5,5 Hz, Hₐᵣₒₘ), 7,15 (d, 1H, J = 5,5 Hz, Hₐᵣₒₘ), 7,99 (d, 1H, J = 5,5 Hz, Hₐᵣₒₘ), 8,17 (d, 1H, J = 5,5 Hz, Hₐᵣₒₘ), 8,24 (s, 1H, Hₐᵣₒₘ); SM (IS) m/z 228 (M + H⁺).

### Exemple 19 : Synthèse de la 3-[(2-amino)pyrimidin-4-yl]-4-hydroxy-1-méthyl-1H-pyrrolo[2,3-b]pyridine (16) : mérioline 8

Suivant la procédure décrite pour la préparation de **15,** le composé **16** est obtenu avec un rendement de 92% à partir de **14b.** PF > 210 °C (MeOH); ¹H RMN (300 MHz, DMSO-d₆+ D₂O) δ 3,78 (s, 3H, CH₃), 6,56 (d, 1H, J = 5,5 Hz, Hₐᵣₒₘ), 7,08 (d, 1H, J = 5,5 Hz, Hₐᵣₒₘ), 8,04 (d, 1H, J = 5,5 Hz, Hₐᵣₒₘ), 8,16 (d, 1H, *J* = 5,5 Hz, Hₐᵣₒₘ), 8,28 (s, 1H, Hₐᵣₒₘ); SM (IS) m/z 242 (M + H⁺).

### Exemple 20 : Synthèse de la 3-(pyrimidin-4-yl)-4-hydroxy-1H-pyrrolo[2,3-b]pyridine (17): mérioline 13

Suivant la procédure décrite pour la préparation de **15,** le composé **17** est obtenu avec un rendement de 92% à partir de **5**. PF > 210 °C (MeOH); ¹H RMN (300 MHz, DMSO-d₆) δ 6,53 (d, 1H, J = 5,1 Hz, Hₐᵣₒₘ), 8,05 (d, 1H, J = 5,1 Hz, Hₐᵣₒₘ), 8,12 (d, 1H, J = 5,6 Hz, Hₐᵣₒₘ), 8,58 (s, 1H, Hₐᵣₒₘ), 8,71 (d, 1H, J = 5,6 Hz, Hₐᵣₒₘ), 9,10 (s, 1H, Hₐᵣₒₘ), 12,42 (s large, 1H, NH), 14,24 (s, 1H, OH); SM (IS) m/z 213 (M + H⁺).

De la même manière, les composés suivants ont été préparés:
- 3-[(2-amino)pyrimidin-4-yl]-6-bromo-4-hydroxy-1*H*-pyrrolo[2,3-b]pyridine
- 3-[(2-amino)pyrimidin-4-yl]-6-bromo-4-hydroxy-1-méthyl-1*H*-pyrrolo[2,3-b]pyridine
- 3-(pyrimidin-4-yl)-4-hydroxy-1-méthyl-1*H*-pyrrolo[2,3-b]pyridine

### Exemple 21 : Synthèse de la 3-(2-aminopymiridin-4-yl)-4-(2-méthoxyéthoxyl)-1H-pyrrolo[2-3-b]pyridine : mérioline 7.

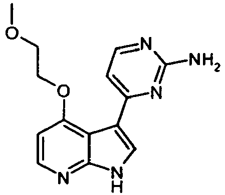

On a synthétisé la mérioline 7 comme décrit à l'exemple 2.

### Exemple comparatif 1 : Synthèse de la 4-méthoxy-3-(2-méthylsul fanylpyrimidin-4-yl)-1H-pyrrolo[2-3-b]pyridine : mérioline 12.

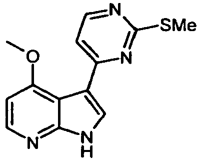

La mérioline 12 a été synthétisée selon la procédure décrite dans Monatsch. Chem. 2004, 135, 615-627.

### Exemple comparatif 2 : Synthèse de la 3-[(2-amino)pyrimidin-4-yl)]-1H-pyrrolo[2,3-b]pyridine : mérioline 1.

La mérioline 1 a été synthétisée selon la procédure décrite dans Tetrahedron 2001, 57, 2355-2363.

### Exemple comparatif 3 : Synthèse de la varioline B.

La varioline B a été synthétisée selon la procédure décrite par Anderson RJ & al., en 2005 dans « Concise total syntheses of variolin B and déoxyvariolin B », J. Org. Chem. 2005, 70, 6204-6212.

### II) TESTS DE L'ACTIVITE D'INHIBITION DES PROTEINES KINASES DES MERIOLINES DE L'INVENTION.

### I) DOSAGE DES PROTEINES KINASES

### Réactifs Biochimiques.

L'ortho-vanadate de sodium, l'EGTA, l'EDTA, le Mops, le β-glycérophosphate, le phénylphosphate, le fluorure de sodium, le dithiothréitol (DTT), le glutathione-agarose, le glutathione, l'albumine de sérum bovin (BSA), le nitrophénylphosphate, la leupeptine, l'aprotinine, la pepstatine, l'inhibiteur de trypsine de soja, la benzamidine, l'histone H1 (type III-S) ont été obtenus de Sigma Chemicals. [γ-³³P]-ATP a été obtenu d'Amersham. Le peptide GS-1 (YRRAAVPPSPSLSRHSSPHQSpEDEEE) a été synthétisé par la Peptide Synthesis Unit, Institut des Sciences Biomoléculaires, Université de Southampton, Southampton SO16 7PX, Royaume-Uni. Le substrat peptidique spécifique au CK1 (RRKHAAIGSpAYSITA) était un don généreux des Docteurs F. Meggio et L. Pinna (Marin et *al.,* 1994).

### Tampons.

*Tampons d'homogénéisation:* 60 mM β-glycérophosphate, 15 mM p-nitrophénylphosphate, 25 mM Mops (pH 7.2), 15 mM EGTA, 15 mM MgCl₂, 1 mM DTT, 1 mM sodium vanadate, 1 mM NaF, 1 mM phénylphosphate, 10 µg leupeptine/ml, 10 µg aprotinine/ml, 10 µg d'inhibiteur de trypsine de soja /ml et 100 µM benzamidine.

*Tampon A:* 10 mM MgCl₂, 1 mM EGTA, 1 mM DTT, 25 mM Tris-HCl pH 7.5, 50 µg héparine/ml.

*Tampon C*: tampon d'homogénéisation mais 5 mM EGTA, pas de NaF et pas d'inhibiteurs de protéase.

### Préparations et dosage des kinases.

Les kinases ont été dosées dans le tampon A ou le tampon C, à 30°C, à une concentration finale en ATP de 15 µM. Les valeurs de blanc ont été soustraites et les activités calculées en tant que pmoles de phosphate incorporés pour 10 minutes d'incubation. Les activités sont habituellement exprimées en % (pourcentage) de l'activité maximale, c'est-à-dire en l'absence d'inhibiteurs. Des contrôles ont été effectués avec des dilutions appropriées de diméthylsulfoxide.

*CDK1*/*cycline B :* a été extrait dans un tampon d'homogénisation à partir d'oocytes d'étoiles de mer de phase M (*Marthasterias glacialis*) purifié par chromatographie d'affinité sur des billes de sépharose marquées à p9^{CKShs1} dont il a été élué par p9^{CKSh1} libre comme antérieurement décrit dans Meijer et al., (1997) « Biochemical and cellular effects of roscovitine, a potent and selective inhibitor of the cyclin-dependent kinases cdc2, cdk2 and cdk5 », Eur. J. Biochem. 1997, 243, 527-536. L'activité kinase a été dosée dans le tampon C, avec 1 mg d'histone H1/ml, en présence de 15 µM de [γ-³³P] ATP (3000 Ci/mmol; 10 mCi/ml) dans un volume final de 30 µl. Après 30 minutes d'incubation à 30°C, des aliquotes de 25 µl du surnageant ont été mises en tâches sur des filtres en papier de phosphocellulose Whatman P81, et, 20 secondes plus tard, les filtres ont été lavés cinq fois (pendant au moins for 5 (*cinq*) minutes, chaque fois) dans une solution de 10 ml d'acide phosphorique/litre d'eau. Les filtres humides ont été soumis à un comptage en présence d'un fluide de scintillation ACS d'Amersham.

*CDK2*/*cycline A* (humain, recombinant, exprimé dans des cellules d'insecte) a été dosé comme décrit pour CDK1/cycline B.

*CDK5lp25* a été reconstitué en mélangeant des quantités égales de CDK5 mammalien recombinant et p25 exprimé dans *E*. *coli* en tant que protéine de fusion GST (glutathione-S-transférase) et purifié par chomatographie d'affinité sur glutathione-agarose (vecteurs fournis par le Docteur L.H. Tsai) (p25 est une version tronquée de p35, l'activateur de CDK5 de 35 kDa). Son activité a été dosée avec de l'histone H1 dans le tampon C comme décrit pour CDK1/cycline B.

*CDK7*/*cycline H* (humain, recombinant, exprimé dans des cellules d'insecte) a été dosé comme décrit pour CDK1/cycline B, mais en utilisant une protéine basique de myéline (MBP) (1mg/ml) en tant que substrat.

*CDK9*/*cycline T* (humain, recombinant, exprimé dans des cellules d'insecte) a été dosé comme décrit pour CDK1/cycline B, mais en utilisant un fragment pRB (a.a.773-928) (3.5µg/dosage) en tant que substrat.

*GSK-3α*/*β* a été purifié à partir de cerveau porcin par chomatographie d'affinité sur une axine immobilisée (Primot et *al*., 2003). Il a été dosé à la suite d'une dilution à 1/100 dans 1 mg BSA/ml et 10 mM DTT, avec 5 µl de substrat de peptide GS-1à 4 µM dans le tampon A, en présence de 15 µM [γ-³³P] ATP (3000 Ci/mmol; 10 mCi/ml) dans un volume final de 30 µl. Après 30 (*trente*) minutes d'incubation à 30°C, les aliquotes de 25 µl du surnageant ont été traités comme décrit ci-dessus.

*CK1δ*/*ε* a été purifié à partir de cerveau porcin par chomatographie d'affinité sur un fragment d'axine immobilisée. Il a été dosé comme décrit pour CDK1 mais en utilisant un substrat de peptide spécifique à CK1.

*DYRK1A* kinase provenant de rat, recombinant, exprimée chez *E*. *coli* comme protéine de fusion GST, a été purifiée par chromatographie d'affinité sur billes de glutathion-agarose et dosée comme décrit pour la CDK1/cycline B avec la protéine basique de la myéline (1 mg/ml) comme substrat.

Cette étude a permis de mettre en évidence l'activité inhibitrice particulièrement marquée des mériolines vis-à-vis des CDKs et en particulier de CDK9, dont peu d'inhibiteurs ont été décrits. Les mériolines inhibent également GSK-3. Ces deux familles d'enzymes sont particulièrement impliquées dans la maladie d'Alzheimer et d'autres maladies neurodégénératives. Elles inhibent également la kinase DYRK1A, impliquée directement dans la maladie d'Alzheimer et les problèmes liés à la trisomie 21.

Il ressort également de cette étude que les mériolines 3, 4, 5, 6, 15, 16, 17, 18, 22 et 23 montrent le meilleur effet inhibiteur la prolifération cellulaire et que la mérioline 19 montre un effet particulièrement actif et sélectif sur l'enzyme DYRK1A, ce qui en fait un candidat particulièrement intéressant pour le traitement des maladies neurodégénératives, et en particulier la maladie d'Alzheimer et la trisomie 21.

### II) BIOLOGIE CELLULAIRE

### Anticorps et réactifs chimiques

Un kit Cell Titer 96^{®} contenant le réactif MTS a été acheté à Promega (Madison, WI, USA). Le cocktail inhibiteur de protéase provenait de Roche et le sérum de veau foetal (FCS) provenait d'Invitrogen. Les réactifs non listés provenaient de Sigma, sauf s'il en est indiqué autrement.

### Lignes cellulaires et conditions de culture.

La ligne de cellules du neuroblastome humain SH-SY5Y a été mise à croître dans un milieu DMEM avec de la L-glutamine provenant d'Invitrogen (Cergy Pontoise, France), des antibiotiques et 10% en volume of FCS provenant d'Invitrogen.

Les cellules HEK293 ont été mises à croître dans un milieu MEM avec du Glutamax provenant d'Invitrogen, des antibiotiques et 10% en volume de FCS.

Les conditions générales de culture étaient une atmosphère de 5% en CO₂ et une température de 37°C.

Les plaques de culture et autres outils en plastique jetables ont été fournis par Corning (Corning, NY, USA). Les traitements médicamenteux ont été effectués sur des cultures en croissance exponentielle au temps, et concentrations indiqués. Les expériences de contrôles ont été mises en oeuvre en utilisant également des dilutions appropriées de DMSO.

### Mise en évidence de la viabilité des cellules.

La viabilité des cellules a été déterminée en mesurant la réduction de 3-(4,5-diméthylthiazol-2-yl)-5-(3-carboxyméthoxyphényl)-2-(4-sulfophényl)-2*H*-tétrazolium (MTS). La procédure était telle que décrite en détails dans Ribas J. et al., 2004, « Cell differentiation, caspase inhibition, and macromolecular synthesis blockage, but not BCL-2 or BCL-XL proteins, protect SH-SYS5 cells from apoptosis triggered by two CDK inhibitory drugs », Exp. Cell Res. 2004, 195, 9-24.

**Mise en évidence de l'effet inhibiteur des mériolines de l'invention sur les protéines kinases CDK1, CDK2, CDK5, CDK9, GSK3, CK1, DYRK1A et sur les lignées de cellules SH-SY5Y et HEK293.**

Des tests ont été effectués avec les mériolines de l'invention numérotées 2 à 10, 13 à 19 et 22 à 23 et à titre de comparaison, sur la varioline B, et la mérioline 1 et la mérioline 12 décrites dans l'art antérieur.

Ainsi la varioline B et les mériolines ont été testées à diverses concentrations dans les dosages de sept kinases, comme décrit ci-dessus.

Les valeurs IC₅₀ ont été calculées à partir des courbes dose-réponse et sont reportées au tableau 1 suivant en micromoles.

Les données des kinases pour les méridianines proviennent de Gompel et al., Bioorg. Med. Chem. Leil. 2004, 14, 1703-1707.

Ces composés ont également été testés à diverses concentrations quant à leurs effets sur les cellules SH-SY5Y et HEK293. La survie des cellules a été estimée 48 (quarante huit) heures après l'addition de chaque composé en utilisant le dosage de réduction de MTS. Les valeurs IC₅₀ ont été calculées à partir des courbes dose-réponse et sont reportées en micromoles (moyenne ± écart type. de deux mesures indépendantes effectuées en triple).

Les mériolines de l'invention et les mériolines 1 et 12 de l'art antérieur testées ont la formule I suivante : 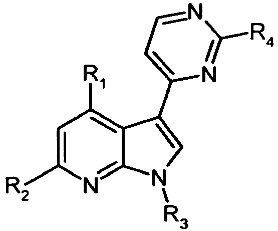 dans laquelle les substituants R₁, R₂, R₃ et R₄ sont les suivants :

| | R₁ | R₂ | R₃ | R₄ |
|---|---|---|---|---|
| Mérioline 1 | H | H | H | NH₂ |
| Mérioline 2 | OH | H | H | NH₂ |
| Mérioline 3 | OCH₃ | H | H | NH₂ |
| Mérioline 4 | OC₂H₅ | H | H | NH₂ |
| Mérioline 5 | OC₃H₇ | H | H | NH₂ |
| Mérioline 6 | OCH(CH₃)₂ | H | H | NH₂ |
| Mérioline 7 | O(CH₂)₂OCH₃ | H | H | NH₂ |
| Mérioline 8 | OH | H | CH₃ | NH₂ |
| Mérioline 9 | OCH₃ | H | CH₃ | NH₂ |
| Mérioline 10 | Cl | H | H | NH₂ |
| Mérioline 12 | OCH₃ | H | H | SCH₃ |
| Mérioline 13 | OH | H | H | H |
| Mérioline 14 | OCH₃ | H | H | H |
| Mérioline 15 | OCH₂C₆H₅ | H | H | NH₂ |
| Mérioline 16 | OCH₂C₆H₁₁ | H | H | NH₂ |
| Mérioline 17 | OC₆H₁₁ | H | H | NH₂ |
| Mérioline 18 | C≡C-(CH₂)₂-CH₃ | H | H | NH₂ |
| Mérioline 19 | (CH₂)₄-CH₃ | H | H | NH₂ |
| Mérioline 22 | (CH₂)₂-C₆H₅ | H | H | NH₂ |
| Mérioline 23 | C₆H₅ | H | H | NH₂ |

Les résultats obtenus sont reportés au tableau 1 suivant:

**Tableau 1**

| **Composés** | **CDK1/ cycline B** | **CDK2/ cyclineA** | **CDK5/ p25** | **CDK9/ cycline T** | **GSK-3α/β** | **CK1** | **DYRK1A** |
|---|---|---|---|---|---|---|---|
| Varioline B | 0,06 | 0,08 | 0,09 | 0,026 | 0,07 | 0,005 | 008 |
| Mérioline 1 | 0,78 | 0,09 | 0,51 | 0,026 | 0,63 | 0,2 | 0,13 |
| Mérioline 2 | 0,057 | 0,018 | 0,050 | 0,018 | 0,40 | 0,05 | 0,035 |
| Mérioline 3 | 0,17 | 0,011 | 0,17 | 0,006 | 0,23 | 0,2 | 0,029 |
| Mérioline 4 | 0,01 | 0,007 | 0,005 | 0,007 | 0,03 | 0,1 | 0,032 |
| Mérioline 5 | 0,007 | 0,003 | 0,003 | 0,0056 | 0,025 | 0,2 | 0,037 |
| Mérioline 6 | 0,008 | 0,051 | 0,003 | 0,0056 | 0,021 | 0,14 | 0,040 |
| Mérioline 7 | 35,0 | > 10 | 14,0 | 5,30 | 63,0 | 100,0 | > 10 |
| Mérioline 8 | 1,20 | 1,8 | 5,50 | 1,2 | 4,60 | 2,3 | 1,2 |
| Mérioline 9 | 25,0 | > 10 | 73,0 | > 10 | > 100 | > 10 | > 10 |
| Mérioline 10 | 0,24 | 0,06 | 0,23 | 0,05 | 2,00 | 3,0 | 0,13 |
| Mérioline 12 | 1,80 | 2,1 | 2,30 | 1,10 | 7,00 | 0,6 | 1,0 |
| Mérioline 13 | 0,9 | 0,7 | 0,7 | 0,25 | 1,8 | 0,9 | 0,9 |
| Mérioline 14 | 1,3 | 0,8 | 1,3 | 0,22 | 1,1 | 0,6 | 0,23 |
| Mérioline 15 | 0,0023 | 0,0016 | 0,002 | 0,0072 | 0,0033 | 0,13 | 0,040 |
| Mérioline 16 | 0,0022 | 0,0012 | 0,003 | - | 0,0060 | > 0,1 | 0,045 |
| Mérioline 17 | 0,0029 | 0,0021 | 0,0021 | - | 0,0041 | > 0,1 | 0,026 |
| Mérioline 18 | 0,011 | 0,005 | 0,0045 | - | 0,082 | 1,3 | 0,020 |
| Mérioline 19 | 0,350 | 0,195 | 0,20 | - | 1,750 | 8,1 | 0,056 |
| Mérioline 22 | 0,200 | 0,073 | 0,130 | - | 0,800 | - | 0,073 |
| Mérioline 23 | 0,010 | 0,009 | 0,007 | - | 0,080 | - | 0,050 |

On constate à partir du tableau 1 que les mériolines 3 à 6, 15 à 18 et 22 à 23 présentent un effet d'inhibition des protéines kinases CDK1, CDK2 et CDK9 très supérieur à la varioline B et à la mérioline 1, ce qui indique leur effet antiprolifératif entraînant la mort cellulaire.

De la même façon, on voit à partir du tableau 1 que les mériolines 3 à 6, et 15 à 19 et 22 à 23 présentent un effet d'inhibition des protéines kinases CDK5, GSK3, CK1 et DYRK1A également très supérieur à celui de la varioline B, de la mérioline 1 et de la mérioline 12, ce qui indique un effet neuroprotecteur puissant.

L'effet apoptotique des mériolines 3 à 6, 15 à 18 et 22 à 23 est démontré par les résultats obtenus sur la survie des cellules de neuroblastomes SH-SY5Y et HEK293, qui sont reportés au tableau 2 ci-après.

Cet effet exceptionnellement efficace sur les sept protéines kinases testées sur les cellules de neuroblastomes est particulièrement marqué pour la mérioline 3, la mérioline 4, la mérioline 5, la mérioline 6, la mérioline 15, la mérioline 16, la mérioline 17, la mérioline 18, la mérioline 22 et la mérioline 23.

De plus, on voit à partir du tableau 1 que la mérioline 19 présente un effet particulièrement efficace sur la protéine kinase DYKR1A avec un effet modéré sur les autres protéines kinases, ce qui montre non seulement son efficacité mais également sa sélectivité sur cette kinase et la rend particulièrement appropriée pour le traitement des maladies neurodégénératives telles que la maladie d'Alzheimer et la trisomie 21.

**Mise en évidence de l'activité antiproliférative des mériolines de l'invention.**

Des cellules SH-SY5Y ont été exposées pendant 24 heures à des concentrations croissantes de chaque mérioline, et à titre de comparaison de varioline B.

La survie des cellules a été estimée par le dosage du taux de réduction de MTS induit par cette exposition.

Les résultats obtenus sur chaque mérioline de l'invention et sur les mériolines 1 et 12 et la varioline B de l'art antérieur sont reportés au tableau 2 suivant.

Ces résultats sont exprimés sous la forme de la valeur IC₅₀, micromoles.

**Tableau 2**

| **Composé** | **SH-SY5Y** | **Composé** | **SH-SY5Y** |
|---|---|---|---|
| Varioline B | 0,24 | Mérioline 12 | > 100 |
| Mérioline 1 | 0,67 | Mérioline 13 | 28,0 |
| Mérioline 2 | 0,41 | Mérioline 14 | 10,2 |
| Mérioline 3 | 0,073 | Mérioline 15 | 0,013 |
| Mérioline 4 | 0,081 | Mérioline 16 | 0,022 |
| Mérioline 5 | 0,026 | Mérioline 17 | 0,027 |
| Mérioline 6 | 0,038 | Mérioline 18 | 0,100 |
| Mérioline 7 | > 100 | Mérioline 19 | 13,0 |
| Mérioline 8 | > 30 | Mérioline 22 | 2,4 |
| Mérioline 9 | > 30 | Mérioline 23 | 0,088 |
| Mérioline 10 | 1,92 | | |

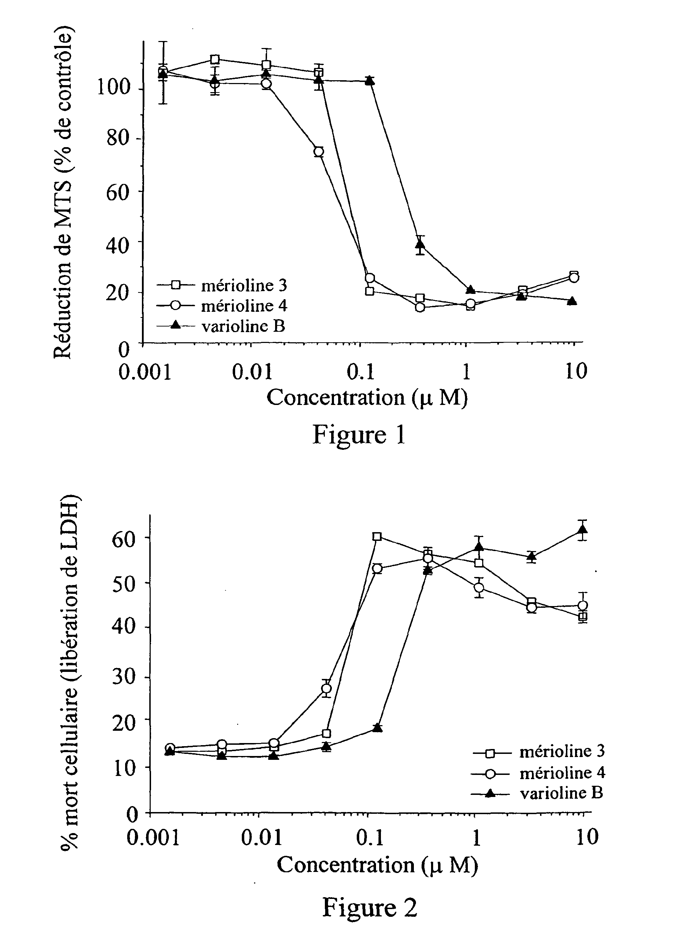

De plus, la Figure 1 représente les résultats obtenus pour la mérioline 3 et la mérioline 4. Ces résultats sont exprimés en % de survie par rapport à des cellules non traitées.

Comme on le voit à partir du tableau 2 et le la Figure 1, les mériolines de l'invention ont un effet antiprolifératif bien supérieur à celui de la varioline B.

**Mise en évidence de l'effet apoptotique des mériolines de l'invention.**

La même expérience que ci-dessus a été menée, mais on a mesuré les taux de libération de LDH induits par les mériolines 3 et 4 de l'invention et la varioline B.

Le taux de libération de LDH est représentatif du taux de mortalité des cellules. Plus le taux de LDH libéré est élevé, plus la mortalité des cellules est élevée.

Les résultats obtenus sont représentés en Figure 2.

On voit, à partir de la Figure 2, que les mériolines de l'invention induisent la mort cellulaire.

### Activité antitumorale in vivo.

Des souris mâles nues athymiques (âgées de 5 à 6 semaines) ont été obtenues de l'Institut National du Cancer. Les souris ont été logées dans l'animalerie de la division de l'Université de Médecine Comparative de Georgetown. Tous les travaux sur les animaux ont été effectués sous des protocoles approuvés par le Comité d'Utilisation de Soins des Animaux de l'Université de Georgetown. Les souris ont été inoculées par injection sous cutanée dans le flanc postérieur droit avec 4 x 10⁶ cellules A4573 dans 100 µL de matrice de membrane de base Matrigel (Becton Dickinson). Des xénogreffes ont été mises à croître à un volume moyen de tumeur de 129 ± 30 mm³. Les composés testés ont été d'abord dissous dans soit du méthanol absolu soit du DMSL (1 volume). Une solution porteuse a été produite en utilisant un diluant contenant 10% Tween 80, 20% *N-N-*diméthylacetamide, et 70% polyéthylène glycol 400 (Fisher Scientific, Pittsburgh, PA). Les souris ont été réparties au hasard en deux groupes (six animaux par groupe) et le traitement a été initié. Un groupe a été traité avec la mérioline 3, administrée par injection intra péritonéale une fois par jour et à une dose 50 mg/kg, pendant soit cinq jours, soit deux séries de cinq jours avec une pause de deux jours entre chaque série de cinq jours. Le groupe de contrôle a reçu des injections intra péritonéales de la solution porteuse suivant des programmes identiques. Toutes les souris ont été sacrifiées par asphyxie avec CO₂. Les souris traitées par la Mérioline 3 ont été euthanasiées soit 7 jours après la première injection soit jusqu'à quatre semaines après la fin du traitement. A ces temps, les tumeurs ont été enlevées, mesurées et préparées pour des dosages TUNEL. Les volumes primaires de tumeur ont été calculés par la formule *V* = (1/2)*a* x *b*², où *a* est l'axe tumoral le plus long et *b* est l'axe tumoral le plus court. Les valeurs sont données sous la forme de valeurs moyennes ± écart type dans des expériences quantitatives. L'analyse statistique des différences entre les groupes a été effectuée par un ANOVA une voie suivi par un test de Student t non apparié.

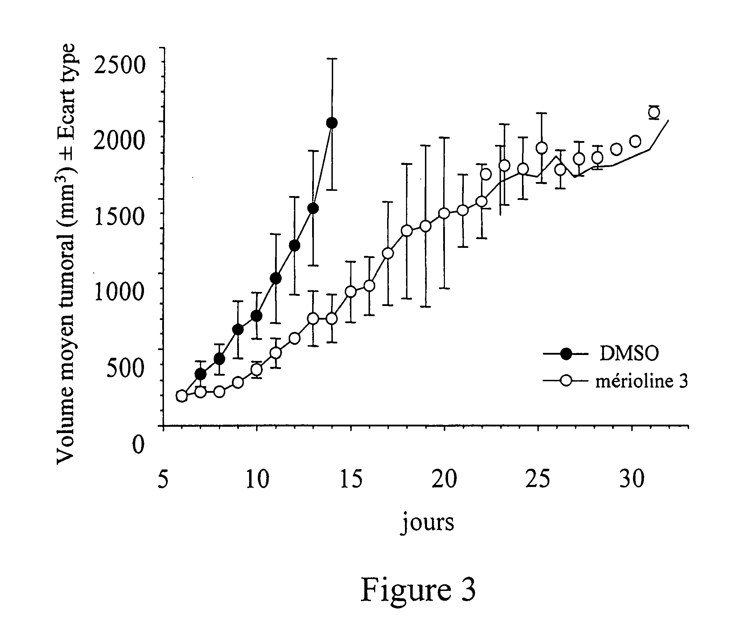

Les résultats sont montrés à la Figure 3 annexée.

Comme on le voit en Figure 3, le volume moyen tumoral, en mm³, n'augmente que peu avec le temps lorsque la tumeur est exposée à la mérioline 3, en comparaison à l'exposition à un témoin, le DMSO.

Les effets des mériolines 2 et 3 sur la survie de diverses lignes de cellules ont également été testés à diverses concentrations pour déterminer leurs effets sur huit lignées de cellules différentes. La survie des cellules a été exprimée quarante huit heures après l'addition de chaque mérioline, en utilisant le test de réduction de MTS. Les valeurs IC50 ont été calculées à partir courbes dose-réponse qui sont reportées en micromoles au tableau 3 ci-dessous.

**Tableau 3**

| | **Survie des cellules (IC₅₀, µM) (réduction de MTS)** | |
|---|---|---|
| **Lignées de cellules** | **Mérioline 2** | **Mérioline 3** |
| HCT116 (colon) | 0,080 | 0,94 |
| MDA-MB-231 (sein) | 1,8 | 19,00 |
| PC3 (prostate) | 17,30 | 95,00 |
| Huh7 (hépatome) | 1,0 | 0,12 |
| F1 (hépatome) | 2,0 | 0,26 |
| SH-SY5Y (neuroblastome) | 0,41 | 0,072 |
| HEK293 (rein embryonnaire) | 2,6 | 0,38 |
| Fibroblastes de prépuce humain | 20 | 8,00 |

Ainsi, on constate que les mériolines de l'invention provoquent la mort cellulaire de lignées de cellules impliquées dans différents cancers.

En conclusion, les mériolines de l'invention induisent la mort de lignées de cellules en particulier impliquées dans les processus cancéreux, par apoptose. Cependant, ce processus n'est pas le seul impliqué dans le processus induisant la mort cellulaire par les mériolines, les mériolines agissant également en tant qu'inhibiteurs puissants de la prolifération de ces cellules.

Ces propriétés d'inhibition de la prolifération des cellules et d'induction de la mort cellulaire sont certes très efficaces pour le traitement des tumeurs mais pas uniquement.

En effet, ces propriétés rendent les mériolines de l'invention appropriées pour être utilisées dans des pathologies non cancéreuses, telles que les maladies rénales, dont les glomérulonéphrites, les polykystoses rénales, les inflammations, les diabètes de type II et même les maladies neurodégénératives telles que la maladie d'Alzheimer.

De plus, bien que dans les exemples qui précèdent on a mis en évidence l'activité antiproliférative, apoptotique et antitumorale des mériolines de l'invention prises seules, il apparaîtra clairement à l'homme du métier que les mêmes effets sont obtenus avec des associations de plusieurs mériolines selon l'invention les unes avec les autres et également avec des associations d'au moins une mérioline selon l'invention avec un autre agent, en particulier anti-tumoral tel que, par exemple, le taxol.

Egalement, bien que dans les exemples qui précèdent seules les activités des composés mériolines de formule I aient été testées, il apparaîtra clairement à l'homme de l'art que les sels pharmaceutiquement acceptables de ces composés de formule I auront les mêmes activités et pourront présenter des avantages supplémentaires, tels qu'une meilleure solvabilité dans un solvant physiologiquement acceptable, des effets secondaires moindres, etc.

Les sels pharmaceutiquement acceptables appropriés sont bien connus dans l'art. Il s'agit en particulier des sels chlorhydrate, bromhydrate, sulfate, hydrogénosulfate, maléate, fumarate des composés de formule 1.

## Revendications

1. Composés de Formule I suivante: 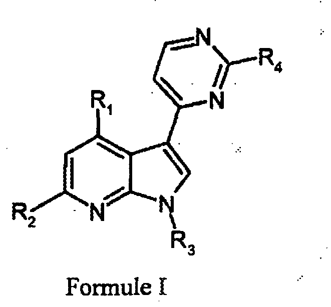
- R₁ est choisi parmi un halogène ou un groupe alkyle en C₁₋C₁₀ substitué ou non substitué, un groupe (alkyle en C₁-C₁₀)cycloalkyle en C₅-C₈; un groupe (alkyle en C₁-C₁₀)aryle en C₆-C₁₈, un groupe (alkyle en C₁-C₁₀)hétérocyle en C₅-C₁₂ aromatique ou non aromatique comportant un à trois hétéroatomes, un groupe alcoxy en C₁-C₁₀ substitué ou non substitué, un groupe fluoroalcoxy en C₁-C₁₀, un groupe (alcoxy en C₁-C₁₀)alcoxy en C₁-C₁₀, un groupe cycloalcoxy en C₅-C₈, un groupe (alcoxy en C₁-C₁₀)cycloalkyle en C₅-C₈, un groupe (alcoxy en C₁-C₁₀)aryle en C₆-C₁₈, un groupe (alcoxy en C₁-C₁₀)hétérocyle en C₅-C₁₂ aromatique ou non aromatique comportant un à trois hétéroatomes, un groupe alcényle en C₂-C₁₀ substitué ou non substitué, un groupe (alcényle en C₂-C₁₀)cycloakyle en C₅-C₈, un groupe (alcényle en C₂-C₁₀)aryle en C₆-C₁₈, un groupe (alcényle en C₂-C₁₀)hétérocycle en C₅-C₁₂ aromatique ou non aromatique comportant un à trois hétéroatomes, un groupe alcynyle en C₂-C₁₀ substitué ou non substitué, un groupe (alcynyle en C₂-C₁₀)cycloalkyle en C₅-C₈, un groupe (alcynyle en C₂-C₁₀)aryle en C₆-C₁₈, un groupe (alcynyle en C₂-C₁₀)hétérocyle en C₅-C₁₂ aromatique ou non aromatique comportant un à trois hétéroatomes, -OH, -OCORₐ, -CN, -NO₂, -SRₐ, - NRₐR_{b},=NHCORₐ, -NHSO₂Rₐ,-NHSO₂Rₐ,-NHCONRₐR_{b}, NHCO₂Rₐ, un groupe phényle, un groupe aryle en C₆-C₁₈, un groupe hétérocycle en C₅-C₁₂ aromatique ou non aromatique comportant de un à trois hétéroatomes,
- R₂ représente un atome d'hydrogène ou d'halogène, ou indépendamment de R₁, un groupement tel que défini pour R₁,
- R₃ représente H ou un groupe -SO₂Rₐ ou -CORa, ou alkyle en C₁-C₁₀,
- R₄ représente un atome d'hydrogène ou un groupe NH₂,
- Rₐ et R_{b} représentent chacun indépendamment l'un de l'autre un atome d'hydrogène, ou un groupe éventuellement substitué choisi parmi un alkyle en C₁-C₁₀, un alcényle en C₂-C₁₀, un alcynyle en C₂-C₁₀, un cycloalkyle en C₅-C₈, un (cycloalkyle en C₅-C₈)alkyle en C₁-C₁₀, un (cycloalkyle en C₅-C₈)alcényle en C₂-C₁₀, un (cycloalkyle en C₅-C₈)alcynyle en C₂-C₁₀, un (hétérocycle en C₅-C₁₂)alkyle en C₁-C₁₀, un (hétérocycloalkyle en C₅-C₁₂)alcényle en C₂-C₁₀, un (hétérocycloalkyle en C₅-C₁₂)alcényle en C₂-C₁₀, ou bien Rₐ et R_{b} sont liés entre eux pour former, avec l'atome d'azote auquel ils sont liés, un hétérocycle éventuellement substitué choisi parmi un groupement pyrrolidinyle, pipéridinyle, pipérazinyle et morpholinyle,
et les sels pharmaceutiquement acceptables de ces composés de formule I.

2. Composés selon la revendication 1 ayant la formule I dans laquelle:
- R₁ est choisi parmi un halogène ou un groupe alkyle en C₁-C₁₀, alcoxy en C₁-C₁₀ non substitué ou substitué, fluoroalcoxy en C₁-C₁₀, alcoxy en C₁-C₁₀-alcoxy en C₁-C₁₀, cycloalcoxy en C₅-C₈, -OH, -OCORₐ, -CN, -NO₂, -SRₐ, -NRₐRₕ, - NHCORₐ, -NHSO₂Rₐ, -NHCONRₐR_{b}, NHCO₂Rₐ, phényle, aryle, hétéroaryle,
- R₂ représente un atome d'hydrogène ou indépendamment de R₁, un halogène ou un groupe tel que défini pour R₁,
- R₃ représente H ou un groupe -SO₂Rₐ ou -CORₐ, ou alkyle,
- R₄ représente un atome d'hydrogène ou un groupe NH₂,
- Rₐ et R_{b} représentent chacun indépendamment l'un de l'autre un atome d'hydrogène, ou un groupe éventuellement substitué choisi parmi les groupes alkyle, àlcényle, alcynyle, cycloalkyle, cycloalkylalkyle, cycloalkylalcényle, cycloalkylalcynyle, hétérocycloalkyle, hétérocycloalkylalkyle, hétérocycloalkylalcényle, ou bien Rₐ et R_{b} sont liés entre eux pour former, avec l'atome d'azote auquel ils sont liés, un hétérocycle éventuellement substitué choisi parmi un groupement pyrrolidinyle, pipéridinyle, pipérazinyle ou morpholinyle,

3. Composés selon la revendication 1 ou 2 de formule I dans laquelle R₃ est H ou CH₃.

4. Composés selon la revendication 1 ou 3 ayant la formule I dans laquelle :
- R₁ est choisi parmi OH, Cl ou un groupe méthoxy, éthoxy, propoxy, butyloxy, isopropoxy, benzyloxy, cyclohéxylméthoxy, cyclohéxyloxy, 2-propyléthynyle, 2-butyléthynyle, 2-cyclohexyléthynyle, phénéth-1-ynyle, phényle, pentyle ou phényléthyle,
- R₂ est H ou Bᵣ,
- R₃ est H ou CH₃, et
- R₄ est H ou NH₂.

5. Composés selon l'une quelconque des revendications 1 et 3 à 4 de formule 1 dans laquelle :
- R₁ est choisi parmi un groupe méthoxy, éthoxy, propoxy, isopropoxy, benzyloxy, cyclohéxylméthoxy, cyclohéxyloxy, 2-propyléthynyle, pentyle,phényle et phényléthyle,
- R₂ est H ou Bᵣ,
- R₃ est H ou CH₃, et
- R₄ est H ou NH₂.

6. Composé selon l'une quelconque des revendications précédentes choisi parmi :
- la 3-[(2-amino)pyrimidin-4-yl]-4-méthoxy-1*H*-pyrrolo[2,3-*b*]pyridine de formule Ia suivante : 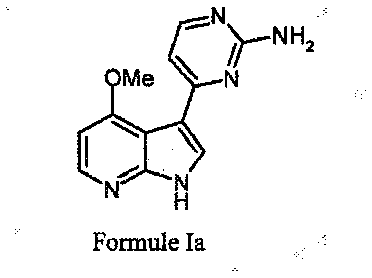
- la 3-[(2-amino)pyrimidin-4-yl]-4-éthoxy-1*H*-pyrrolo[2,3-*b*]pyridine de formule Ib suivante : 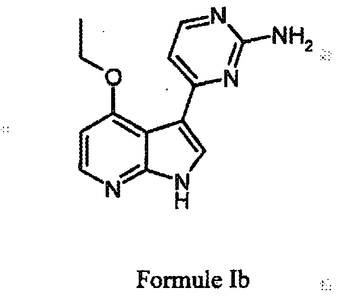
- la 3-[(2-amino)pyrimidin-4-yl]-4-propoxy-1*H*-pyrrolo[2,3-*b*]pyridine de formule Ic suivante : 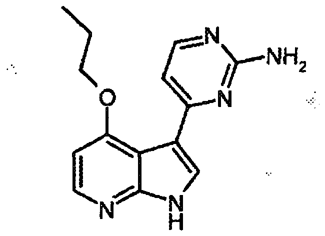 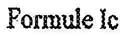
- la 3-[(2-amino)pyrimidin-4-yl]-4-(1-méthyléthoxy)-1*H*-pyrrolo[2,3-*b*]pyridine de formule Id suivante : 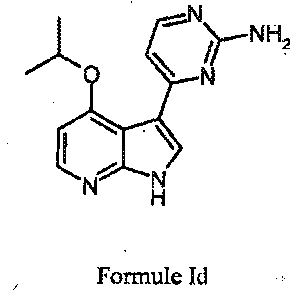
- la 3[(2-amino)pyrimidin-4-yl]-4-(benzyloxy)-1*H*-pyrrolo[2,3-*b*]pyridine de formule Ie suivante : 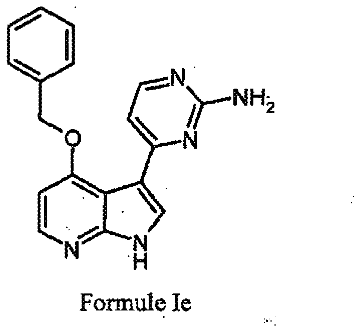
- 3-[(2-amino)pyrimidin-4-yl]-4-(1-cyclohéxylméthoxy)-1*H*-pyrrolo[2,3-*b*]pyridine de formule If suivante : 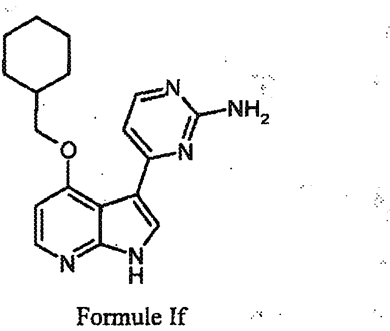
- la 3-[(2-amino)pyrimidin-4-yl]-4-(cyclohéxyloxy)-1*H*-pyrrolo[2,3-*b*]pyridine de formule Ig suivante : 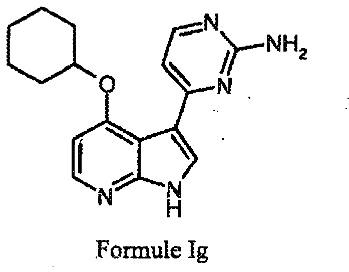
- la 3-[(2-amino)pyrimidin-4-yl]-4-(pent-1-ynyl)-1*H*-pyrrolo[2,3-*b*]pyridine de formule Ih suivante : 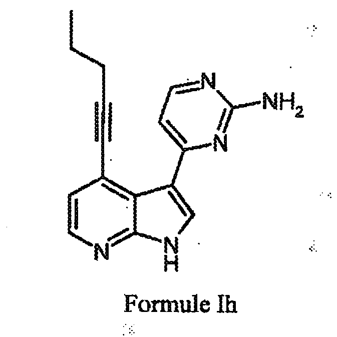
- la 3-[(2-amino)pyrimidin-4-yl]-4-pentyl-1*H*-pyrrolo[2,3-*b*]pyridine de formule Ij suivante : 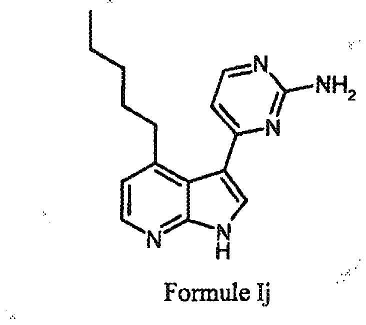
- la 3-[(2-amino)pyrimidin-4-yl]-4-phénéthyl-1*H*-pyrrrolo[2,3-*b*]pyridine de formule Ik suivante : 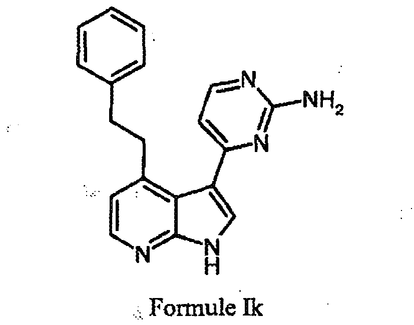
- la 3-[(2-amino)pyrimidin-4-yl]-4-phényl-1*H*-pyrrrolo[2,3-*b*]pyridine de formule Im suivante : 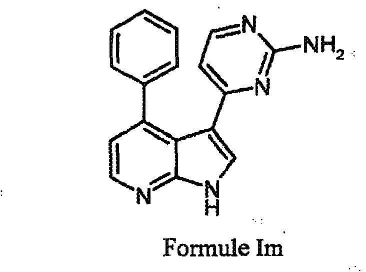
et ses sels pharmaceutiquement acceptables.

7. Composé selon l'une quelconque des revendications 1 à 5 choisi parmi
- 3-[(2-amino)pyrimidin-4-yl]-4-(4-méthoxybenzyloxy)-1*H*-pyrrolo[2,3-*b*]pyridine
- 3-[(2-amino)pyrimidin-4-yl]-4-(3-méthoxybenzyloxy)-1*H*-pyrrolo[2,3-*b*]pyridine
- 3-[(2-amino)pyrimidin-4-yl]-4-(2-méthoxybenzyloxy)-1*H*-pyrrolo[2,3-*b*]pyridine
- 3-[(2-amino)pyrimidin-4-yl]-4-(4-chlorobenzyloxy)-1*H*-pyrrolo[2,3-*b*]pyridine
- 3-[(2-amino)pyrimidin-4-yl]-4-(3-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(pipéridine-4-ylméthoxy)-1*H*-pyrrolo[2,3-*b*]pyridine
- 3-[(2-amino)pyrimidin-4-yl]-4-(1-méthanesulfonylpipéridin-4-ylméthoxy)-1*H*-pyrrolo[2,3-*b*]pyridine

8. Procédé de synthèse des composés selon l'une quelconque des revendications 1 à 7, **caractérisé en ce qu'**il comprend une étape dans laquelle au moins un composé de formule II est utilisé, la formule II étant définie comme suit : 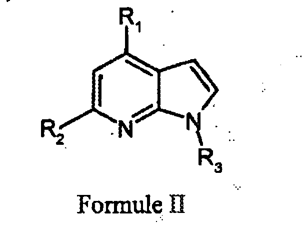 dans laquelle
- R₁ est un groupe éthoxy, propoxy, butyloxy, isopropoxy, benzyloxy, cyclohéxylméthoxy, cyclohéxyloxy,
- R₂ est H et,
R₃ est H.

9. Composé de formule I selon l'une quelconque des revendications 1 à 7, ou un de ses sels pharmaceutiquement acceptables, pour utilisation comme médicament.

10. Composition pharmaceutique **caractérisée en ce qu'**elle comprend au moins un composé de formule I selon l'une quelconque des revendications 1 à 7 ou au moins un de ses sels pharmaceutiquement acceptables et un excipient pharmaceutiquement acceptable.

11. Utilisation d'au moins un composé selon l'une quelconque des revendications 1 à 7 ou d'au moins un de ses sels pharmaceutiquement acceptables pour la fabrication d'un médicament pour le traitement des désordres et maladies liés à une prolifération anormale des cellules, notamment choisi parmi une tumeur, et des maladies rénales, telles que la glomérulonéphrite ou les polykystoses rénales.

12. Utilisation d'au moins un composé selon l'une quelconque des revendications 1 à 7 ou d'au moins un de ses sels pour la fabrication d'un médicament pour le traitement de la maladie d'Alzheimer.

13. Utilisation du composé de formule (Ij) de la revendication 6 ou d'au moins un de ses sels pour la fabrication d'un médicament pour le traitement de la trisomie 21.

## Claims

1. A compound of following formula I: 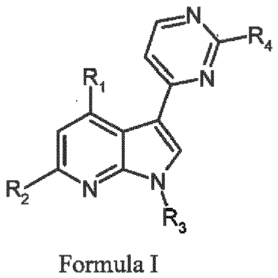 in which:
- R₁ is chosen from a halogen or a substituted or unsubstituted C₁-C₁₀ alkyl group, a C₅-C₈ (C₁-C₁₀ alkyl)cycloalkyl group, a C₆-C₁₈ (C₁-C₁₀ alkyl)aryl group, an aromatic or nonaromatic C₅-C₁₂ (C₁-C₁₀ alkyl)heterocyclyl group comprising from one to three heteroatoms, a substituted or unsubstituted C₁-C₁₀ alkoxy group, a C₁-C₁₀ fluoroalkoxy group, a C₁-C₁₀ (C₁-C₁₀ alkoxy)alkoxy group, a C₅-C₈ cycloalkoxy group, a C₅-C₈ (C₁-C₁₀ alkoxy)cycloalkyl group, a C₆-C₁₈ (C₁-C₁₀ alkoxy)aryl group, an aromatic or nonaromatic C₅-C₁₂ (C₁-C₁₀ alkoxy)heterocyclyl group comprising from one to three heteroatoms, a substituted or unsubstituted C₂-C₁₀ alkenyl group, a C₅-C₈ (C₂-C₁₀ alkenyl)cycloalkyl group, a C₆-C₁₈ (C₂-C₁₀ alkenyl)aryl group, an aromatic or nonaromatic C₅-C₁₂ (C₂-C₁₀ alkenyl)heterocyclyl group comprising from one to three heteroatoms, a substituted or unsubstituted C₂-C₁₀ alkynyl group, a C₅-C₈ (C₂-C₁₀ akynyl)cycloalkyl group, a C₆-C₁₈ (C₂-C₁₀ alkynyl)aryl group, an aromatic or nonaromatic C₅-C₁₂ (C₂-C₁₀ alkynyl)heterocyclyl group comprising from one to one to three heteroatoms, an -OH group, an -OCORₐ group, a -CN group, an -NO₂ group, an -SRₐ group, an -NRₐR_{b} group, an -NHCORₐ group, an -NHSO₂Rₐ group, an -NHSO₂Rₐ group, an -NHCONRₐR_{b} group, an -NHCO₂Rₐ group, a phenyl group, a C₆-C₁₈ aryl group or an aromatic or nonaromatic C₅-C₁₂ heterocyclyl group comprising from one to three heteroatoms,
- R₂ represents a hydrogen or halogen atom or, independently of R₁, a group as defined for R₁,
- R₃ represents H or an -SO₂Rₐ or -CORₐ or C₁-C₁₀ alkyl group,
- R₄ represents a hydrogen atom or an NH₂ group,
- Rₐ and R_{b} represent, each independently of one another, a hydrogen atom or an optionally substituted group chosen from a C₁-C₁₀ alkyl, a C₂-C₁₀ alkenyl, a C₂-C₁₀ alkynyl, a C₅-C₈ cycloalkyl, a C₁-C₁₀ (C₅-C₈ cycloalkyl)alkyl, a C₂-C₁₀ (C₅-C₈ cycloalkyl)alkenyl, a C₂-C₁₀ (C₅-C₈ cycloalkyl)alkynyl, a C₁-C₁₀ (C₅-C₁₂ heterocycle)alkyl, a C₂-C₁₀ (C₅-C₁₂ heterocycloalkyl)alkenyl or a C₂-C₁₀ (C₅-C₁₂ heterocycloalkyl)alkynyl or else Rₐ and R_{b} are bonded together to form, with the nitrogen atom to which they are bonded, an optionally substituted heterocycle chosen from a pyrrolidinyl, piperidinyl, piperazinyl and morpholinyl group,
and the pharmaceutically acceptable salts of this compound of formula I.

2. The compound as claimed in claim 1 having the formula I, in which:
- R₁ is chosen from a halogen or a C₁-C₁₀ alkyl, unsubstituted or substituted C₁-C₁₀ alkoxy, C₁-C₁₀ fluoroalkoxy, C₁-C₁₀ (C₁-C₁₀ alkoxy) alkoxy, C₅-C₈ cycloalkoxy, -OH, -OCORₐ, -CN, -NO₂, -SRₐ, -SRₐ, -NRₐR_{b}, -NHCORₐ, -NHCO₂Rₐ, -NHSO₂Rₐ, -NHCONRₐR_{b}, -NHCO₂Rₐ, phenyl, aryl or heteroaryl group,
- R₂ represents a hydrogen atom or, independently of R₁, a halogen or a group as defined for R₁,
- R₃ represents H or an -SO₂Rₐ or -CORₐ or alkyl group,
- R₄ represents a hydrogen atom or an NH₂ group,
- Rₐ and R_{b} represent, each independently of one another, a hydrogen atom or an optionally substituted group chosen from alkyl, alkenyl, alkynyl, cycloalkyl, cycloalkylalkyl, cycloalkylalkenyl, cycloalkylalkynyl, heterocycloalkyl, heterocycloalkylalkyl or heterocycloalkylalkenyl groups or else Rₐ and R_{b} are bonded together to form, with the nitrogen atom to which they are bonded, an optionally substituted heterocycle chosen from a pyrrolidinyl, piperidinyl, piperazinyl or morpholinyl group.

3. The compound as claimed in claim 1 or 2 of formula I, in which R₃ is H or CH₃.

4. The compound as claimed in claim 1 or 3 having the formula I, in which:
- R₁ is chosen from OH, Cl or a methoxy, ethoxy, propoxy, butyloxy, isopropoxy, benzyloxy, cyclohexylmethoxy, cyclohexyloxy, 2-propylethynyl, 2-butylethynyl, 2-cyclohexylethynyl, pheneth-1-ynyl, phenyl, pentyl or phenylethyl group,
- R₂ is H or Br,
- R₃ is H or CH₃, and
- R₄ is H or NH₂.

5. The compound as claimed in any one of claims 1 and 3 to 4 of formula I, in which:
- R₁ is chosen from a methoxy, ethoxy, propoxy, isopropoxy, benzyloxy, cyclohexylmethoxy, cyclobexyloxy, 2-propylethynyl, pentyl, phenyl and phenylethyl group,
- R₂ is H or Br,
- R₃ is H or CH₃, and
- R₄ is H or NH₂.

6. The compound as claimed in any one of the preceding claims chosen from :
- 3-[(2-amino)pyrimidin-4-yl]-4-methoxy-1*H*-pyrrolo[2,3-*b*]pyridine of following formula Ia: 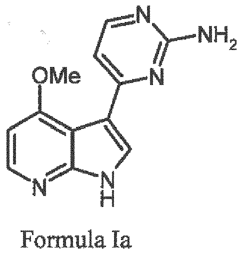
- 3-[(2-amino)pyrimidin-4-yl]-4-ethoxy-1*H*-pyrrolo[2,3-*b*]pyridine of following formula Ib: 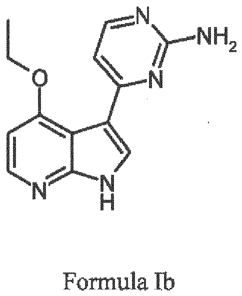
- 3-[(2-amino)pyrimidin-4-yl]-4-propoxy-1*H*-pyrrolo[2,3-*b*]pyridine of following formula Ic: 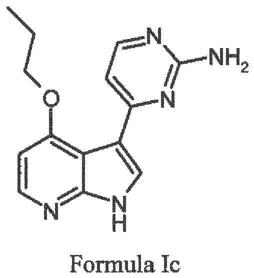
- 3-[(2-amino)pyrimidin-4-yl]-4-(1-methylethoxy)-1*H*-pyrrolo[2,3-*b*]pyridine of following formula Id: 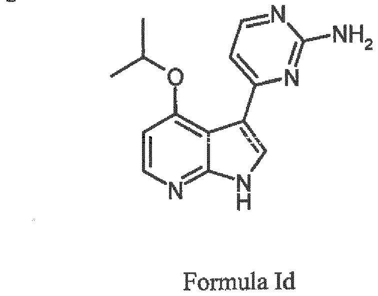
- 3-[(2-amino)pyrimidin-4-yl]-4-(benzyloxy)-1*H*-pyrrolo[2,3-*b*]pyridine of following formula Ie: 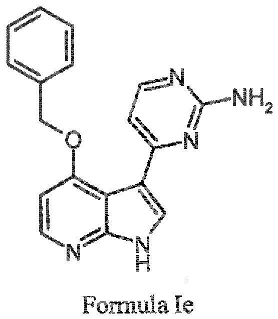
- 3-[(2-amino)pyrimidin-4-yl]-4-(1-cyclohexylmethoxy)-1*H-*pyrrolo[2,3-*b*]pyridine of following formula If: 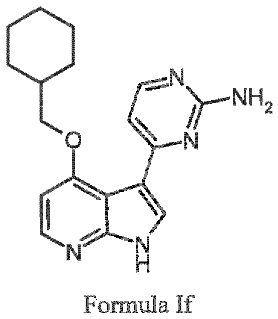
- 3-[(2-amino)pyrimidin-4-yl]-4-(cyclohexyloxy)-1*H*-pyrrolo[2,3-*b*]pyridine of following formula Ig: 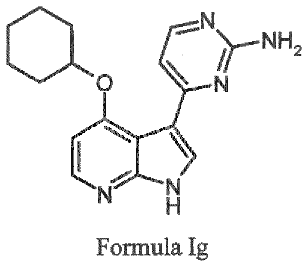
- 3-[(2-amino)pyrimidin-4-yl]-4-(pent-1-ynyl)-1*H*-pyrrolo[2,3-*b*]pyridine of following formula Ih: 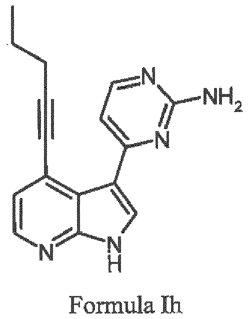
- 3-[(2-amino)pyrimidin-4-yl]-4-pentyl-1*H*-pyrrolo[2,3-*b*]pyridine of following formula Ij: 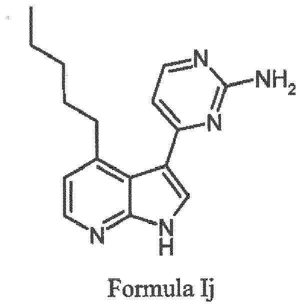
- 3-[(2-amino)pyrimidin-4-yl]-4-phenethyl-1*H*-pyrrolo[2,3-*b*]pyridine of following formula Ik: 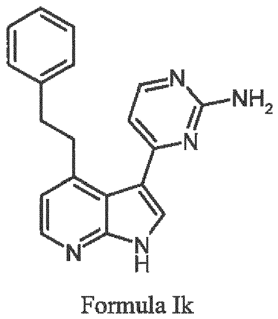
- 3-[(2-amino)pyrimidin-4-yl]-4-phenyl-1*H*-pyrrolo[2,3-*b*]pyridine of following formula Im: 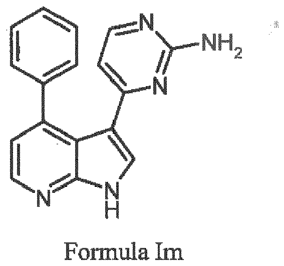
and its pharmaceutically acceptable salts.

7. The compound as claimed in any one of claims 1 to 5 chosen from
- 3-[(2-amino)pyrimidin-4-yl]-4-(4-methoxybenzyloxy)-1*H*-pyrrolo[2,3-*b*]pyridine
- 3-[(2-amino)pyrimidin-4-yl]4-(3-methoxybenzyloxy)-1*H*-pyrrolo[2,3-*b*]pyidine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-chlorobenzyloxy)-1*H*-pyrrolo[2,3-*b*]pyridine
- 3-[(2-amino)pyrimidin-4-yl]-4-(2-fluorobenzyloxy)-1*H*-pyrrolo[2,3-*b*]pyridine
- 3-[(2-amino)pyrimidin-4-yl]-4-(2-flulorobenzyloxy)-1*H*-pyrrolo[2,3-*b*]pyridine
- 3-[(2-amino)pyrimidin-4-yl]-4-(2-hydroxybenzyloxy)-1*H*-pyrrolo[2,3-*b*]pyridine
- 3-[(2-amino)pyrimidin-4-yl]-4-(2-hydroxybenzyloxy)-1*H*-pyrrolo[2,3-*b*]pyridine
- 3-[(2-amino)pyrimidin-4-yl]-4-(pyridin-4-ylmethoxy)-1*H*-pyrrolo[2,3-*b*]pyridine
- 3-[(2-amino)pyrimidin-4-yl]-4-(pyridin-3-ylmethoxy)-1*H*-pyrrolo[2,3-*b*]pyridine
- 3-[(2-amino)pyrimidin-4-yl]-4-(pyridin-2-ylmethoxy)-1*H*-pyrrolo[2,3-*b*]pyridine
- 3-[(2-amino)pyrimidin-4-yl]-4-(pyridin-5-ylmethoxy)-1*H*-pyrrolo[2,3-*b*]pyridine
- 3-[(2-amino)pyrimidin-4-yl]-4-(pyridazin-4-ylmethoxy)-1*H*-pyrrolo[2,3-*b*]pyridine
- 3-[(2-amino)pyrimidin-4-yl]-4-(piperidin-4-ylmethoxy)-1*H*-pyrrolo[2,3-*b*]pyridine
- 3-[(2-amino)pyrimidin-4-yl]-4-(1-methanesulfonylpiperidin-4-ylmethoxy)-1*H*-pyrrolo[2,3-*b*]pyridine

8. A process for synthetizing compounds according to any one of claims 1 to 7, **characterized in that** it comprises a stage wherein at least one compound of formula II is used, formula II being defined as follows: 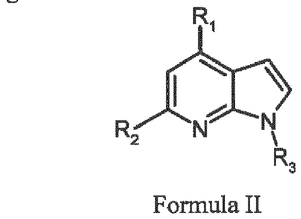 in which
- R₁ is an ethoxy, propoxy, butyloxy, isopropoxy, benzyloxy, cyclohexylmethoxy or cyclohexyloxy group,
- R₂ is H and
- R₃ is H.

9. The compound of formula I as claimed in any one of claims 1 to 7 or one of its pharmaceutically acceptable salts for use as medicament.

10. A pharmaceutical composition, **characterized in that** it comprises at least one compound of formula I as claimed in any one of claims 1 to 7 or at least one of its pharmaceutically acceptable salts and a pharmaceutically acceptable excipient.

11. The use of at least one compound as claimed in any one of claims 1 to 7 or of at least one of its pharmaceutically acceptable salts in the manufacture of a medicament for the treatment of disorders and diseases related to an abnormal proliferation of cells, in particular chosen from a tumor, and kidney diseases, such as glomerulonephritis or polycystic kidney disease.

12. The use of at least one compound as claimed in any one of claims 1 to 7 or of at least one of its salts in the manufacture of a medicament for the treatment of Alzheimer's disease.

13. The use of the compound of formula (Ij) of claim 6 or of at least one of its salts in the manufacture of a medicament for the treatment of trisomy 21.

## Patentansprüche

1. Verbindungen der folgenden Formel I: 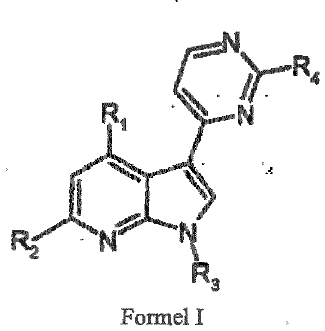
- worin R₁ ausgewählt ist aus einem Halogen oder einer substituierten oder nicht substituierten C₁-C₁₀-Akylgruppe, einer (C₁-C₁₀-Alkyl)C₅-C₈-Cycloalkylgruppe, einer (C₁-C₁₀-Alkyl)C₆-C₁₈-Arylgruppe, einer aromatischen oder nicht aromatischen (C₁-C₁₀-Alkyl)C₅-C₁₂-Heterocyclusgruppe, welche ein bis drei Heteroatome aufweist, einer substituierten oder nicht substituierten C₁-C₁₀-Alkoxygruppe, einer C₁-C₁₀-Fluoralkoxy-gruppe, einer (C₁-C₁₀-Alkoxy)C₁-C₁₀-Alkoxygruppe, einer C₅-C₈-Cycloalkoxygruppe, einer (C₁-C₁₀-Alkoxy)C₅-C₈-Cycloalkylgruppe, einer (C₁-C₁₀-Alkoxy)C₆-C₈-Arylgruppe, einer aromatischen oder nicht aromatischen (C₁-C₁₀-Alkoxy)C₅-C₁₂-Heterocyclusgruppe, welche ein bis drei Heteroatome aufweist, einer substituierten oder nicht substituierten C₂-C₁₀-Alkenylgruppe, einer (C₂-C₁₀-Alkenyl)C₅-C₈-Cycloalkylgruppe,
einer (C₂-C₁₀-Akenyl)C₆-C₁₈-Arylgruppe, einer aromatischen oder nicht aromatischen (C₂-C₁₀-Alkenyl)C₅-C₁₂-Heterocyclusgruppe, die ein bis drei Heteroatome aufweist, einer substituierten oder nicht substituierten C₂-C₁₀-Alkinylgruppe, einer (C₂-C₁₀-Alkinyl)C₅-C₈-Cycloalkylgruppe, einer (C₂-C₁₀-Alkinyl)C₆-C₁₈-Arylgruppe, einer aromatischen oder nicht aromatischen (C₂-C₁₀-Alkinyl)C₅-C₁₂-Heterocyclusgruppe mit ein bis drei Heteroatomen, -OH, -OCORₐ, -CN, -NO₂,-SRₐ, -NRₐR_{b}, -NHCORₐ, -NHSO₂Rₐ, - NHSO₂Rₐ, -NHCONRₐR_{b}, -NHCO₂Rₐ, einer Phenylgruppe, einer C₆-C₁₈-Arylgruppe und und einer aromatischen oder nicht aromatischen C₅-C₁₂-Heterocyclusgruppe mit ein bis drei Heteroatomen,
- R₂ ein Wasserstoffatom oder Halogen darstellt, oder unabhängig von R₁ eine Gruppe, die wie für R₁ definiert ist,
- R₃ H oder eine Gruppe -SO₂Rₐ oder-CORₐ oder eine C₁-C₁₀-Alkylgruppe bedeutet,
- R₄ ein Wasserstoffatom oder eine NH₂-Gruppe bedeutet, und
- Rₐ und R_{b} jeweils unabhängig voneinander ein Wasserstoffatom bedeuten, oder eine gegebenenfalls substituierte Gruppe, ausgewählt aus einem C₁-C₁₀-Alkyl, einem C₂-C₁₀-Alkenyl, einem C₂-C₁₀-Alkinyl, einem C₅-C₈-Cycloakyl, einem (C₅-C₈-CyCloalkyl) C₁-C₁₀-Alkyl, einem (C₅-C₈-Cycloalkyl)C₂-C₁₀-Alkinyl, einem (C₅-C₈-Cycloalkyl)C₂-C₁₀-Alkinyl, einem (C₅-C₁₂-Heterocyclo)C₁-C₁₀-Alkyl, einem (C₅-C₁₂-Heterocycloalkyl)C₂-C₁₀-Alkenyl, einem (C₅-C₁₂-Heteroeycloalkyl)C₂-C₁₀-Alkinyl, oder Rₐ und R_{b} miteinander verbunden sind, um mit dem Stickstoffatorn, woran sie gebunden sind, einen gegebenenfalls substituierten zu bilden, ausgewählt aus einer Pyrrolidinyl-, Piperidinyl-, Piperazinyl- oder Morpholinylgruppe,
sowie phannazeutisch akzeptable Salze der Verbindungen der Formel I.

2. Verbindungen nach Anspruch 1 mit der Formel I, worin:
- R₁ ausgewählt ist aus einem Halogen oder einer C₁-C₁₀ -Alkylgruppe, einer substituierten oder unsubstiuierten C₁-C₁₀-Alkₒₓygruppe, einer C₁-C₁₀-Fluoralkoxygruppe, einer C₁-C₁₀-Alkoxy-C₁-C₁₀-Alkoxygruppe, einer C₅-C₈-Cycloalkoxygruppe, -OH, -OCORₐ, -NO₂, -CN, -NO₂, -SRₐ, -NₐR_{b}, -NHCORₐ, -NHSO₂Rₐ, NHCONRₐR_{B}, Phenyl, Aryl und Heteroaryl,
- R₂ ein Wasserstoffatom darstellt oder unabhängig von R₁, ein Halogen oder eine Gruppe die wie für R₁ definiert ist,
- R₃ H oder eine Gruppe -SO₂Rₐ oder -CORₐ oder Alkyl darstellt,
- R₄ ein Wasserstoffatom oder eine NH₂-Gruppe darstellt,
- Rₐ und R_{b} jeweils unabhängig voneinander ein Wasserstoffatom oder eine gegebenenfalls substituierte Gruppe darstellen, ausgewählt aus Alkyl-, Alkenyl-, Alkinyl-, Cycloalkyl-, Cycloalkylalkyl-, Cycloalkylalkenyl-, Cycloalkylalkinyl-, Heterocycloalkyl-, Heterocycloalkylalkyl- und Heteroeycloalkylalkenyl-, oder Rₐ und R_{b} miteinander verbunden sind, um mit dem Stickstoff, an welchen sie gebunden sind, einen gegebenenfalls substituierten Heterocyclus zu bilden ausgewählt aus einer Pyrrolidinylgruppe, einer Piperidinylgruppe, Piperazinylgmppe oder Morpholinylgruppe.

3. Verbindungen nach Anspruch 1 oder 2 der Formel I worin R₃ H oder CH₃ ist.

4. Verbindungen nach Anspruch 1 oder 3 mit der Formel I, worin
R₁ OH, Cl oder einer Methoxy-, Ethoxy-, Propoxy-, Butyloxy-, Isopmpoxy-, Benzyloxy-, Cyclohexyhnethoxy-, Cyclohexyloxy, 2-Propyletlünyl-, 2-Butylethinyl-, 2-Cyclohexylethinyl-, Pheneth-1-inyl-, Phenyl-, Pentyl- oder Phenylethylgruppe ausgewählt ist,
R₂ H oder Br ist,
R₃ H oder CH₃ ist, und
R₄ H oder NH₂ ist.

5. Verbindungen gemäß den Ansprüchen 1 und 3 bis 4 der Formel I, worin:
R₁ ausgewählt ist aus einer Methoxygruppe, Ethoxygruppe, Propoxygruppe, Isopropoxygruppe, Benzyloxygruppe, Cyclohexylmethoxygruppe, Cyclohexyloxygruppe, 2-Propylethinylgruppe, Pentylgruppe, Phenylgruppe und Phenylethylgruppe,
- R₂ H oder Br ist,
- R₃ H oder CH₃ ist, und
- R₄ H oder NH₂ ist.

6. Verbindung gemäß einem der vorhergehenden Ansprüche, ausgewählt aus:
- dem *3*-[(2-Amino)pyrimidin-4-yl]-4-methoxy-1*H-*pymolo[2,3-*b*]pyridin der folgenden Formel Ia: 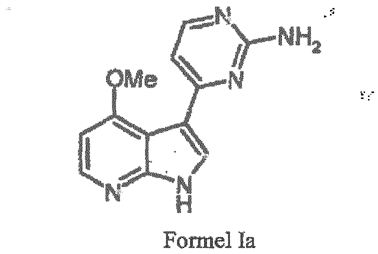
- dem 3-[(2-Amino)pyrimidin-4-yl]-4-ethoxy-1*H-*pyrrolo[2,3-*b*]pyridin der folgenden Formel Ib. 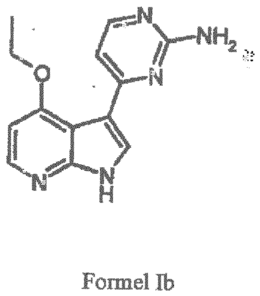
- dem 3-[(2-Amino)pyrimidin-4-yl]-4-propoxy-1*H-*pyrrolo[2,3-*b*]pyridin der folgenden Formel Ic: 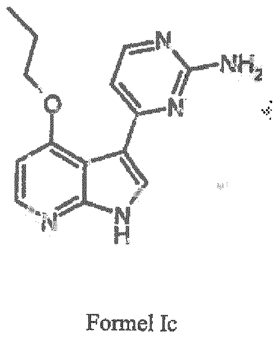
- dem 3-[(2-Amino)pyrimidin-4-yl]-4-methylethoxy-1*H-*pyrrolo[2,3-*b*]pyridin der folgenden Formel Id: 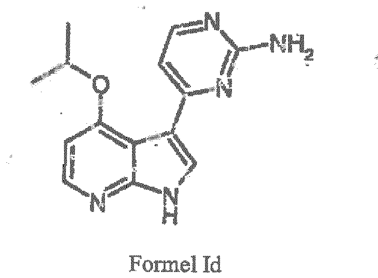
- dem 3-[(2-Amino)pyrimidin-4-yl]-4-benzyloxy-1*H-*pyrrolo[2,3-*b*]pyridin der folgenden Formel Ie: 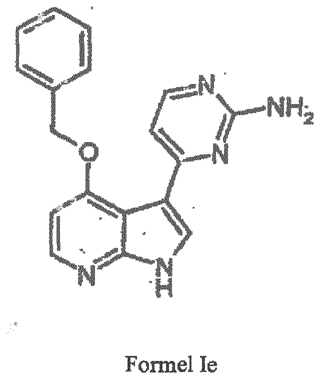
- dem 3-[(2-Amino)pyrimidin-4-yl]-4-(1-cyclohexylmethoxy)-1*H*-pyrrolo[2,3-*b*] pyridin der folgenden Formel If: 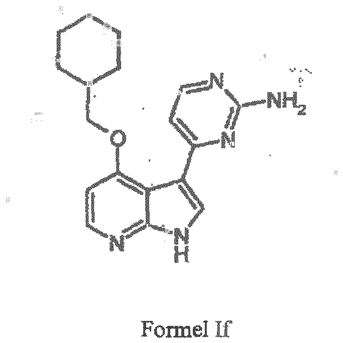
- dem 3-[(2-Amino)pyrimidin-4-yl]-4-(cyclohexyloxy)-1*H*-pyrrolo[2,3-*b*]pyridin der folgenden Formel Ig: 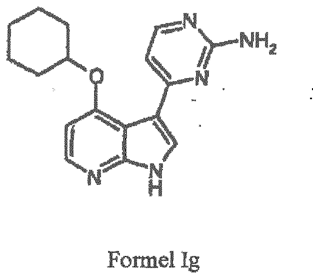
- dem 3-[(2-Amino)pyrimidin-4-yl]-4-(pent-1-inyl)-1*H-*pyrrolo[2,3-*b*]pyridin der folgenden Formel Ih: 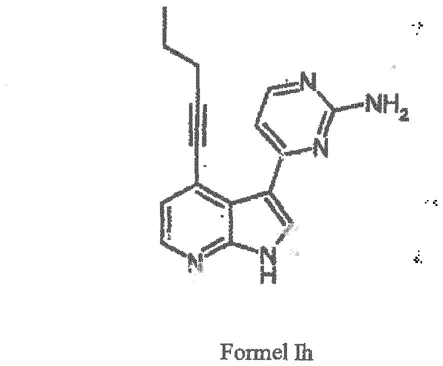
- dem 3-[(2-Amino)pyrimidin-4-yl]-4-pentyl-1*H-*pyrrolo[2,3*-b*]pyridin der folgenden Formel Ij: 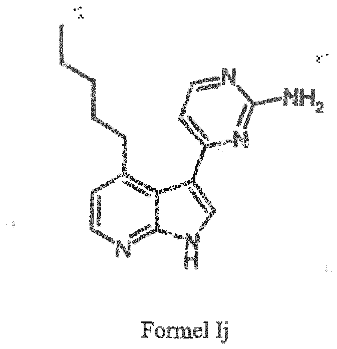
- dem 3-[(2-Amino)pyrimidin-4-yl]-4-phenethyl-1*H-*pyrrolo[2,3*-b*]pyridin der folgenden Formel Ik: 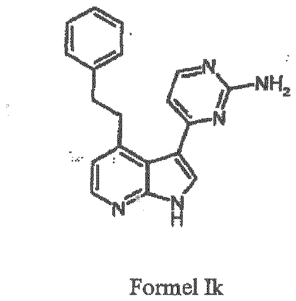
- dem 3-[(2-Amino)pyrimidin-4-yl]-4-phenyl-1*H-*pyrrolo[2,3*-b*]pyridin der folgenden Formel Im. 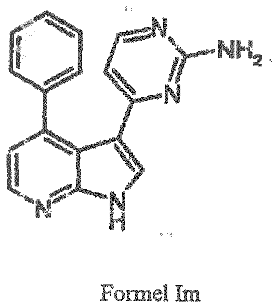
und pharmazeutisch akzeptablen Salzen davon.

7. Verbindung nach einem der Ansprüche 1 bis 5, ausgewählt aus:
- 3-[(2-Amino)pyrimidin-4-yl]-4-(4-methoxybenzyloxy)-1*H-*pyrrolo[2,3*-b*]pyridin,
- 3-[(2-Amino)pyrimidin-4-yl]-4-(3-methoxybenzyloxy)-1*H-*pyrrolo[2,3*-b*]pyridin,
- 3-[(2-Amino)pyrimidin-4-yl]-4-(2-methoxybenzyloxy)-1*H-*pyrrolo[2,3*-b*]pyridin,
- 3-[(2-Amino)pyrimidin-4-yl]-4-(4-chlorobenzyloxy)-1*H-*pyrrolo[2,3*-b*]pyridin,
- 3-[(2-Amino)pyrimidin-4-yl]-4-(3-chlorbenzyloxy)-1*H-*pyrrolo[2,3*-b*]pyridin,
- 3-[(2-Amino)pyrimidin-4-yl]-4-(2-chlorbenzyloxy)-1*H-*pyrrolo[2,3*-b*]pyridin,
- 3-[(2-Amino)pyrimidin-4-yl]-4-(4-fluorbenzyloxy)-1*H-*pyrrolo[2,3*-b*]pyridin,
- 3-[(2-Amino)pyrimidin-4-yl]-4-(3-fluorbenzyloxy)-1*H-*pyrrolo[2,3*-b*]pyridin,
- 3-[(2-Amino)pyrimidin-4-yl]-4-(4-hydroxybenzyloxy)-1*H-*pyrrolo[2,3*-b*]pyridin,
- 3-[(2-Amino)pyrimidin-4-yl]-4-(3-hydroxybenzyloxy)-1*H-*pyrrolo[2,3*-b*]pyridin,
- 3-[(2-Amino)pyrimidin-4-yl]-4-(pyridin-4-yknethoxy)-1*H-*pyrrolo[2,3*-b*]pyridin,
- 3-[(2-Amino)pyrimidin-4-yl]-4-(pyridin-3-ylmethoxy)-1*H-*pyrrolo[2,3*-b*]pyridin,
- 3-[(2-Amino)pyrimidin-4-yl]-4-(pyridin-2-ylmethoxy)-1*H-*pyrrolo[2,3*-b*]pyridin,
- 3-[(2-Amino)pyrimidin-4-yl]-4-(pyrimidin-5-ylmethoxy)-1*H-*pyrrolo[2,3*-b*]pyridin,
- 3-[(2-Amino)pyrimidin-4-yl]-4-(pyridazin-4-ylmethoxy)-1*H-*pyrrolo[2,3*-b*]pyridin,
- 3-[(2-Amino)pyrimidin-4-yl]-4-(piperidin-4-ylmethoxy)-1*H-*pyrrolo[2,3*-b*]pyridin,
- 3-[(2-Amino)pyrimidin-4-yl]-4-(1-methansulfonylpiperidin-4-ylmethoxy)-1*H-*pyrrolo[2,3*-b*]pyridin.

8. Verfahren zur Herstellung der Verbindungen nach einem der Ansprüche 1 bis 7, **dadurch gekennzeichnet dass** es einen Schritt beinhaltet, bei dem mindestens eine Verbindung der Formel II eingesetzt wird, wobei Formel II wie folgt definiert ist: 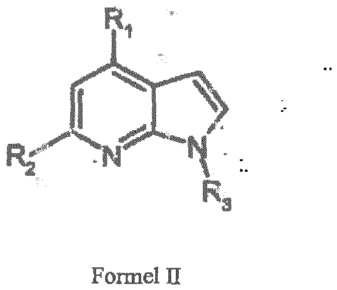 worin
- R₁ eine Ethoxy-, Propoxy-, Butyloxy-, Isopropoxy-, Benzyloxy-, Cyclohexylmethoxy- oder Cyclohexyloxygruppe ist,
- R₂ H ist, und
- R₃ H ist.

9. Verbidung der Formel I nach einem der Ansprüche bis 7, oder ein pharmazeutisch akzeptables Salz davon zur Verwendung als Arzneimittel.

10. Pharmazeutische Zusammensetzung, **dadurch gekennzeichnet, dass** diese mindestens eine Verbindung der Formel I nach einem der Ansprüche 1 bis 7 oder mindestens ein pharmazeutisch akzeptables Salz davon sowie einen pharmazeutisch akzeptablen Hilfsstoff enthält.

11. Verwendung mindestens einer Verbindung gemäß einem der Ansprüche 1 bis 7 oder mindestens eines pharmazeutisch akzeptablen Salzes davon zur Herstellung eines Arzneimittels zur Behandlung von Störungen oder Erkrankungen, die mit einer abnormalen Zellproliferation verbunden sind, insbesondere ausgewählt aus einem Tumor, und Nierenerkrankungen wie beispielsweise Glomerulonephritis oder polyzystischen Nieren.

12. Verwendung mindestens einer Verbindung nach einem der Ansprüche 1 bis 7 oder mindestens eines Salzes davon zur Herstellung eines Arzneimittels zur Behandlung der Ahheimer Krankheit.

13. Verwendung der Verbindung der Formel (Ij) nach anspruch 6 oder mindestens eines Salzes davon zur Herstellung eines Arzneimittels zur Behandlung von Trisomie 21.
